(19) Europäisches Patentamt
European Patent Office
Office européen des brevets

(11) EP 0 564 358 B1

(12) **FASCICULE DE BREVET EUROPEEN**

(45) Date de publication et mention de la délivrance du brevet:
**04.11.1998 Bulletin 1998/45**

(21) Numéro de dépôt: **93400839.2**

(22) Date de dépôt: **01.04.1993**

(51) Int Cl.[6]: **C07D 491/107**, C07D 519/00, A61K 31/40, A61K 31/445 // (C07D491/107, 311:00, 209:00), (C07D491/107, 311:00, 221:00), (C07D519/00, 498:00, 491:00)

(54) **Nouveaux dérivés spiraniques du 3-amino chromane, leurs procédés de préparation et les compositions pharmaceutiques qui les contiennent**

3-Aminochroman-Spiroderivate, Verfahren zu deren Herstellung und diese enthaltende pharmazeutische Zusammensetzungen

3-aminochromane spiro derivatives, processes for their preparation and pharmaceutical compositions containing them

(84) Etats contractants désignés:
**AT BE CH DE DK ES FR GB GR IE IT LI LU NL PT SE**

(30) Priorité: **01.04.1992 FR 9203935**

(43) Date de publication de la demande:
**06.10.1993 Bulletin 1993/40**

(73) Titulaire: **ADIR ET COMPAGNIE**
**92415 Courbevoie Cédex (FR)**

(72) Inventeurs:
- **Guillaumet, Gérald**
  **F-45100 Orléans (FR)**
- **Podona, Tchao**
  **F-45100 Orléans la Source (FR)**
- **Adam, Gérard**
  **F-78600 Le Mesnil le Roi (FR)**
- **Guardiola, Béatrice**
  **F-92200 Neuilly sur Seine (FR)**
- **Renard, Pierre**
  **F-78000 Versailles (FR)**

(56) Documents cités:
**EP-A- 0 222 996** **EP-A- 0 286 516**
**FR-A- 2 255 067**

- **JOURNAL OF MEDICINAL CHEMISTRY. vol. 21, no. 6, Juin 1978, WASHINGTON US pages 585 - 588 P. A. CROOKS ET AL. 'Synthesis of spiro(tetralin-2,2 -pyrrolidine) and spiro(indan-2,2 -pyrrolidine)derivatives as potential analgesics'**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 564 358 B1

Printed by Jouve, 75001 PARIS (FR)

## Description

La présente invention concerne de nouveaux dérivés spiraniques du 3-aminochromane, leurs procédés de préparation, et les compositions pharmaceutiques qui les contiennent.

Les 3-amino chromane, 3-amino thiochromane et certains de leurs dérivés, sont connus pour être des ligands des récepteurs du système nerveux central, plus particulièrement du système sérotoninergique, ce qui les rend utilisables pour le traitement de l'anxiété, de la dépression et plus particulièrement des troubles du système nerveux central (brevets européens EP 279150 et EP 222996). Les composés décrits dans ces brevets présentent une affinité certaine pour les récepteurs 5-$HT_{1A}$ mais également pour les récepteurs D2, ce qui entraine une très faible selectivité.

On connait également dans la littérature des dérivés spiraniques de la 2-aminotétraline (a) (J. Med. Chem. (1978) 21 (6) pp 585-7 et J. Pharm. Pharmacol. (1976) 28 suppl pp 83 P) et de la 3-aminoquinoléine (b) (brevet FR 2255067 et Yakugaku Zasshi (1974) 94 (12) pp 1566-73)

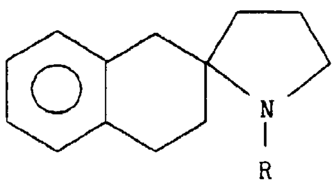

R = H, $CH_3$

(a)

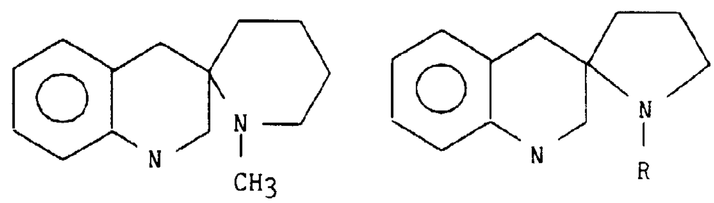

R = H, $CH_3$

(b)

Ces composés sont surtout décrits comme antalgiques, spasmolytiques et antihistaminiques, avec également pour les composés tétraliniques une très légère composante antidépressante.

Le spiro [pyrrolidine-2 : 3' chromane] (c) est également décrit dans la littérature, sans aucune autre précision que sa structure.

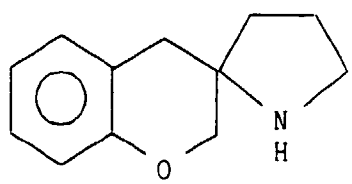

(c)

Les composés de la présente invention, qui sont des dérivés spiraniques du 3-amino chromane, outre le fait que leurs structures soient nouvelles, possèdent de remarquables propriétés pharmacologiques.

Ces composés sont en effet de puissants ligands des récepteurs 5-$HT_{1A}$. Cette forte affinité est d'autant plus intéressante qu'elle se double d'une grande sélectivité en faveur des récepteurs 5-$HT_{1A}$ par rapport aux récepteurs dopaminergiques, alpha adrénergiques et aux autres récepteurs sérotoninergiques (5-$HT_{1B}$, 5-$HT_{1C}$, 5-$HT_{1D}$, 5-$HT_2$).

Ces remarquables propriétés pharmacologiques rendent les composés de la présente invention utilisables dans le traitement des troubles du système nerveux central, spécialement du système sérotoninergique, comme la dépression, le stress, les psychoses, l'anxiété, la schizophrénie ainsi que de la douleur, des migraines, de l'hypertension et de l'ischémie cérébrale. Ces composés peuvent également être intéressants comme modificateurs du comportement alimentaire et sexuel.

Plus spécifiquement, la présente invention concerne les composés de formule générale (I) :

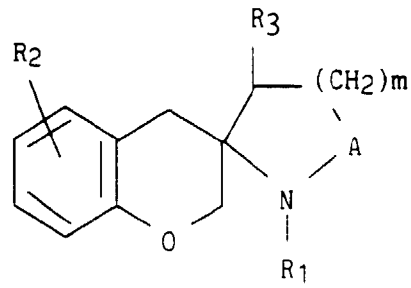

(I)

dans laquelle :

- m, nombre entier peut prendre les valeurs 1 ou 2,
- A représente un méthylène ($CH_2$) ou un carbonyle (CO),
- $R_1$ représente :
  - un hydrogène,
  - un groupement - CO - $R_4$ avec $R_4$ représentant un alkyle linéaire ou ramifié de 1 à 6 atomes de carbone, un phényl éventuellement substitué ou un phénylalkyle éventuellement substitué dont la chaine alkyle comporte de 1 à 3 atomes de carbone,
  - ou un alkyle linéaire ou ramifié de 1 à 6 atomes de carbone éventuellement substitué par :
    - un nitrile,
    - un phényl éventuellement substitué,
    - un groupement $-NR_5R_6$ avec $R_5$ représentant un hydrogène ou un alkyle linéaire ou ramifié de 1 à 6 atomes de carbone et R6 représentant un hydrogène, un alkyle linéaire ou ramifié de 1 à 6 atomes de carbone éventuellement substitué par un phényl éventuellement substitué, un groupement alkylcarbonyl linéaire ou ramifié comprenant de 2 à 7 atomes de carbone, un groupement benzoyl éventuellement substitué, un groupement phénylalkylcarbonyl éventuellement substitué dont la chaine alkyle comporte de 1 à 3 atomes de carbone, un groupement alkyle sulfonyle linéaire ou ramifié de 1 à 6 atomes de carbone, un groupement phénylsulfonyle éventuellement substitué,
    - un quelconque des groupements suivants :

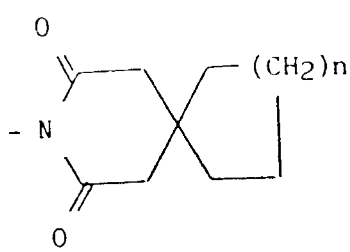

,

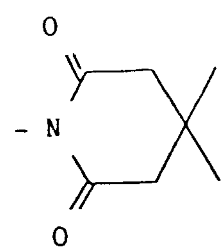

,

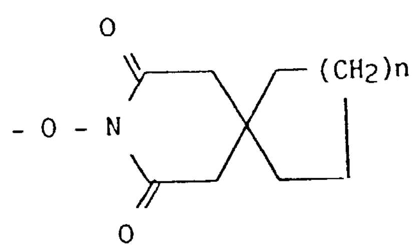

dans lesquels :

- n, nombre entier, peut prendre les valeurs 1 ou 2,

- $R_2$ représente :
  - un hydrogène,
  - un groupement acétyl,
  - un groupement $CF_3SO_2$-O-,
  - ou un groupement - $OR_7$ avec $R_7$ ayant la même définition que $R_1$,
- $R_3$ représente un hydrogène ou un alkyle linéaire ou ramifié de 1 à 4 atomes de carbone avec la réserve que lorsque $R_1 = R_2 = R_3 =$ alors m ne peut pas être égal à 1,
  l'expression "éventuellement substitué" associée aux termes phényl. phénylalkyle, phénylsulfonyl, benzoyl ou phénylalkylcarbonyl signifie que le noyau aromatique peut être substitué par un ou plusieurs alkyles inférieurs de 1 à 4 atomes de carbone ramifié ou non, nitro, alkoxy inférieur de 1 à 4 atomes de carbone, halogène, trifluorométhyle, hydroxy,
- leurs isomères, diastéréoisomères, énantiomères isolés ou sous forme de mélange,
- leurs sels d'addition à un acide minéral ou organique pharmaceutiquement acceptable.

L'invention s'étend également au procédé d'obtention des composés de formule générale (Ia) :

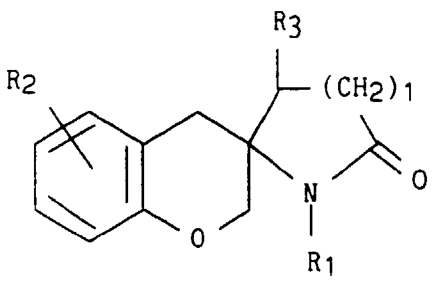

(Ia)

dans laquelle $R_1$, $R_2$ et $R_3$ sont tels que définis dans la formule (I),
cas particulier des composés de formule (I) pour lesquels m est égal à 1, et A représente un carbonyle (CO), caractérisé en ce que l'on fait réagir un benzaldéhyde substitué de formule générale (II) :

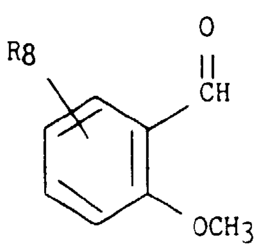

(II)

dans laquelle $R_8$ représente un hydrogène ou un alkoxy linéaire ou ramifié de 1 à 6 atomes de carbone, à une température comprise entre - 5° et 0°C, avec du tribromure de bore de manière à obtenir le composé de formule générale (III) :

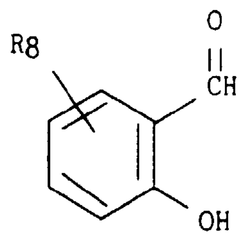

(III)

dans laquelle $R_8$ a la même signification que précédemment, que l'on fait réagir à chaud et en présence d'un sel d'ammonium substitué avec du 2-nitro éthanol, de manière à obtenir le composé de formule générale (IV) :

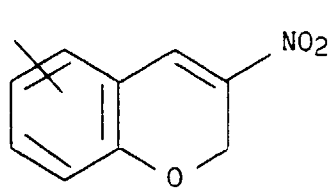

(IV)

dans laquelle $R_8$ a la même signification que précédemment, que l'on réduit de manière à obtenir le composé de formule générale (V) :

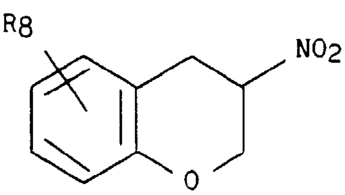

(V)

dans laquelle $R_8$ a la même signification que précédemment, que l'on fait réagir en présence de méthylate de benzyltriméthylammonium, en milieu alcoolique, avec un composé acrylique de formule générale (VI) :

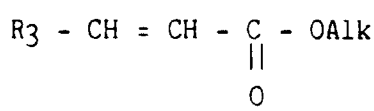

(VI)

dans laquelle $R_3$ a la même signification que précédemment et Alk représente un alkyle de 1 à 4 atomes de carbone, de manière à obtenir les composés de formule générale (VIIa) :

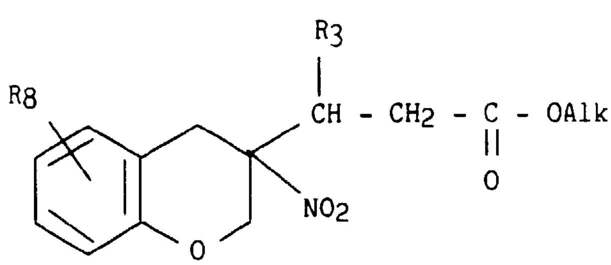

(VIIa)

dans laquelle $R_3$, $R_8$ et Alk ont la même signification que précédemment, que l'on réduit, en présence de Nickel de

Raney et sous atmosphère d'hydrogène, de manière à obtenir, après isolement et éventuelle purification, le composé spiranique de formule générale (VIIIa) :

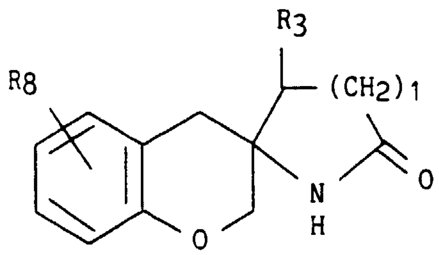

(VIIIa)

dans laquelle $R_3$ et $R_8$ ont la même signification que précédemment,
que l'on peut faire réagir en présence d'une base forte avec un composé de formule générale (IX) :

$$Hal'R'_1 \quad (IX)$$

dans laquelle $R'_1$ a la même signification que $R_1$ dans la formule (I) avec la réserve que R'1 ne peut représenter un hydrogène, et Hal' représente un atome d'halogène, de manière à obtenir, après isolement et éventuelle purification, le composé de formule générale (Xa) :

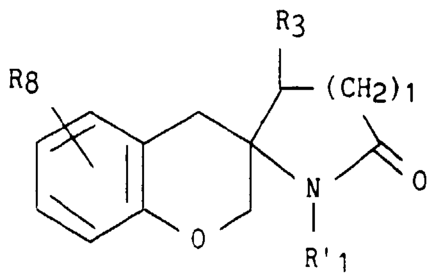

(Xa)

dans laquelle $R'_1$, $R_3$ et $R_8$ ont la même signification que précédemment, composés de formule (VIIIa) et (Xa) que l'on peut traiter, dans le cas où $R_8$ représente un alkoxy, par une solution aqueuse d'acide bromhydrique, pour obtenir, après isolement et éventuelle purification, le composé de formule générale (XIa):

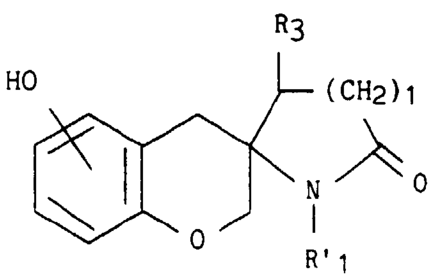

(XIa)

dans laquelle $R'_1$ et $R_3$ ont la même signification que précédemment, que l'on peut faire réagir avec un composé de formule générale (XII) :

$$Hal'' - R'_7 \quad (XII)$$

dans laquelle Hal" représente un atome d'halogène et $R'_7$ a la même signification que $R_7$ dans la formule (I), avec la réserve que $R'_7$ ne peut représenter un hydrogène, de manière à obtenir, après isolement et éventuelle purification, le composé de formule générale (XIIIa) :

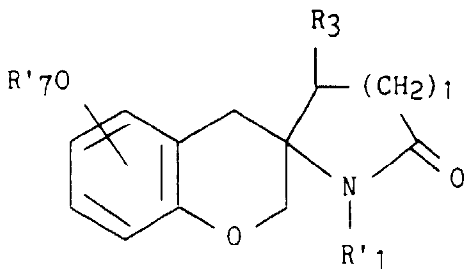

(XIIIa)

dans laquelle $R'_1$, $R_3$ et $R'_7$ ont la même signification que précédemment, étant entendu que les composés de formule générale VIIIa, Xa, XIa, et XIIIa font partie de l'invention, constituent les composés de formule générale (Ia) tels que définis précedemment, et peuvent, si on le désire, être purifiés, séparés en leurs isomères, ou si cela est possible salifiés avec un acide pharmaceutiquement acceptable.

L'invention s'étend également au procédé d'obtention des composés de formule générale (Ib) :

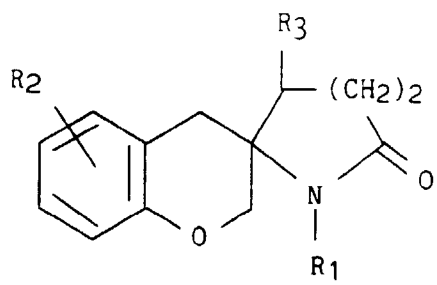

(Ib)

dans laquelle $R_1$, $R_2$ et $R_3$ sont tels que définis dans la formule (I),
cas particulier des composés de formule (I) pour lesquels m est égal à 2 et A représente un carbonyle (CO), caractérisé en ce que l'on fait réagir un composé de formule (V) :

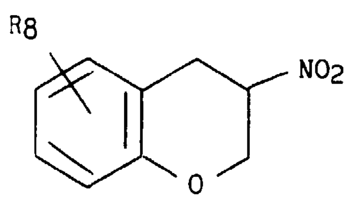

(V)

dans laquelle $R_8$ représente un hydrogène ou un alkoxy linéaire ou ramifié de 1 à 6 atomes de carbone,
avec un halogéno ester de formule générale (X) :

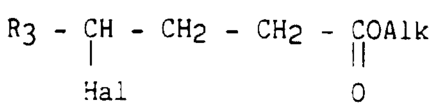

(X)

dans laquelle $R_3$ a la même signification que précedemment, Alk représente un alkyle de 1 à 4 atomes de carbone et Hal représente un atome d'halogène, de manière à obtenir les composés de formule générale (VIIb) :

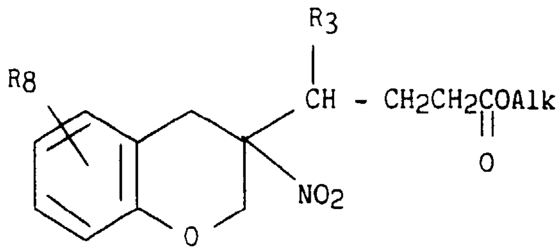

(VIIb)

dans laquelle $R_3$, $R_8$ et Alk ont la même signification que précédemment, que l'on réduit, en présence de Nickel de Raney et sous atmosphère d'hydrogène, de manière à obtenir, après isolement et éventuelle purification, le composé spiranique de formule générale (VIIIb) :

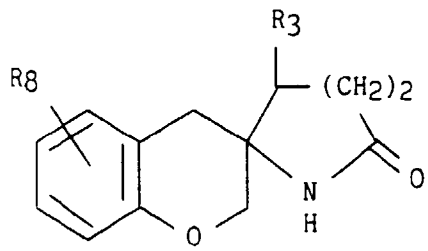

(VIIIb)

dans laquelle $R_3$ et $R_8$ ont la même signification que précédemment que l'on peut faire réagir en présence d'une base forte avec un composé de formule générale (IX) :

$$Hal'R'_1 \qquad (IX)$$

dans laquelle $R'_1$ a la même signification que $R_1$ dans la formule (I) avec la réserve que $R'_1$ ne peut représenter un hydrogène et Hal' représente un atome d'halogène, de manière à obtenir, après isolement et éventuelle purification, le composé de formule générale (Xb) :

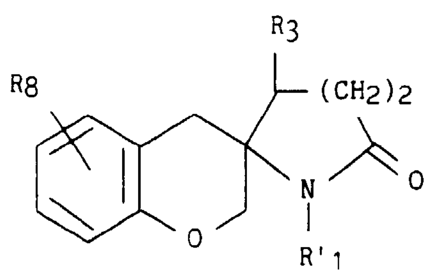

(Xb)

dans laquelle $R'_1$, $R_3$ et $R_8$ ont la même signification que précédemment, composés de formule (VIIIb) et (Xb) que l'on peut traiter, dans le cas où $R_8$ représente un alkoxy, par une solution aqueuse d'acide bromhydrique, pour obtenir, après isolement et éventuelle purification, le composé de formule générale (XIb):

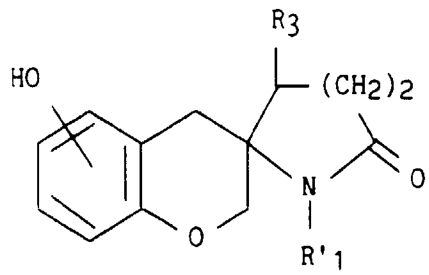

(XIb)

dans laquelle $R'_1$ et $R_3$ ont la même signification que précédemment, que l'on peut faire réagir avec un composé de formule générale (XII) :

$$Hal'' - R'_7 \quad (XII)$$

dans laquelle Hal" représente un atome d'halogène et $R'_7$ a la même signification que $R_7$ dans la formule (I), avec la réserve que $R'_7$ ne peut représenter un hydrogène, de manière à obtenir, après isolement et éventuelle purification, le composé de formule générale (XIIIb) :

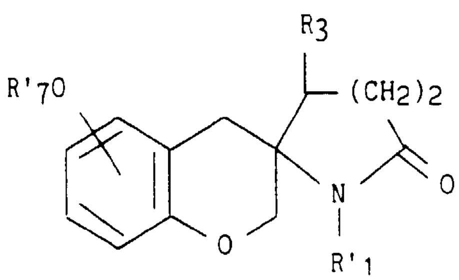

(XIIIb)

dans laquelle $R'_1$, $R_3$ et $R'_7$ ont la même signification que précédemment, étant entendu que les composés de formule générale VIIIb, Xb, XIb, et XIIIb font partie de l'invention, constituent les composés de formule générale (Ib) tels que définis précedemment, et peuvent, si on le désire, être purifiés, séparés en leurs isomères, ou si cela est possible salifiés avec un acide pharmaceutiquement acceptable.

L'invention s'étend également au procédé d'obtention des composés de formule générale (Ic) :

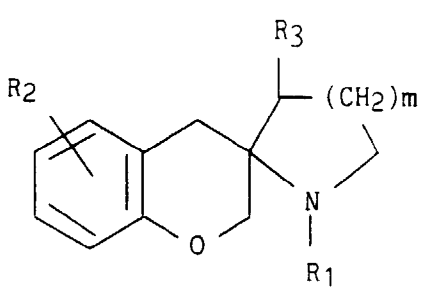

(Ic)

dans laquelle $R_1$, $R_2$, $R_3$ et m sont tels que définis dans la formule (I),
cas particulier des composés de formule (I) pour lesquels A représente un méthylène ($CH_2$),
caractérisé en ce que l'on réduit en milieu aprotique un composé de formule (VIII) :

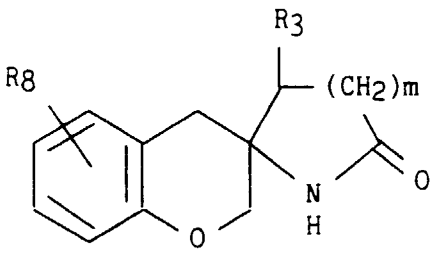

(VIII)

dans laquelle $R_3$ et m sont tels que définis précedemment et $R_8$ représente un hydrogène ou un alkoxy linéaire ou ramifié de 1 à 6 atomes de carbone, par un agent réducteur de manière à obtenir, après isolement et éventuelle purification, le composé de formule générale (XIV) :

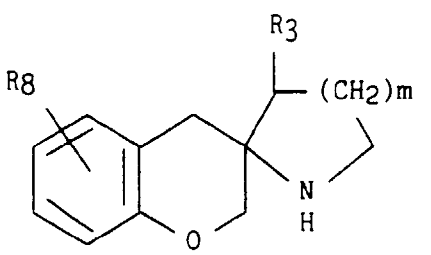

(XIV)

dans laquelle $R_3$, $R_8$ et m ont la même signification que précédemment, que l'on peut faire réagir avec un composé de formule générale (IX) :

$$Hal'R'_1 \qquad (IX)$$

dans laquelle $R'_1$ a la même signification que $R_1$ dans la formule (I) avec la réserve que $R'_1$ ne peut représenter un hydrogène et Hal représente un atome d'halogène, de manière à obtenir, après isolement et éventuelle purification, le composé de formule générale (XV):

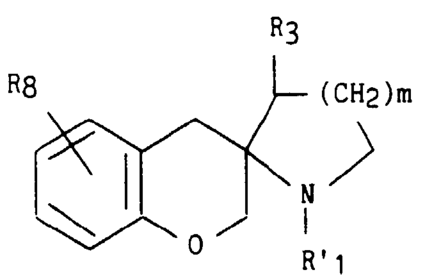

(XV)

dans laquelle $R'_1$, $R_3$, $R_8$ et m ont la même signification que précédemment, composés de formule (XIV) et (XV) que l'on peut traiter, dans le cas où $R_8$ représente un alkoxy, par une solution aqueuse d'acide bromhydrique, pour obtenir, après isolement et éventuelle purification, le composé de formule générale (XVI) :

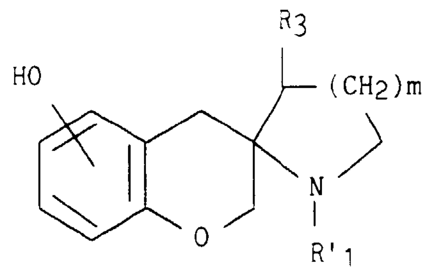

(XVI)

dans laquelle $R'_1$, $R_3$ et m ont la même signification que précédemment, que l'on peut faire réagir avec un composé de formule générale (XII) :

$$Hal'' - R'_7 \qquad (XII),$$

dans laquelle Hal" représente un atome d'halogène et $R'_7$ a la même signification que $R_7$ dans la formule (I), avec la réserve que $R'_7$ ne peut représenter un hydrogène, de manière à obtenir, après isolement et éventuelle purification, le composé de formule générale (XVII) :

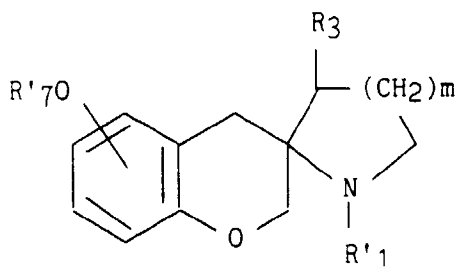

(XVII)

dans laquelle $R'_1$, $R_3$, $R'_7$ et m ont la même signification que précédemment,
étant entendu que les composés de formule générale XIV, XV, XVI, et XVII font partie de l'invention, constituent les composés de formule générale (Ic), et peuvent, si on le désire, être purifiés, séparés en leurs isomères, ou si cela est possible salifiés avec un acide pharmaceutiquement acceptable.

L'invention s'étend également au procédé d'obtention des composés de formule générale (Id) :

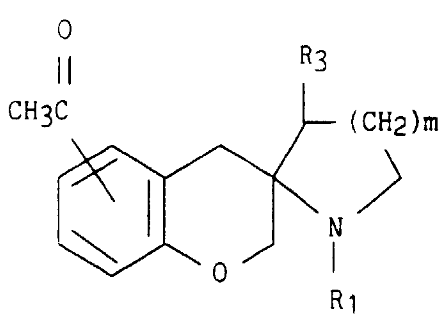

(Id)

dans laquelle $R_1$, $R_3$ et m sont tels que définis dans la formule (I),
cas particulier des composés de formule (I) pour lesquels $R_2$ représente un groupe acétyl, caractérisé en ce que l'on fait réagir avec de l'anhydride trifluorométhanesulfonique un composé de formule générale (XVIII) :

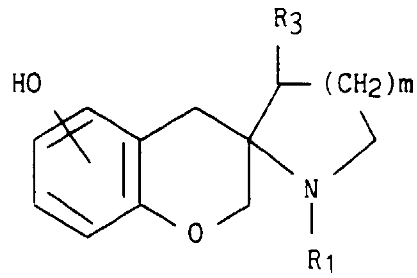

(XVIII)

dans laquelle $R_1$, $R_2$ et m sont tels que définis précédemment, de manière à obtenir le dérivé trifluorométhylsulfonyloxy de formule générale (XIX) :

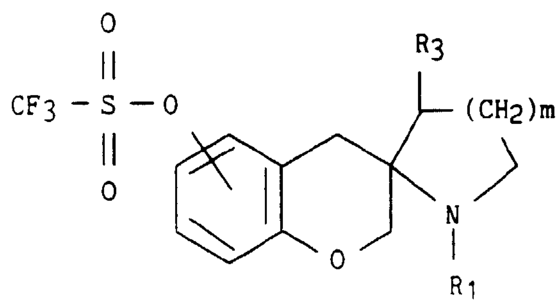

(XIX)

dans laquelle $R_1$, $R_3$ et m ont la même définition que précédemment, que l'on traite ensuite en milieu aprotique par du butylvinyléther en présence de triéthylamine, de 1,2-bis (diphénylphosphino) éthane et de Pd $(OAc)_2$ de manière à obtenir après isolement et purification le composé acétylé de formule générale (XX) :

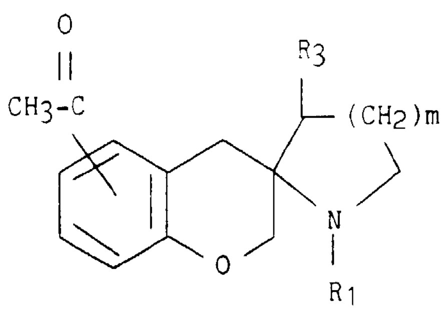

(XX)

dans laquelle $R_1$, $R_3$ et m ont la même signification que précédemment,
étant entendu que les composés de formule générale XIX et XX font partie de l'invention, et peuvent, si on le désire être purifiés, séparés en leurs isomères, ou, si cela est possible salifiés avec un acide pharmaceutiquement acceptable.

Les composés de formule générale (I), ainsi que leurs sels d'addition à un acide minéral ou organique pharmaceutiquement acceptable, sont de puissants ligands des récepteurs $5\text{-}HT_{1A}$, avec une activité agoniste ou antagoniste, au niveau du système nerveux central.

Cette forte affinité qui se double d'une très grande sélectivité vis à vis de ces récepteurs par rapport aux récepteurs $D_2$, $\alpha_2$ et aux autres récepteurs sérotoninergiques rend ces composés très intéressants pour le traitement du stress, de l'anxiété, de la dépression, des psychoses, de la schizophrénie, de la douleur, des maladies cardiovasculaires, de l'hypertension, des migraines et de l'ischémie cérébrale.

Ils peuvent également modifier le comportement alimentaire et sexuel.

Les composés de formule générale (I), ainsi que leurs sels d'addition à un acide minéral ou organique pharmaceutiquement acceptable, comme par exemple les acides chlorhydrique, méthanesulfonique, nitrique, maléique peuvent être présentés sous forme de compositions pharmaceutiques, selon des procédés connus, comme par exemple :

comprimés, gélules, dragées, solutions injectables, solution ou suspensions buvables, émulsions, suppositoires.

Outre les excipients inertes, non toxiques et pharmaceutiquement acceptables, tels par exemple l'eau distillée, le glucose, le lactose, l'amidon, le talc, les huiles végétales, l'éthylène glycol, ces compositions peuvent également contenir des agents de préservation.

Les compositions pharmaceutiques ainsi obtenues font également partie de l'invention et peuvent contenir, selon les affections traitées, l'age et le poids du malade, de 0,1 à 100 mg de principe actif pour un traitement en 1 à 3 prises par 24 heures.

Les exemples suivants illustrent l'invention.

**PREPARATION 1 : N-(4-BROMOBUT-1-YL)-8-AZASPIRO [4,5] DECANE-7,9-DIONE**

Ajouter 7,43 g (53,8 mmoles) de carbonate de potassium, 4,26 g (19,73 mmoles) de 1,4-dibromo butane et une quantité catalytique d'iodure de potassium à une solution de 3 g ( 17,94 mmoles) de 8-azaspiro [4,5] décane-7,9-dione.

Chauffer 6 heures à 60°C puis refroidir et éliminer l'acétonitrile par évaporation sous pression réduite.

Après hydrolyse avec 10 $cm^3$ d'eau, extraire le produit avec du chlorure de méthylène puis, après séchage et mise à sec, purifier le produit brut par chromatographie sur colonne de silice (Eluant : chlorure de méthylène).

On obtient finalement la N-(4-bromobut-1-yl)-8-azaspiro [4,5] décane-7,9-dione sous forme d'huile avec un rendement de 69 %.

Infra Rouge (film) : 1660 et 1720 $cm^{-1}$ $\nu$ C = 0

RMN $^1H$ 300 MHz ($CDCl_3$) $\delta$ : ppm

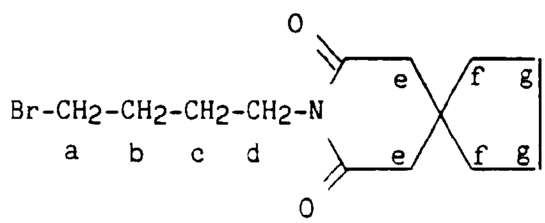

1,45 à 1,52; multiplet; 4H; 2 $CH_2$
1,45 à 1,52; multiplet; 6H; 3 $CH_2$
1,79 à 1,90; multiplet; 2H;$H_c$
2,60; singulet; 4H; $H_e$
3,42; triplet J = 7,1 Hz; 2H; Ha
3,80; triplet J = 7,1 Hz; 2H; Hd

**PREPARATION 2 et 3**

En procédant de la même façon que pour la préparation 1 mais en remplaçant le 1,4-dibromobutane par :

- le 1,5-dibromobutane, on obtient la N-(5-bromopent-1-yl)-8-azaspiro [4,5] décane-7,9-dione avec un rendement de 50 %.
  Infra Rouge (film) : 1655 et 1715 $cm^{-1}$ $\nu$ C = 0
  RMN $^1H$ 300 MHz ($CDCl_3$) $\delta$ : ppm

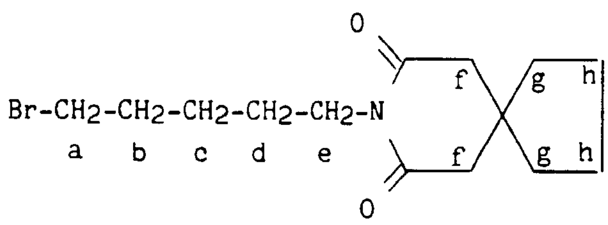

1,35 à 1,73; multiplet; 12H; 6 $CH_2$

1,81 à 1,90; multiplet; 2H; $CH_2$
2,57; singulet; 4H; Hf
3,38; triplet J = 7,1 Hz; 2H; Ha
3,74; triplet J = 7,1 Hz; 2H; He

- le 1,3-dibromopropane, on obtient la N-(3-bromoprop-1-yl)-8-azaspiro [4,5] décane-7,9-dione avec un rendement de 70 %.
  Infra Rouge (film) : 1665 et 1720 $cm^{-1}$ ν C = 0
  RMN $^1H$ 300 MHz ($CDCl_3$) δ : ppm

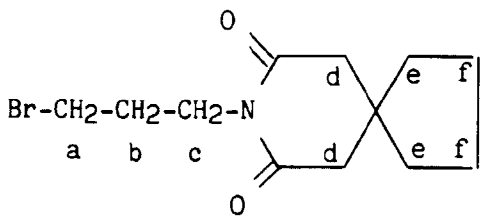

1,45 à 1,56; multiplet; 4H; 2 $CH_2$
1,66 à 1,75; multiplet; 4H; 2 $CH_2$
2,06 à 2,15; multiplet; 2H; Hb
2,59; singulet; 4H; Hd
3,36; triplet J = 7,1 Hz; 2H; Ha
3,89; triplet J = 7,1 Hz; Hc

**PREPARATION 4 : N-(2-BROMOETHYL)-8-AZASPIRO [4,5] DECANE-7,9-DIONE**

Additionner 0,65 g (26,9 mmoles) d'hydrure de sodium à une solution de 3 g (17,94 mmoles) de 8 -azaspiro [4,5] décane-7,9-dione dans 30 $cm^3$ de N,N-diméthylformamide anhydre.

Agiter 1 heure à 60°C puis additionner 20,21 g (107,6 mmoles) de 1,2-dibromoéthane et une quantité catalytique d'iodure de potassium.

Maintenir le chauffage une heure après l'addition puis, après refroidissement, éliminer le solvant sous pression réduite, puis, après hydrolyse aqueuse, extraire le produit au chlorure de méthylène et purifier le produit brut obtenu par chromatographie sur colonne de silice.

On obtient ainsi la N-(2-bromoéthyl)-8-azaspiro [4,5] décane-7,9-dione avec un rendement de 63 %.
Infra Rouge (film) : 1660 et 1715 $cm^{-1}$ ν C = 0
RMN $^1H$ 300 MHz ($CDCl_3$) δ : ppm

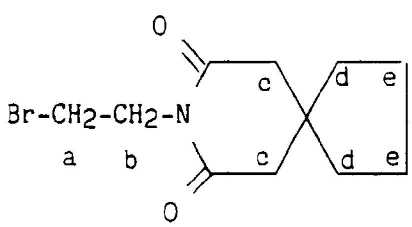

1,46 à 1,54; multiplet; 4H; 2 $CH_2$
1,67 à 1,76; multiplet; 4H; 2 $CH_2$
2,62; singulet; 4H; $H_c$
3,48; triplet J = 6,9 Hz; 2H; Ha
4,20; triplet J = 6,9 Hz; 2H; Hb

**PREPARATIONS 5 A 7**

En procédant de la même façon que pour la préparation 1 mais en remplaçant la 8-azaspiro [4,5] décane-7,9-dione par :

- la 1,1-dioxo-1,2-benzisothiazol-3(2H)-one on obtient la N-(4-bromobutyl)-1,1-dioxo-1,2-benzisothiazol-3(2H)-one avec un rendement de 60 %.

  Infra Rouge (film) : 1720 $cm^{-1}$ $\nu$ C = 0 1300 et 1170 $cm^{-1}$ $\nu$ $SO_2$

  RMN $^1H$ 300 MHz ($CDCl_3$) $\delta$ : ppm

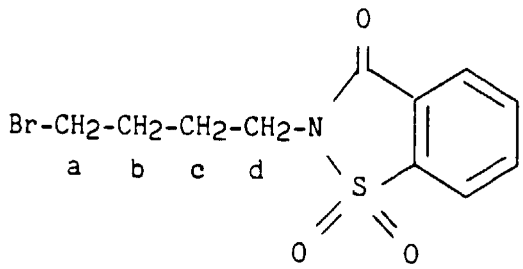

  1,95 à 2,05; multiplet; 4H; Hb et Hc
  3,44; triplet J = 7,1 Hz; 2H; Ha
  3,82; triplet J = 7,1 Hz; 2H; Hd
  7,78 à 8,05; multiplet; 4H aromatiques

- le 2,4-dioxo-3-azabicyclo [3.3.0] octane on obtient la N-(4-bromobutyl)-3-azabicyclo [3.3.0] octane-2,4-dione avec un rendement de 58 %.
  Infra Rouge (film) : 1685 et 1760 $cm^{-1}$ $\nu$ C = 0
  RMN $^1H$ 300 MHz ($CDCl_3$) $\delta$ : ppm

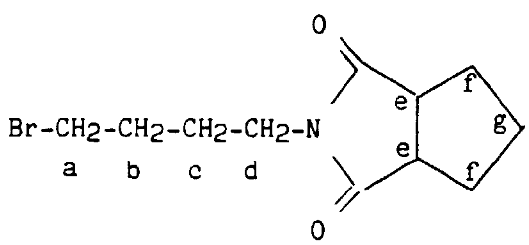

  1,15 à 1,38; multiplet; 2H; $CH_2$
  1,62 à 2,14; multiplet; 8H; 4 $CH_2$
  3,08 à 3,17; multiplet; 2H; $H_e$
  3,38; triplet J = 6,6 Hz; 2H; Ha
  3,48; triplet J = 6,6 Hz; 2H; Hd

- la 4,4-diméthylpipéridine-2,6-dione on obtient la N-(4-bromobutyl)-4,4-diméthyl pipéridine-2,6-dione avec un rendement de 65 %.
  Infra Rouge (film) : 1660 et 1715 $cm^{-1}$ $\nu$ C = 0
  RMN $^1H$ 300 MHz ($CDCl_3$) $\delta$ : ppm

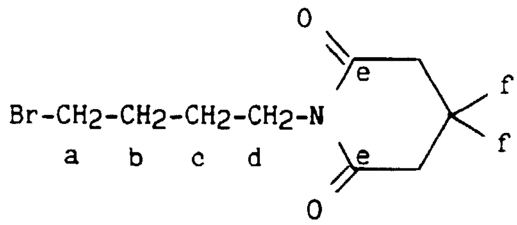

1,06; singulet; 6H; Hf
1,61 à 1,73; multiplet; 2H; $CH_2$
1,80 à 1,91; multiplet; 2H; $CH_2$
2,51; singulet; 4H; He
3,41; triplet J = 6,3 Hz; 2H; Ha
3,80; triplet J = 6,3 Hz; 2H; Hd

**PREPARATIONS 8 ET 9**

En procédant de la même façon que pour la préparation 4, mais en remplaçant le 1,2-dibromobutane par le 1,4-dibromobutane et la 8-azaspiro [4,5] décane-7,9-dione par :

- la 3-azabicyclo[3.3.0] octane-2-one on obtient le N-(4-bromobutyl)-3-azabicyclo [3.3.0] octane-2-one avec un rendement de 52 %.
  Infra Rouge (film) : 1670 $cm^{-1}$ $\nu$ C = 0
  RMN $^1H$ 300 MHz ($CDCl_3$) $\delta$ : ppm

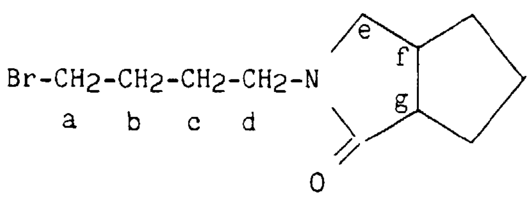

1,40 à 2,29; multiplet; 10H; $5CH_2$
2,70 à 2,95; multiplet; 2H; $CH_2$
3,00 ; doublet dédoublé $J_1$ = 9,7 Hz $J_2$ = 2,6 Hz; Hf
3,20 à 3,40; multiplet; 2H; $CH_2$
3,44; triplet J = 6,5 Hz; 2H; Ha
3,58; triplet J = 9,7 Hz; 1H; Hg

- l'oxazolo [4,5-b] pyridin-2(3H)-one on obtient la 3-(4-bromobutyl) oxazolo [4,5-b] pyridin -2(3H) -one avec un rendement de 64 %.
  Infra Rouge (film) : 1775 $cm^{-1}$ $\nu$ C = 0
  RMN $^1H$ 300 MHz ($CDCl_3$) $\delta$ : ppm

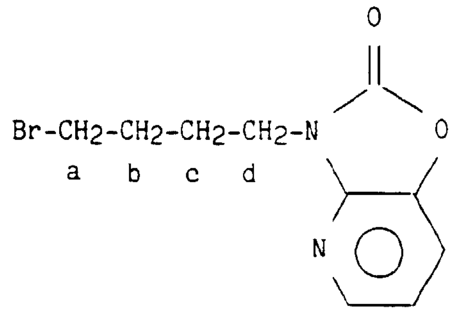

2,41 à 2,51; multiplet; 4H, Hb et Hc
3,47; triplet J = 6,7 Hz ; 2H ; Ha
4,10; triplet J = 6,7 Hz ; 2H ; Hd
7,08; doublet dédoublé $J_1$ = 8 Hz $J_2$ = 5,1 Hz ; 1H; pyridine
7,41; doublet J = 8 Hz ; 1H; pyridine
8,11; doublet J = 5,1 Hz, 1H; pyridine

**PREPARATION 10 : N-[(3-BROMOPROPYL)OXY]-8-AZASPIRO [4,5] DECANE-7,9-DIONE**

Ce composé est préparé, avec un rendement de 68 %, à partir de la 8-hydroxy -8-azaspiro [4,5] décane-7,9-dione selon la méthode décrite par Nicholas J. Hrib et al (J. Med.Chem. (1991) 34 1068)
Infra Rouge (film) : 1690 et 1740 $cm^{-1}$ ν C = 0
RMN $^1H$ 300 MHz ($CDCl_3$) δ : ppm

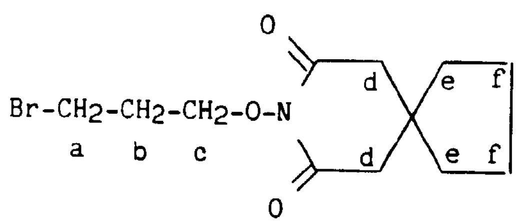

1,51 à 1,61; multiplet; 4H; 2 $CH_2$
1,68 à 1,78; multiplet; 4H; $CH_2$
2,21 à 2,28; multiplet; 2H; Hb
2,68; singulet; 4H; Hd
3,65; triplet J = 6,3 Hz; 2H; Ha
4,12; triplet J = 6,3 Hz; 2H; Hc

**EXEMPLE 1 : SPIRO [ (5-METHOXYCHROMANE)-3 : 2'-(PYRROLIDINE-5'-ONE)]**

STADE I : 3-(5-METHOXY 3-NITROCHROMAN-3-YL) PROPIONATE DE METHYLE

Ajouter 0,4 cm3 de méthylate de benzyl triméthyl ammonium et 5,16 cm3 d'acrylate de méthyle à une solution de 4 g (0,019 moles) de 5-méthoxy 3-nitro chromane dans 60 cm3 de méthanol.
Le milieu réactionnel est agité 90 minutes à 70°C, puis refroidi et concentré sous pression réduite.
Ajouter 30 cm3 d'eau et extraire au chlorure de méthylène.
Le produit brut obtenu par élimination du chlorure de méthylène est purifié par chromatographie sur colonne de silice (éluant : chlorure de méthylène 50/ éther de pétrole 50)
On obtient ainsi 5,1 g (90 %) de 3-(5-méthoxy 3-nitro chroman-3-yl) propionate de méthyle sous forme d'huile incolore.
Infra Rouge (film) : 1730 $cm^{-1}$ ν C = 0
RMN $^1H$ 300 MHz ($CDCl_3$) δ : ppm

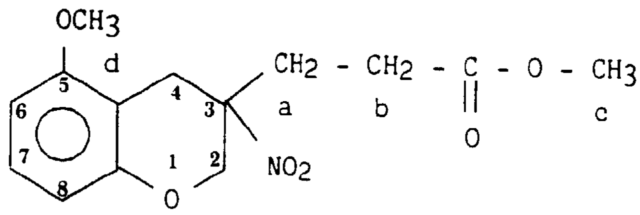

2,15 à 2,59; multiplet; 4H; Ha et Hb
2,91; doublet J = 17,5 Hz; 1H; $H_4$
3,57; doublet J = 17,5 Hz; 1H; $H_4$
3,68; singulet; 3H; Hc
3,84; singulet; 3H; Hd
4,10 et 4,57; 2 doublets J = 11,6 Hz; 2H; $H_2$
6,48 et 6,51; 2 doublets J = 8,3 Hz; 2H; H aromatiques
7,11; triplet J = 8,3 Hz; 1H; H aromatique

STADE II : SPIRO [ (5-METHOXYCHROMANE)-3 : 2'-(PYRROLIDINE-5'-ONE)]

Chauffer 20 heures à 60° C sous atmosphère d'hydrogène une solution de 5,1 g (0,017 moles) de 3-(5-méthoxy 3-nitro chromane-3-yl) propionate de méthyle dans 100 cm3 de méthanol en présence de 0,715 g de nickel de Raney.

Après élimination du catalyseur par filtration, la solution méthanolique est chauffée à reflux pendant 4 heures.

Après refroidissement et mise à sec, le produit brut obtenu est purifié par chromatographie sur colonne de silice (éluant : chlorure de méthylène 80/ méthanol 20)

On obtient ainsi 3,66 g (91 %) de spiro [ (5-méthoxychromane)-3 : 2'-(pyrrolidine-5'-one)] sous forme d'un solide blanc.

Point de fusion : 210° C

Infra Rouge (KBr) : 3250 $cm^{-1}$ $\nu$ NH 1670 $cm^{-1}$ $\nu$ C = 0

RMN $^1H$ 300 MHz ($CDCl_3$) $\delta$ : ppm

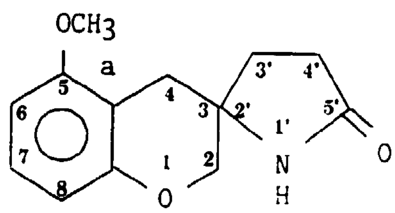

1,94 à 2,16; multiplet; 2H; $H_{3'}$
2,43 à 2,59;multiplet; 2H; $H_{4'}$
2,75 et 2,87; 2 doublets J = 16,5 Hz; $H_4$
3,83; singulet; 3H; Ha
3,89 et 3,94; 2 doublets J = 11 Hz; $H_2$
5,83; massif; 1H; NH
6,46 et 6,53; 2 doublets J = 8,3 Hz; 2H; H aromatiques
7,11; triplet J = 8,3 Hz; 1H; H aromatique

**EXEMPLE 2 : SPIRO [ CHROMANE-3 : 2'-(PYRROLIDINE-5'-ONE)]**

En procédant comme dans l'exemple 1 mais en remplaçant dans le stade I le 5-méthoxy 3-nitrochromane par le 3-nitrochromane on obtient le spiro [chromane-3 : 2'-(pyrrolidine-5'-one)] avec un rendement de 96 %

Point de fusion : 179° C

Infra Rouge (KBr) : 3225 $cm^{-1}$ ν NH 1675 $cm^{-1}$ ν C = 0

RMN $^1H$ 300 MHz ($CDCl_3$) δ : ppm

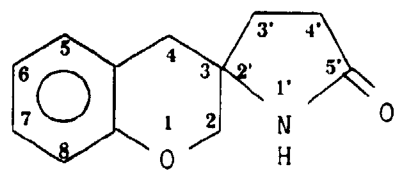

1,91 à 2,17; multiplet; 2H; $H_{3'}$
2,51; triplet J = 8,2 Hz; 2H; $H_{4'}$
2,88 et 2,97; 2 doublets J = 16,4 Hz; 2H; $H_4$
3,94 et 3,98; 2 doublets J = 10,3 Hz; 2H; $H_2$
6,04; massif; 1H; NH
6,83 à 7,18; multiplet; 4H; H aromatiques

**EXEMPLE 3 : SPIRO [(5-METHOXYCHROMANE)-3 : 2'-(N-PROPYL PYRROLIDINE-5'-ONE)]**

Ajouter 1 g (4,29 mmoles) de spiro [(5-méthoxychromane)-3 : 2'-( pyrrolidine-5'-one)] en solution dans 2 cm3 de DMF à une suspension de 0,113 g (4,71 mmoles) d'hydrure de sodium dans 18 cm3 de DMF.

Agiter une heure à 60°C, puis ajouter 3,61 g (0,021 mmoles) de 1-iodopropane. Maintenir le chauffage pendant 8 heures puis refroidir et éliminer le solvant sous pression réduite .

Ajouter 10 cm3 d'eau et extraire au chlorure de méthylène.

Le produit brut obtenu par mise à sec du chlorure de méthylène est purifié par chromatographie sur colonne de silice (éluant : éther éthylique 50/ chlorure de méthylène 50)

On obtient ainsi le spiro [(5-méthoxychromane)-3 : 2'-(N-propyl pyrrolidine-5'-one)] sous forme de cristaux blancs avec un rendement de 64%
Point de fusion : 90° C
Infra Rouge (KBr) : 1670 $cm^{-1}$ ν C = 0
RMN $^1H$ 300 MHz ($CDCl_3$) δ : ppm

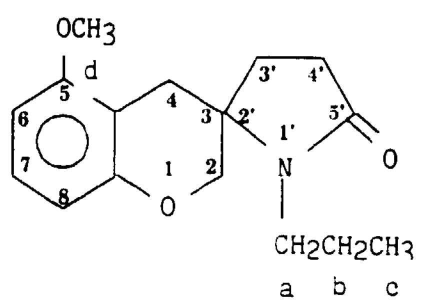

0,88; triplet J = 8,5 Hz; 3H; Hc
1,50 à 1,66; multiplet; 2H; Hb
1,74 à 1,84 et 2,07 à 2,16; 2 muitiplets; 2H; $H_{3'}$
2,44; triplet J = 8,1 Hz; 2H; $H_{4'}$
2,69; doublet de doublet J = 17,1 Hz; J = 2,4 Hz ; 1H; $H_4$
2,90; doublet J = 17,1 Hz; 1H; $H_4$
3 à 3,23; multiplet; 2H; $H_a$
3,84; singulet; 3H; $H_d$
3,88; doublet de doublet J = 10,3 Hz; J = 2,4 Hz; 1H; $H_2$
3,97; doublet J = 10,3 Hz; 1H; $H_2$
6,48 et 6,52; 2 doublets J = 8,3 Hz; 2H; H aromatiques
7,11; triplet J = 8,3 Hz; 1H; H aromatique

**EXEMPLE 4 : SPIRO [ (5-METHOXYCHROMANE)-3 : 2'-PYRROLIDINE]**

Ajouter 0,35 cm3 de complexe borane-diméthylsulfure (2M) à une solution de 0,1 g (0,42 mmoles) de spiro [ (5-méthoxychromane)-3 : 2'-(pyrrolidine-5'-one)] dans 5 cm3 de THF.

Chauffer le milieu réactionnel à reflux pendant 4 heures, mettre à sec sous pression réduite et reprendre le brut réactionnel par 10 cm3 d'acide chlorhydrique 2M et 5 cm3 de méthanol.

La solution est portée à reflux 90 minutes, puis basifiée avec une solution aqueuse de soude 2 M et extraite au chlorure de méthylène.

Après séchage, la phase chlorométhylénique est mise à sec et le produit brut purifié par chromatographie sur colonne de silice (éluant : chlorure de méthylène 95/ méthanol 5)

On obtient 70 mg (76 %) de spiro [ (5-méthoxychromane)-3 : 2'-pyrrolidine] sous forme d'huile.

Infra Rouge (film) : 3300 $cm^{-1}$ $\nu$ NH 1585 $cm^{-1}$ $\nu$ C = C aromatique

RMN $^1H$ 300 MHz ($CDCl_3$) $\delta$ : ppm

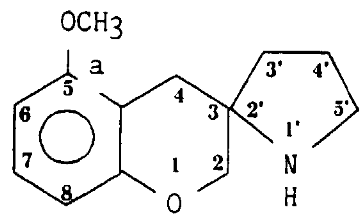

1,56 à 1,95; multiplet; 4H; $H_{4'}$ et $H_{3'}$
2,21; singulet; 1H; NH
2.68; singulet; 2H; $H_4$
2,96 à 3,17; multiplet; 2H; $H_{5'}$
3,80; singulet; 3H; Ha
3,82; singulet ; 2H; $H_2$
6,43 et 6,52; 2 doublets J = 8,3 Hz; 2H; H aromatiques
7,06; triplet J = 8,3 Hz; 1H; H aromatiques

**EXEMPLE 5 : SPIRO [ (5-METHOXYCHROMANE)-3 : 2'-(N-PROPYL PYRROLIDINE)]**

Ajouter 0,23 g (1,37 mmoles) de 1-iodopropane et 0,19 g (1,37 mmoles) de carbonate de potassium à une solution de 0,1 g (0,456 mmoles) de spiro [(5-méthoxychromane)-3 : 2'-pyrrolidine]

Chauffer à 60° C pendant 3 heures, refroidir, éliminer le solvant sous pression réduite, hydrolyser et extraire la phase aqueuse au chlorure de méthylène.

Après mise à sec le produit brut obtenu est purifié par chromatographie sur colonne de silice (éluant : éther éthylique 20/ éther de pétrole 80)

On obtient ainsi le spiro [(5-méthoxychromane)-3 : 2'-(N-propyl pyrrolidine)] avec un rendement de 84 %
Point de fusion (oxalate) : 62° C

Infra Rouge (film) : 2960 à 2800 $cm^{-1}$ $\nu$ CH 1585 $cm^{-1}$ $\nu$ C = C aromatique

RMN $^1H$ 300 MHz ($CDCl_3$) $\delta$ : ppm

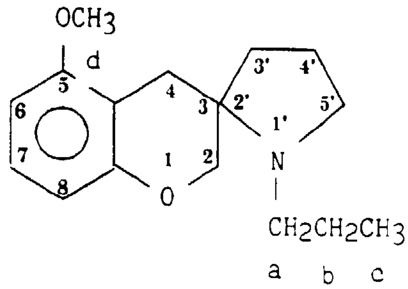

0,90; triplet; J = 7,4 Hz; 3H; Hc
1,42 à 1,97; multiplet; 6H; $H_{3'}$; $H_{4'}$; $H_b$
2,38 à 2,61; multiplet; 3H; $H_a$; $H_4$
2,70; doublet J = 17 Hz; 1H; $H_4$
2,82 à 3,03; multiplet; 2H; $H_{5'}$
3,78; doublet J = 10,3 Hz; 1H; $H_2$
3,81; singulet; 3H; $H_d$
3,83; doublet J = 10,3 Hz; 1H; $H_2$
6,42 et 6,48; 2 doublets J = 8,3 Hz; 2H; H aromatiques
7,06; triplet J = 8,3 Hz; 1H; H aromatiques

**EXEMPLE 6 : SPIRO [ CHROMANE-3 : 2'-(N-PROPYL PYRROLIDINE)]**

En procédant comme dans l'exemple 5 mais en remplaçant le spiro méthoxychromane-3 : 2'-pyrrolidine] par le spiro [chromane-3 : 2'-(N-propyl pyrrolidine)] on obtient le spiro [chromane-3 : 2'-(N-propyl pyrrolidine)] avec un rendement de 61 %
Point de fusion (oxalate) : 48° C

Infra Rouge (film) : 2800 à 2980 $cm^{-1}$ ν CH 1575 $cm^{-1}$ ν C = C aromatiques

RMN $^1H$ 300 MHz ($CDCl_3$) δ : ppm

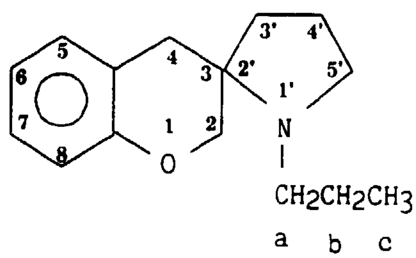

0,91; triplet; J = 7,4 Hz; 3H; $H_c$
1,43 à 2,01; multiplet; 6H; $H_b$; $H_{3'}$; $H_{4'}$
2,39 à 2,59; multiplet; 3H; $H_a$; $H_4$
2,83 à 2,96; multiplet; 2H; $H_{5'}$
3,02;doublet J = 16,1 Hz ; 1H; $H_4$
3,83; doublet de doublet J = 10,3 Hz J = 2,4 Hz; 1H; $H_2$
3,88; doublet J = 10,3 Hz; 1H; $H_2$
6,78 à 7,11; multiplet ; 4H; H aromatiques

**EXEMPLE 7 : SPIRO [(5-HYDROXY CHROMANE)-3 : 2'-(N-PROPYL PYRROLIDINE)]**

Ajouter 11 cm3 d'acide bromhydrique à 48 % dans l'eau à une solution de 1,1 g (4,2 mmoles) de spiro [(5-méthoxy chromane)-3 : 2'-(N-propyl pyrrolidine)] dans 22 cm3 d'acide acétique.

Le milieu réactionnel est chauffé 5 heures à reflux puis refroidi, mis à sec sous pression réduite, repris par 40 cm3

d'une solution aqueuse saturée de bicarbonate de sodium et extrait au chlorure de méthylène.

Le produit brut obtenu par mise à sec du chlorure de méthylène est purifié par chromatographie sur colonne de silice (éluant : éther éthylique 50/ éther de pétrole 50)

On obtient ainsi 0,91 g (88 %) de spiro [(5-hydroxy chromane)-3 : 2'-(N-propyl pyrrolidine)]

Point de fusion (base) : 185-186° C

Point de fusion (sel d'oxalate) : 98° C

Infra Rouge (KBr) : 3250 $cm^{-1}$ (large bande) ν OH

RMN $^1H$ 300 MHz (DMSO $d_6$) δ : ppm

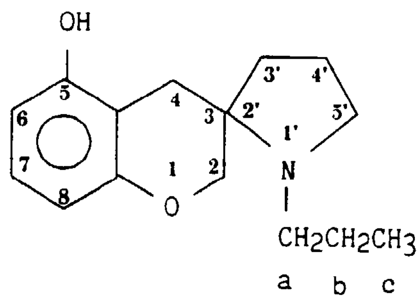

0,83; triplet J = 7,4 Hz; 3H; $H_c$

1,30 à 1,45; multiplet; 3H; $H_b$; $H_{3'}$

1,65 à 1,80; multiplet; 3H; $H_{3'}$; $H_{4'}$

2,32;doublet J = 17 Hz ; 1H; $H_4$

2,39 à 2,53;multiplet ; 2H; $H_a$

2,59; doublet J = 17 Hz; 1H; $H_4$

2,71 à 2,90; multiplet ; 2H; $H_{5'}$

3,69 et 3,76; 2 doublets J = 10,3 Hz; 2H; $H_2$

6,21 et 6,32; 2 doublets J = 8,3 Hz; 2H; H aromatiques

6,81; triplet J = 8,3 Hz; 1H; H aromatique

9,32; singulet; 1H; 0H

**EXEMPLE 8 : SPIRO [(5-METHOXY CHROMANE)-3 : 2'-{N-[4'-(8'-AZASPIRO [4',5'] DECANE-7',9'-DION-8'-YL) nBUT-1'-YL] PYRROLIDINE}]**

Ajouter 1,82 g (6,02 mmoles) de N-(4-bromobut-1-yl) 8-azaspiro [4,5] décane-7,9-dione, 1,66 g (16,41 mmoles) de triéthylamine et une quantité catalytique d'iodure de potassium à une solution de 1,2 g (5,47 mmoles) de spiro [(5-méthoxy chromane)-3 : 2'- pyrrolidine] dans 10 cm3 de N,N-diméthyl formamide.

Le milieu réactionnel est chauffé 8 heures à 60°C, puis refroidi, concentré sous pression réduite, repris par 10 cm3 d'eau et extrait au chlorure de méthylène.

L'huile résiduelle obtenue par mise à sec est purifiée par chromatographie sur colonne de silice (éluant : chlorure de méthylène 50/ éther éthylique 50)

On obtient ainsi 1,3 g (54 %) de spiro [(5-méthoxy chromane)-3 : 2'-{N-[4'-(8'-azaspiro [4',5'] décane-7',9'-dion-8'-yl)nbut-1'-yl] pyrrolidine}]

Point de fusion (oxalate) : 68° C

Infra Rouge (film) : 1665 et 1720 $cm^{-1}$ ν C = 0

RMN $^1H$ 300 MHz ($CDCl_3$) δ : ppm

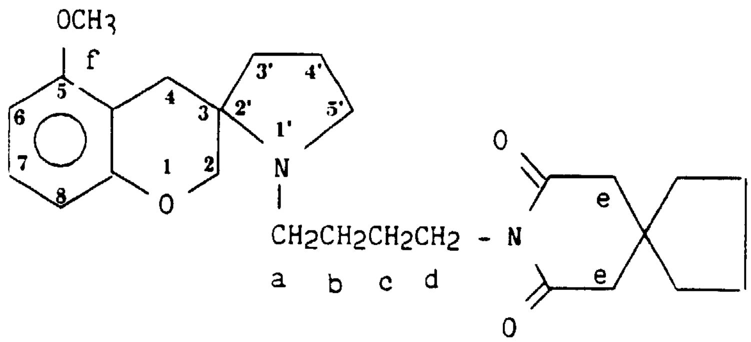

1,35 à 1,95; multiplet; 16H; 8 $CH_2$
2,40 à 2,56; multiplet; 3H; $H_4$ et $H_a$
2,57; singulet; 4H; $H_e$
2,66; doublet J = 16,8 Hz ; 1H; $H_4$
2,76 à 3,03; multiplet ; 2H; $H_{5'}$
3,69 à 3,79; multiplet ; 4H; $H_d$ et $H_2$
3,81; singulet ; 3H; $H_f$
6,41 et 6,46; 2 doublets J = 8,3 Hz; 2H; H aromatiques
7,04; triplet J = 8,3 Hz; 1H; H aromatique

**EXEMPLE 9 : SPIRO [CHROMANE-3 : 2'-{N-[4'-(8'-AZASPIRO [4',5'] DECANE-7',9'-DION-8'-YL)nBUT-1'-YL] PYRROLIDINE)]**

En procédant comme dans l'exemple 8, mais en remplaçant le spiro [(5-méthoxy chromane)-3 : 2'- pyrrolidine] par le spiro [ chromane-3 : 2'-pyrrolidine] on obtient le spiro [chromane-3 : 2'-{N-[4'-(8'-azaspiro [4',5'] décane-7',9'-dion-8'-yl)nbut-1'-yl] pyrrolidine}] avec un rendement de 55 %
Point de fusion (oxalate) : 66° C
Infra Rouge (film) : 1715 et 1660 $cm^{-1}$ $\nu$ C = 0
RMN $^1H$ 300 MHz ($CDCl_3$) $\delta$ : ppm

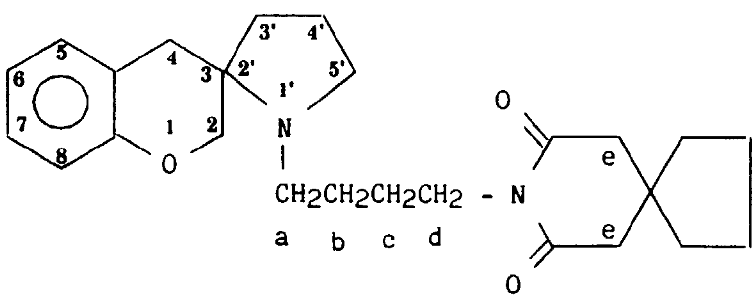

1,40 à 2; multiplet; 16H; 8 $CH_2$
2,44; doublet J = 16,1 Hz; 1H; $H_4$
2,48 à 2,56; multiplet; 2H; Ha
2,58; singulet; 4H; He
2,79 à 2,96; multiplet ; 2H; $H_{5'}$
3,0; doublet J = 16,1 Hz; 1H; $H_4$
3,73 à 3,91; multiplet ; 4H; $H_2$ et Hd
6,77 à 7,10; multiplet; 4H; H aromatiques

**EXEMPLE 10 : SPIRO [{5-[4-(8-AZASPIRO [4,5] DECANE-7,9-DION-8-YL) nBUT-1 -YL]OXY CHROMANE}-3 : 2'-(N-PROPYL PYRROLIDINE)]**

Ajouter 0,076 g (0,25 mmole) de N-(4-bromobut-1-yl)-8-azaspiro [4,5] décane-7,9-dione, 0,095 g (0,69 mmole) de

carbonate de potassium et une quantité catalytique d'iodure de potassium à une solution de 0,058 g (0,23 mmole) de spiro [(5-hydroxy chromane)-3 : 2'-(N-propyl pyrrolidine)] dans 3 cm3 de N,N-diméthyl formamide.

Chauffer 2 heures à 60° C, refroidir, concentrer sous pression réduite, puis reprendre le brut réactionnel dans 10 cm3 d'eau et extraire au chlorure de méthylène.

L'huile brute obtenue est purifiée par chromatographie sur colonne de silice (éluant : éther éthylique 50 / chlorure de méthylène 50).

On obtient ainsi le spiro [{5-[4-(8-azaspiro [4,5] décane-7,9-dion-8-yl) nbut-1-yl]oxy chromane}-3 : 2'-(N-propyl pyrrolidine)] sous forme d'huile avec un rendement de 79 %.

Point de fusion (oxalate) : 68° C

Infra Rouge (film) : 1660 et 1715 $cm^{-1}$ $\nu$ C = 0

RMN $^1H$ 300 MHz ($CDCl_3$) $\delta$ : ppm

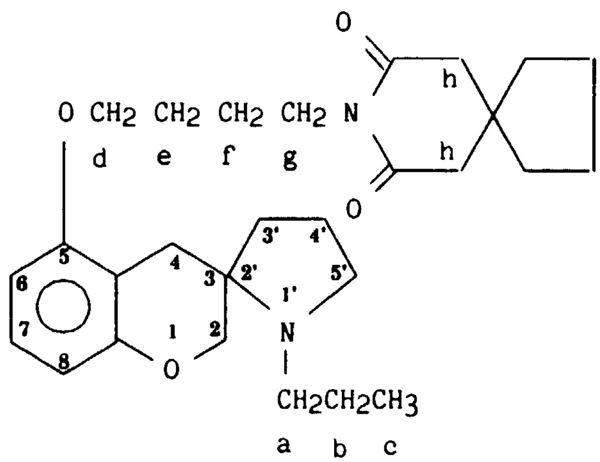

0,94; triplet J = 7,4 Hz; 3H; $H_c$
1,48 à 2,03; multiplet; 18H; 9 $CH_2$
2,40 à 2,68; multiplet; 7H; $H_h$; $H_4$ et $H_a$
2,74; doublet J = 17 Hz; 1H; $H_4$
2,90 à 3,06; multiplet ; 2H; $H_{5'}$
3,82 à 3,92; multiplet; 4H; $H_g$ et $H_2$
3,95 à 4,04; multiplet ; 2H; $H_d$
6,41 et 6,48; 2 doublets J = 8,3 Hz; 2H; H aromatiques
7,04; triplet J = 8,3 Hz; 1H; H aromatique

**EXEMPLES 11 ET 12 :**

En procédant de la même façon que pour l'exemple 10 mais en remplaçant la N-(4-bromo nbut-1-yl)-8-azaspiro [4,5] décane-7,9-dione par :

- la N-(4-bromo nbut-1-yl) 4,4 diméthyl pipéridine-2,6-dione on obtient le spiro[{5-[4-(4,4-diméthyl pipéridine-2,6-dione-1-yl) nbut-1-yl]oxy chromane}-3 : 2'-(N-propyl pyrrolidine)]

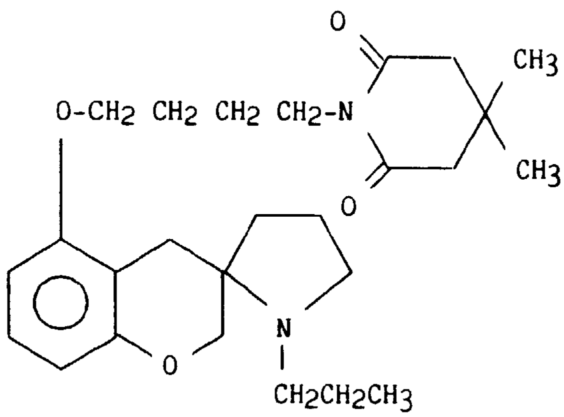

- la N-(4-bromo nbut-1-yl)-3-azabicyclo [3.3.0] octane-2,4-dione on obtient le spiro[{5-[4-(3-azabicyclo [3.3.0] octane -2,4-dione-3-yl) nbut-1-yl]oxy chromane}-3 ; 2'-(N-propyl pyrrolidine)]

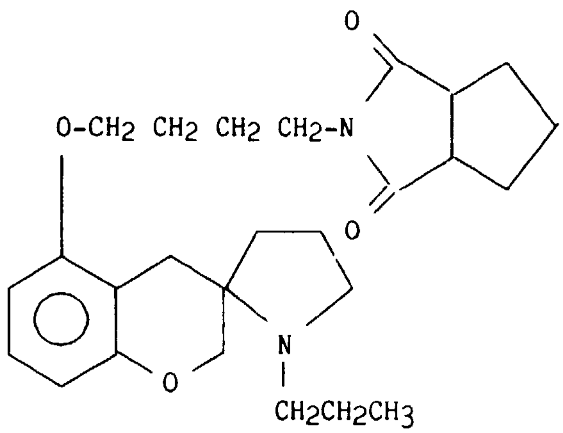

**EXEMPLES 13 A 32**

En procédant de la même façon que pour l'exemple 8, mais en remplaçant la N-(4-bromobut-1-yl)-8-azaspiro [4,5] décane-7,9-dione par :

- le N-(4-bromobutyl) phtalimide, on obtient le spiro [(5-méthoxy chromane)-3 : 2'-[N-(4'-phtalimido nbut-1'-yl) pyrrolidine]]

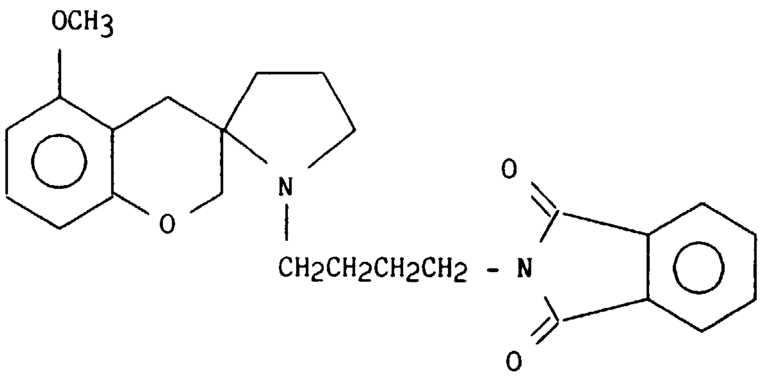

Infra Rouge (film) : 1700 $cm^{-1}$ ν C = 0

- le N-(3-bromopropyl) phtalimide, on obtient le spiro [(5-méthoxy chromane)-3 : 2'-[N-(3'-phtalimido prop-1'-yl) pyrrolidine]]
- la N-(3-bromoprop-1-yl)-8-azaspiro [4,5] décane-7,9-dione, on obtient le spiro [(5-méthoxy chromane)-3 : 2'-{N-[3'-(8'-azaspiro[4',5'] décane-7',9'-dion-8'-yl) nprop-1'-yl] pyrrolidine]]

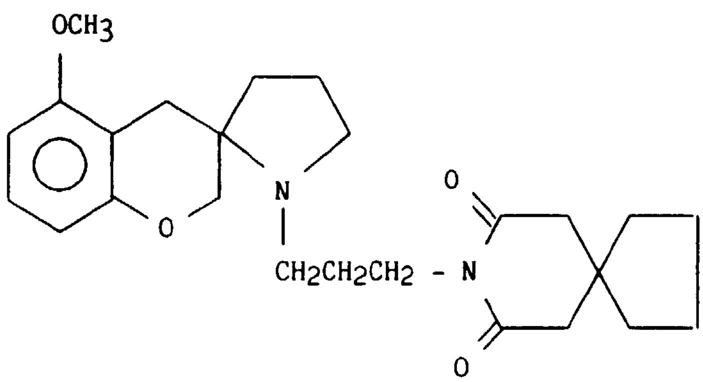

- la N-(5-bromopent-1-yl)-8-azaspiro [4,5] décane-7,9-dione, on obtient le spiro [(5-méthoxy chromane)-3 : 2'-{N-[5'-(8'-azaspiro[4',5'] décane-7',9'-dion-8'-yl) npent-1'-yl] pyrrolidine)]

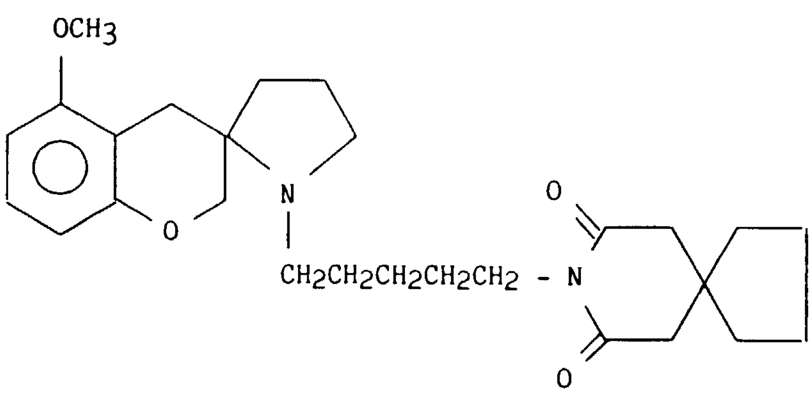

- la N-(2-bromoéthyl)-8-azaspiro [4,5] décane-7,9-dione, on obtient le spiro [(5-méthoxy chromane)-3 : 2'-{N-[2'-(8'-azaspiro[4',5'] décane-7',9'-dion-8'-yl) éthyl] pyrrolidine)]

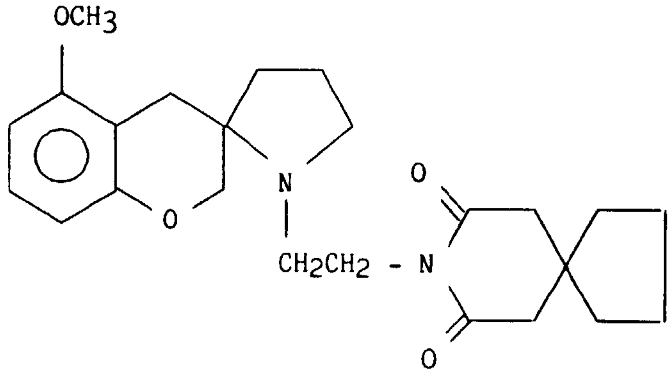

- la N-[(3-bromopropyl)oxy]-8-azaspiro [4,5] décane-7,9-dione, on obtient le spiro [(5-méthoxy chromane)-3 : 2'-{N-[3'-(8'-azaspiro[4',5'] décane-7',9'-dion-8'-yl)oxy nprop-1-yl] pyrrolidine}]

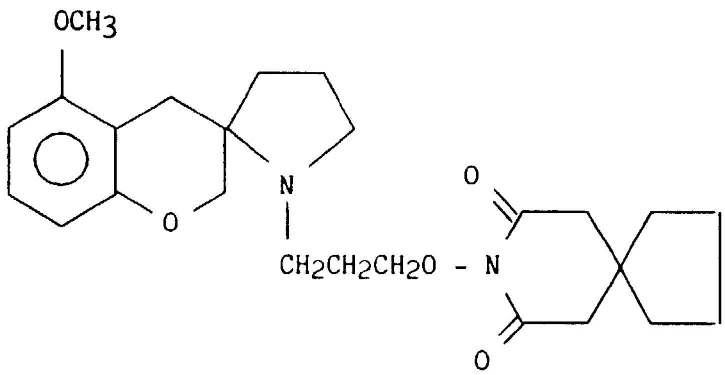

- la N-(4-bromobutyl) oxazolo [4,5-b] pyridine-2(3H)-one on obtient le spiro [(5-méthoxy chromane)-3 : 2'-{N-[4'-(oxazolo[4',5'-b]pyridine-2'(3H)-one-3'-yl)nbut-1'-yl] pyrrolidine}]

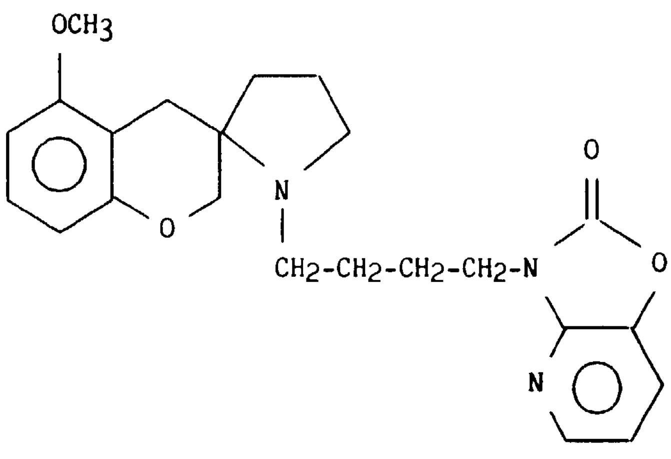

- la N-(4-bromobutyl) oxazolo [5,4-b] pyridine-2(3H)-one on obtient le spiro [(5-méthoxy chromane)-3 : 2'-{N-[4'-(oxazolo[5-b]pyridine-2'(3H)-one-3'-yl)nbut-1'-yl] pyrrolidine}]

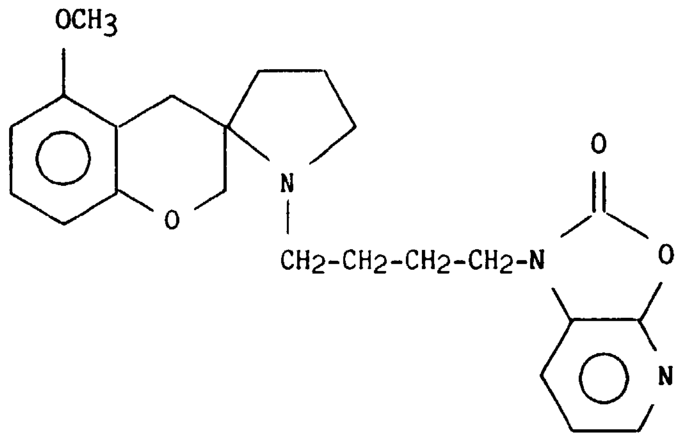

- la N-(4-bromobutyl) 3-azabicyclo [3.3.0] octane-2,4-dione, on obtient le spiro [(5-méthoxy chromane)-3 : 2'-{N-[4'-(3'-azabicyclo[3.3.0] octane-2,4-dione-3-yl) nbut-1'-yl] pyrrolidine}]

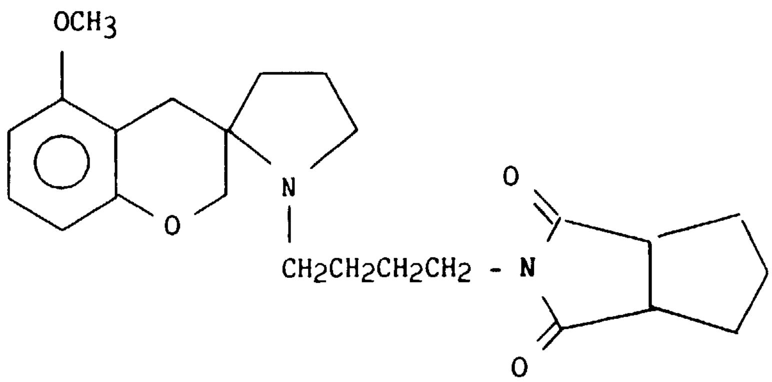

- la N-(4-bromobutyl) 4,4-diméthyl pipéridine-2,6-dione, on obtient le spiro [(5-méthoxy chromane)-3 : 2'-{N-[4'-(4', 4'-diméthyl pipéridine-2',6'-dione-1'-yl) nbut-1'-yl]pyrrolidine}]

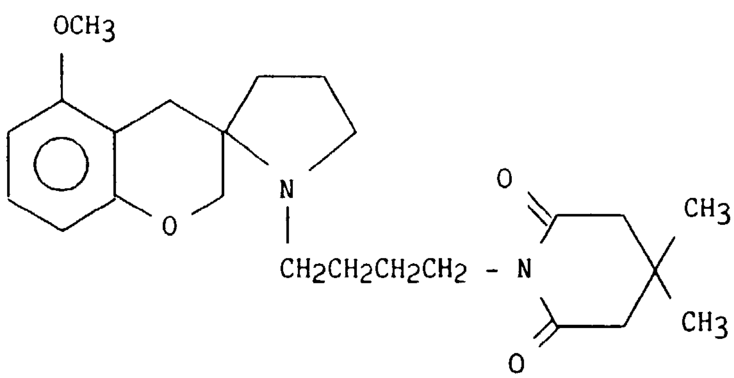

- la N-(4-bromobutyl) benzoxazoline-2-one, on obtient le spiro [(5-méthoxy chromane)-3 : 2'-{N-[4'-(benzoxazoline-2'-one-3'-yl) nbut-1'-yl] pyrrolidine}]

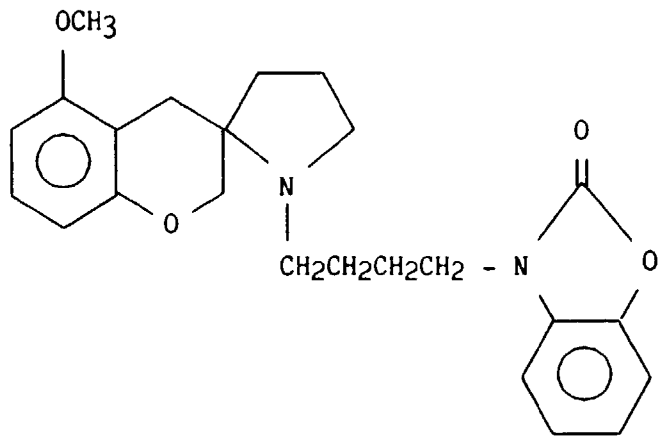

- la N-(4-bromobutyl) 1,1-dioxo- 1,2-benzisothiazol-3 (2H)-one, on obtient le spiro [(5-méthoxy chromane)-3 : 2'-{N-[4'-(1',1'-dioxo 1',2'-benzisothiazol-3'(2H)-one-2'-yl) nbut-1'-yl] pyrrolidine}]

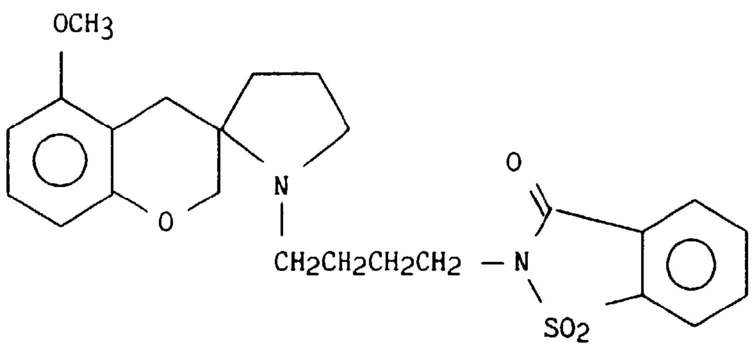

- la N-(4-bromobutyl) 3-azabicyclo [3.3.0] octane-2-one, on obtient le spiro [(5-méthoxy chromane)-3 : 2'-{N-[4'-(3'-azabicyclo [3.3.0] octane-2'-one-3'-yl)nbut-1'-yl] pyrrolidine}]

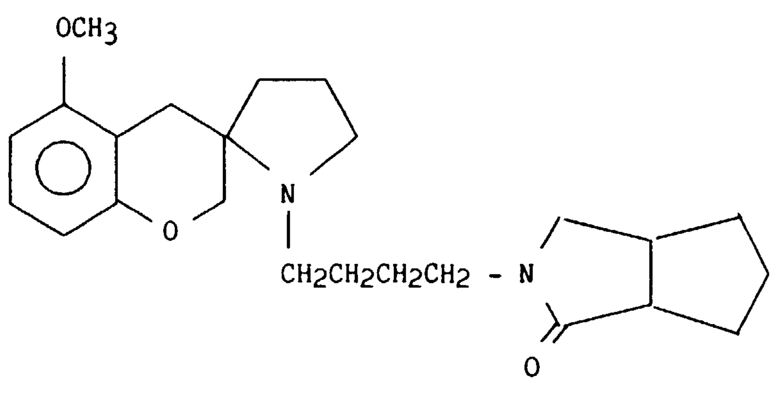

- la N-(4-bromobut-1-yl)pipéridin-2-one on obtient le spiro [(5-méthoxy chromane)-3 : 2'-{N-[4'-(pipéridin-2'-on-1'-yl) nbut-1'-yl] pyrrolidine}]

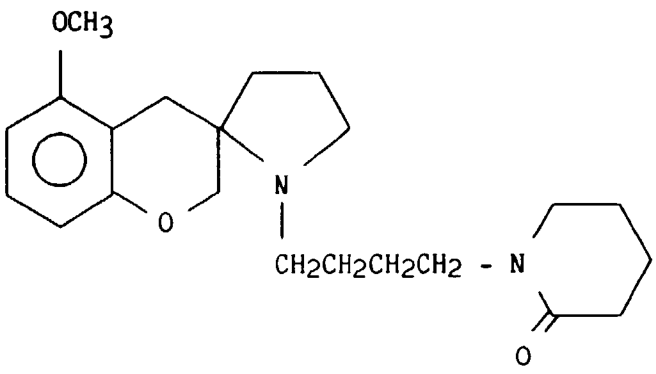

- le bromure de benzyle, on obtient le spiro [(5-méthoxy chromane)-3 : 2'-(N-benzyl pyrrolidine)]

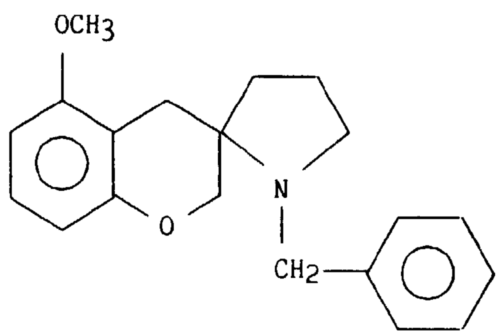

- le bromure de 3,4-difluorobenzyl on obtient le spiro [(5-méthoxy chromane)-3 : 2'-[N-(3',4'-difluorobenzyl) pyrrolidine]]
- le bromure de 4-trifluorométhyl benzyl on obtient le spiro [(5-méthoxy chromane)-3 : 2'-[N-(4'-trifluorométhyl benzyl) pyrrolidine]]
- le bromure de 4-méthyl benzyl on obtient le spiro[(5-méthoxy chromane)-3 : 2'-[N-(4'-méthyl benzyl)pyrrolidine]]
- le bromure de phénéthyl on obtient le spiro [(5-méthoxy chromane)-3 : 2'-(N-phénéthyl pyrrolidine)]
- le 3-bromopropylbenzène on obtient le spiro [(5-méthoxy chromane)-3 : 2'-[N-(3'-phényl nprop-1'-yl)pyrrolidine]]

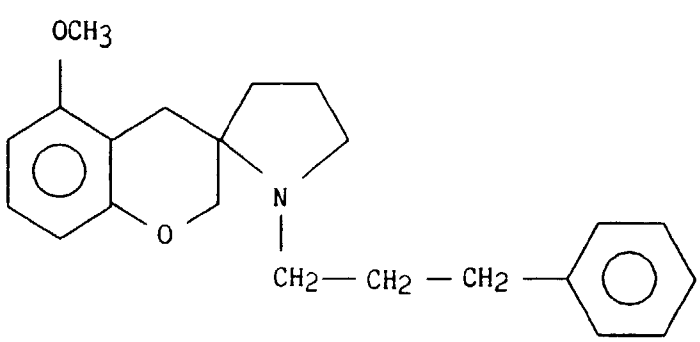

**EXEMPLE 33 : SPIRO [(5-METHOXY CHROMANE)-3 : 2'-[N-(2'-AMINO ETHYL) PYRROLIDINE]]**

STADE I : SPIRO [(5-METHOXY CHROMANE)-3 : 2'-(N-CYANOMETHYL PYRROLIDINE)]

Additionner 0,905 g (12 mmoles) de chloroacétonitrile, 1,66 g (12 mmoles) de carbonate de potassium et une quantité catalytique d'iodure de potassium à une solution de 0,89 g (4,05 mmoles) de spiro [(5-méthoxy chromane)-3 : 2'-pyrrolidine].

Chauffer 20 heures à 60° C puis refroidir, concentrer sous pression réduite, reprendre à l'eau, puis extraire au chlorure de méthylène.

Le produit brut obtenu après mise à sec de la phase chlorométhylénique est purifié par chromatographie sur colonne de silice (éluant : chlorure de méthylène 99/ méthanol 1)

On obtient ainsi le spiro [(5-méthoxy chromane)-3 : 2'-(N-cyanométhyl pyrrolidine)] avec un rendement de 75 %

STADE II : SPIRO [(5-METHOXY CHROMANE)-3 : 2'-[N-(2'-AMINOETHYL) PYRROLIDINE]]

Additionner lentement 5,6 mmoles de $LiAlH_4$ à une solution sous atmosphère d'argon de 0,72 g (2,8 mmoles) de spiro [(5-méthoxy chromane)-3 : 2'-(N-cyanométhyl pyrrolidine)] dans 20 cm3 de tétrahydrofurane.

Agiter 30 minutes à température ambiante, puis refroidir avec de la glace et hydrolyser avec 7 cm3 d'eau glacée.

La phase organique est séparée, mise à sec et le produit brut obtenu purifié par chromatographie sur colonne de silice (éluant : chlorure de méthylène 99/ méthanol 1)

On obtient ainsi le spiro [(5-méthoxy chromane)-3 : 2'-[N-(2'-aminoéthyl) pyrrolidine]] avec un rendement de 70 %.
RMN $^1H$ 300 MHz ($CDCl_3$) $\delta$ : ppm

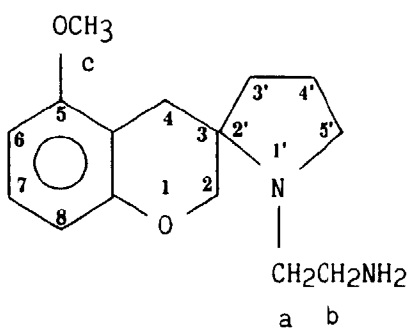

1,45 à 1,90; multiplet; 4H; $H_{3'}$ et $H_{4'}$
2,41 à 2,75; multiplet; 6H; $H_4$; $H_a$; $H_b$
2,95; multiplet ; 2H; $H_{5'}$
3,80 à 3,85; multiplet singulet; 7H; $H_c$ ; $H_2$ et $NH_2$
6,40 et 6,5; 2 doublets J = 8 Hz; 2H; H aromatiques
7,05; triplet J = 8 Hz; 1H ; H aromatique

**EXEMPLE 34 : SPIRO [(5-METHOXY CHROMANE)-3 : 2'-[N-(4'-AMINO nBUT-1'-YL) PYRROLIDINE]]**

STADE I : SPIRO [(5-METHOXY CHROMANE)-3 : 2'-[N-(3'-CYANO nPROP-1'-YL) PYRROLIDINE]]

En procédant comme pour l'exemple 33 stade I mais en remplaçant le chloroacétonitrile par le 4-bromo butyronitrile on obtient le spiro [(5-méthoxy chromane)-3 : 2'-[N-(3'-cyano nprop-1'-yl) pyrrolidine]]

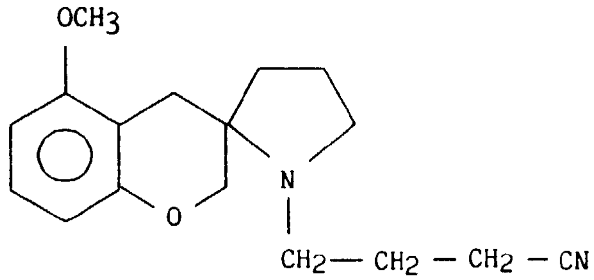

STADE II : SPIRO [(5-METHOXY CHROMANE)-3 : 2'-[N-(4'-AMINO nBUT-1'-YL) PYRROLIDINE]]

En réduisant le spiro [(5-méthoxy chromane)-3 : 2'-[N-(3-cyano nprop-1-yl) pyrrolidine]] dans les conditions de l'exemple 33 stade II on obtient le spiro [(5-méthoxy chromane)-3 : 2'-[N-(4'-amino nbut-1'-yl) pyrrolidine]]

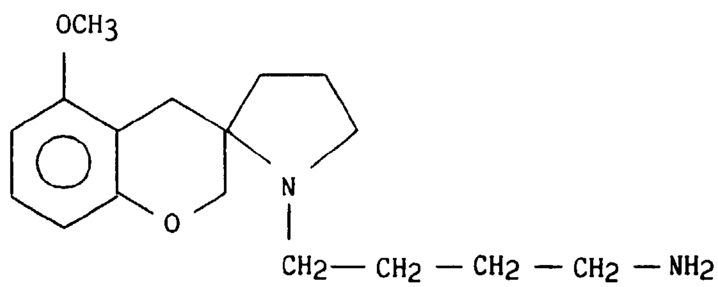

**EXEMPLE 35 : SPIRO [(5-METHOXY CHROMANE)-3 : 2'-[N-(2'-PARATOLUENE SULFONYLAMINO ETHYL) PYRROLIDINE]]**

Additionner goutte à goutte 1,2 g (11,4 mmoles) de triéthylamine puis 0,8 g (5,8 mmoles) de chlorure de tosyle en solution dans du chlorure de méthylène à une solution à 0°C de 1g (3,8 mmoles) de spiro [(5-méthoxy chromane)-3 : 2'-[N-(2'-aminoéthyl) pyrrolidine]] dans 30 cm3 de chlorure de méthylène.

Après 30 minutes d'agitation à température ambiante, le solvant est éliminé et le produit brut obtenu purifié par chromatographie sur colonne de silice (éluant : chlorure de méthylène).

On obtient ainsi le spiro [(5-méthoxy chromane)-3 : 2'-[N-(2'-paratoluènesulfonylamino éthyl) pyrrolidine]] avec un rendement de 75 %.

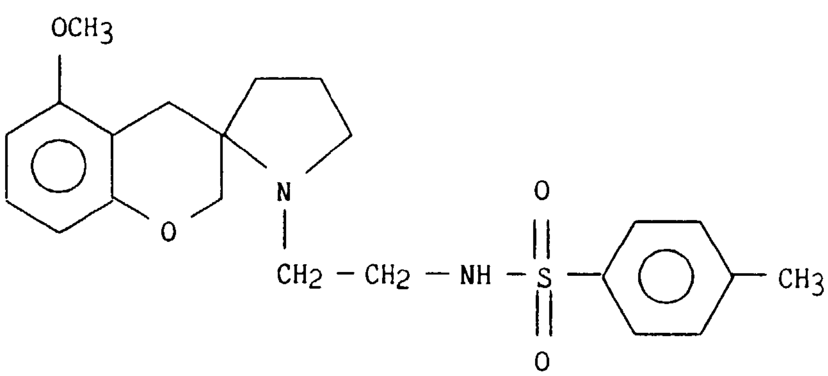

Infra Rouge (KBr) : 1150 $cm^{-1}$ $\nu$ $SO_2$

**EXEMPLE 36 : SPIRO [(5-METHOXY CHROMANE)-3 2'-[N-(4'-PARATOLUENE SULFONYLAMINO nBUT-1'-YL) PYRROLIDINE]]**

En procédant comme pour l'exemple 35 mais en remplaçant le spiro [(5-méthoxy chromane)-3 : 2'-[N-(2'-aminoéthyl) pyrrolidine]] ] par le spiro [(5-méthoxy chromane)-3 : 2'-[N-(4'-amino nbut-1'-yl) pyrrolidine]] on obtient le spiro [(5-méthoxy chromane)-3 : 2'-[N-(4'-paratoluène-sulfonylamino nbut-1'-yl) pyrrolidine]]

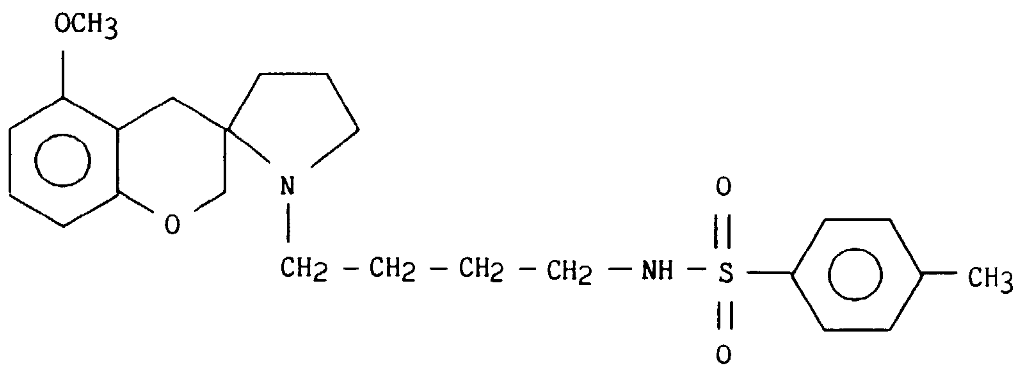

Infra Rouge (KBr) : 1150 $cm^{-1}$ ν $SO_2$

**EXEMPLE 37 à 42**

En procédant de la même façon que pour l'exemple 36 mais en remplaçant le chlorure de tosyle par :

- le chlorure de l'acide 4-iodo-benzoique on obtient le spiro [(5-méthoxy chromane)-3 : 2'-{N-[4'-(4'-iodo-benzamido) nbut-1'-yl] pyrrolidine}]

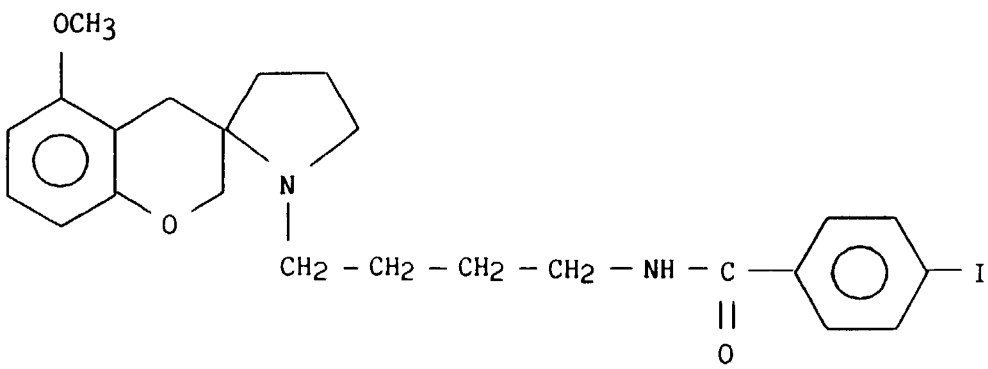

Infra Rouge : 1620 $cm^{-1}$ νC = 0

- le chlorure de l'acide 4-fluorobenzoique on obtient le spiro [(5-méthoxy chromane)-3 : 2'-{N-[4'-(4'-fluorobenzamido) nbut-1'-yl] pyrrolidine}]
  Infra Rouge : 1630 $cm^{-1}$ νC = 0
- le chlorure de l'acide 2-méthoxybenzoique on obtient le spiro [(5-méthoxy chromane)-3 : 2'-{N-[4'-(2'-méthoxybenzamido) nbut-1'-yl] pyrrolidine}]
  Infra Rouge : 1635 $cm^{-1}$ νC = 0
- le chlorure de l'acide butanoique on obtient le spiro [(5-méthoxy chromane)-3 : 2'-[N-(4'-butyramido nbut-1'-yl) pyrrolidine]]
- le chlorure de l'acide 3-phényl propionique on obtient le spiro [(5-méthoxy chromane)-3 : 2'-{N-[4'-(3'-phénylpropionamido) nbut-1'-yl] pyrrolidine}]

- le chlorure de l'acide 3,4-diméthoxyphénylacétique on obtient le spiro [(5-méthoxy chromane)-3 : 2'-{N-[4'-(3-diméthoxyphénylacétamido) nbut-1'-yl] pyrrolidine}]

**EXEMPLE 43 : SPIRO [(5-METHOXY CHROMANE)-3 : 2'-(N-ACETYL PYRROLIDINE)]**

Additionner 0,253 g (2,51 mmoles) de triéthylamine, puis goutte à goutte, 0,256 g (2,51 mmoles) d'anhydride acétique à une solution de 0,5 g (2,28 mmoles) de spiro [(5-méthoxy chromane)-3 : 2'- pyrrolidine] dans 10 cm3 de chlorure de méthylène.

Agiter 30 minutes à température ambiante, puis concentrer sous pression réduite, reprendre le produit brut par 10 cm3 d'eau et extraire au chlorure de méthylène.

L'huile brute obtenue est purifiée par chromatographie sur colonne de silice (éluant : acétate d'éthyle).

On obtient ainsi 0,57 g (96 %) de spiro [(5-méthoxy chromane)-3 : 2'-(N-acétyl pyrrolidine)] sous forme d'un solide blanc.

Point de fusion : 121° C

Infra Rouge (KBr) : 1640 $cm^{-1}$ $\nu$ C = 0

RMN $^1H$ 300 MHz ($CDCl_3$) $\delta$ : ppm

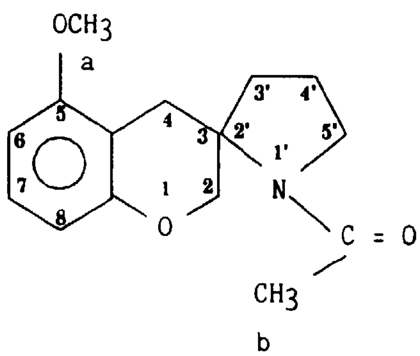

1,78 à 2,04; multiplet; 4H; $H_{3'}$ et $H_{4'}$
2,06; singulet; 3H; $H_b$
2,52; doublet de doublet J = 16,6 Hz; J = 2,6 Hz; 1H; $H_4$
3,50 à 3,63; multiplet; 2H; $H_{5'}$
3,67; doublet J = 16,6 Hz; ; 1H; $H_4$
3,76; doublet de doublet J = 10,2 Hz; J = 2,6 Hz; 1H; $H_2$
3,80; singulet ; 3H; $H_a$
4,96; doublet J = 10,2 Hz; 1H; $H_2$
6,42 et 6,50; 2 doublets J = 8,3 Hz; 2H; H aromatiques
7,06; triplet J = 8,3 Hz; 1 H; H aromatique

**EXEMPLE 44 : SPIRO [(5-METHOXY CHROMANE)-3 : 2'-(N-BENZOYL PYRROLIDINE)]**

En procédant de la même façon que pour l'exemple 35, mais en remplaçant le chlorure de tosyle par le chlorure de benzoyle et le spiro [(5-méthoxy chromane)-3 : 2'-[N-(2'-aminoéthyl) pyrrolidine]] par le spiro [(5-méthoxy chromane)-3 : 2'-pyrrolidine)] on obtient le spiro [(5-méthoxy chromane)-3 : 2'-(N-benzoyl pyrrolidine)].

**EXEMPLES 45 à 51 :**

En procédant de la même façon que pour l'exemple 44 mais en remplaçant le chlorure de benzoyl par :

- le chlorure de l'acide 4-méthoxy benzoique on obtient le spiro [(5-méthoxy chromane)-3 : 2'-[N-(4'-méthoxy benzoyl) pyrrolidine]]

- le chlorure de l'acide 4-iodo benzoique on obtient le spiro [(5-méthoxy chromane)-3 : 2'-[N-(4'-iodo benzoyl) pyrrolidine]]

- le chlorure de l'acide 3-trifluorométhyl benzoique on obtient le spiro [(5-méthoxy chromane)-3 : 2'-[N-(3'-trifluorométhyl benzoyl) pyrrolidine]]

- le chlorure de l'acide 4-méthyl benzoique on obtient le spiro [(5-méthoxy chromane)-3 : 2'-[N-(4'-méthyl benzoyl) pyrrolidine]]

- le chlorure de l'acide butyrique on obtient le spiro [(5-méthoxy chromane)-3 : 2'-(N-butyryl pyrrolidine)]

- le chlorure de l'acide 3-phényl propionique on obtient le spiro [(5-méthoxy chromane)-3 : 2'-[N-(3'-phényl propionyl) pyrrolidine]]

- le chlorure de l'acide 3,4-diméthoxy phénylacétique on obtient le spiro [(5-méthoxy chromane)-3 : 2'-[N-(3',4'-diméthoxy phénylacétyl) pyrrolidine]]

**EXEMPLES 52 à 53 :**

En procédant de la même façon que pour l'exemple mais en remplaçant le spiro [(5-méthoxy chromane)-3 : 2'-(pyrrolidine-5'-one)] par :

- le spiro [(5-méthoxy chromane)-3 : 2'-[N-(4'-butyramido nbut-1'-yl)pyrrolidine]] on obtient le spiro [(5-méthoxy chromane)-3 : 2'-{N-[4-(N-nbut-1-yl)amino nbut-1-yl]pyrrolidine}]

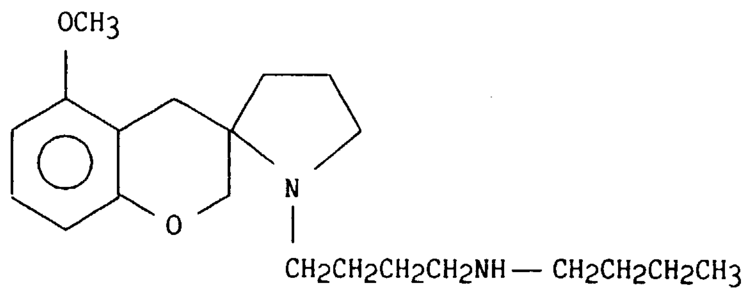

- le spiro [(5-méthoxy chromane)-3 : 2'-{N-[4-(3,4-diméthoxyphénylacétamido)nbut-1-yl]pyrrolidine}] on obtient le spiro [(5-méthoxy chromane)-3 : 2'-(N-[4'-(3-diméthoxy phénéthyl)amino nbut-1'-yl]pyrrolidine}]

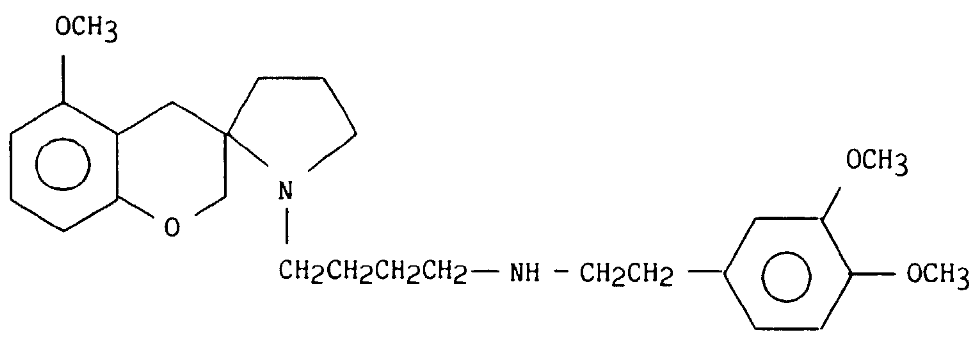

**EXEMPLE 54 : SPIRO [(5-ACETYL CHROMANE)-3 : 2'-(N-PROPYL PYRROLIDINE)]**

STADE I : SPIRO [(5-TRIFLUOROMETHYLSULFONYLOXY CHROMANE)-3 : 2'-(N-PROPYL PYRROLIDINE)]

Dissoudre 1g (4,04 mmoles) de spiro [(5-hydroxy chromane)-3 : 2'-(N-propyl pyrrolidine)] dans 25 cm3 de chlorure

de méthylène puis ajouter 1,62 $cm^3$ de pyridine.

Refroidir à 0°C puis additionner goutte à goutte 0,80 $cm^3$ d'anhydride trifluorométhanesulfonique.

Maintenir l'agitation 1 heure entre 0 et + 5°C puis extraire au chlorure de méthylène, sécher la phase organique et concentrer sous pression réduite.

Le produit brut est purifié par chromatographie sur colonne de silice (Eluant : acétate d'éthyle).

On obtient ainsi le spiro [(5-trifluorométhylsulfonyloxy chromane)-3 : 2'-(N-propyl pyrrolidine)] avec un rendement de 80%.

Infra Rouge : 1405 $cm^{-1}$ et 1200 $cm^{-1}$ $\nu$ sulfonate

STADE II : SPIRO [(5-ACETYL CHROMANE)-3 : 2'-(N-PROPYL PYRROLIDINE)]

Dissoudre 0,8 g (2,11 mmoles) de spiro [(5-trifluorométhylsulfonyloxy chromane)-3 : 2'-(N-propyl pyrrolidine)] dans 8 cm3 de N,N-diméthyl formamide.

Ajouter ensuite 0,43 g (4,22 mmoles) de triéthylamine, 1,16 g (11,61 mmoles) de butylvinyléther, 0,023 g (0,058 mmoles) de 1,2-bis (diphénylphosphino)éthane et 0,012 g (0,052 mmoles) de $Pd(OAc)_2$ puis chauffer à reflux pendant 8 heures.

Après refroidissement, hydrolyser avec 7 $cm^3$ d'acide chlorhydrique 10 N, agiter une heure à température ambiante et extraire au chlorure de méthylène.

Après mise à sec sous pression réduite, le produit brut est purifié par chromatographie sur colonne de silice (éluant : mélange éther éthylique, éther de pétrole en gradient).

On obtient ainsi le spiro [(5-acétyl chromane)-3 : 2'-(N-propyl pyrrolidine)] avec un rendement de 40 %.

**EXEMPLE 55 : SPIRO [(5-METHOXY CHROMANE)-3 : 2'-(3'-METHYL PYRROLIDINE-5'-ONE)]**

En procédant de la même façon que pour l'exemple 1 mais en remplaçant dans le stade I l'acrylate de méthyle par le crotonate de méthyle, on obtient le spiro [(5-méthoxy chromane)-3 : 2'-(3'-méthyl pyrrolidine-5'-one)]

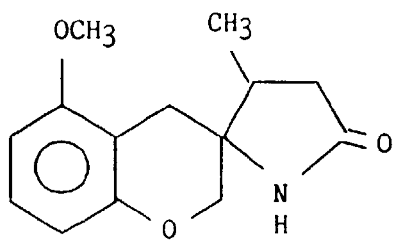

Infra Rouge (KBr) : 1665 $cm^{-1}$ $\nu$ C = 0

**EXEMPLE 56 : SPIRO [(5-METHOXY CHROMANE)-3 : 2'-(3'-METHYL N-PROPYL PYRROLIDINE)]**

En réduisant le spiro [(5-méthoxy chromane)-3 : 2'-(3'-méthyl pyrrolidine-5'-one)] par le borane diméthylsulfure selon l'exemple 4 puis en alkylant le spiro [(5-méthoxy chromane)-3 : 2'-(3'-méthyl pyrrolidine)] ainsi obtenu par le 1-iodopropane selon l'exemple 5 on obtient le spiro[(5-méthoxy chromane)-3 : 2'-(3'-méthyl N-propyl pyrrolidine)]

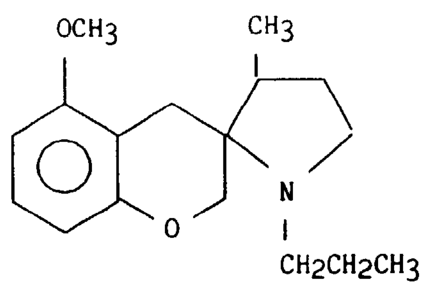

Infra Rouge (film) : 2970 à 2810 $cm^{-1}$ $\nu$ CH 1580 $cm^{-1}$ $\nu$ C=C aromatique.

**EXEMPLE 57 : ISOMERE LEVOGYRE DU SPIRO [(5-METHOXY CHROMANE)-3 : 2'-PYRROLIDINE]**

Dissoudre 1,41 g (4,05 mmoles) d'acide binaphtylphosphorique ((R) - (-) BNPPA) dans 50 cm3 d'un mélange méthanol 90/chlorure de méthylène 10.

Ajouter goutte à goutte une solution de 1,27 g (5,79 mmoles) de spiro [(5-méthoxy chromane)-3 : 2'- pyrrolidine] dans 6 cm3 de méthanol.

Agiter 3 heures à température ambiante puis mettre à sec.

Le sel brut obtenu est recristallisé une première fois dans l'acétonitrile puis trois fois dans l'éthanol. On obtient 580 mg (35 %) de binaphtylphosphate de l'isomère lévogyre du spiro [(5-méthoxy chromane)-3 : 2'- pyrrolidine)].

550 mg (0,97 mmoles) de ce sel sont repris dans un mélange constitué de 10 cm3 d'une solution aqueuse (2M) d'ammoniaque et 5 cm3 d'acétate d'éthyle.

Après une heure d'agitation à température ambiante, extraire l'amine avec de l'acétate d'éthyle puis, après mise à sec, la filtrer sur silice (éluant : acétate d'éthyle).

On obtient ainsi 212 mg de l'isomère levogyre du spiro[(5-méthoxy chromane)-3 : 2'-pyrrolidine]
Rendement de séparation : 35 %
Pureté énantiomérique : 99,7 %
Pouvoir rotatoire $[\alpha]_D^{20}$ = - 15° (22 mg dans 3 $cm^3$ de chloroforme)

**EXEMPLE 58 : ISOMERE DEXTROGYRE DU SPIRO [(5-METHOXY CHROMANE)-3 : 2'-PYRROLIDINE)]**

En procédant de manière analogue à l'exemple 57 on obtient l'isomère dextrogyre du spiro [(5-méthoxy chromane)-3 : 2'- pyrrolidine]

**EXEMPLE 59 : ISOMERE DEXTROGYRE DU SPIRO [(5-METHOXY CHROMANE)-3 : 2'-{N-[4'-(8'-AZASPIRO[4',5'] DECANE-7',9'-DIONE-8'-YL) nBUT-1'-YL] PYRROLIDINE}]**

En procédant comme pour l'exemple 8 mais à partir de l'isomère lévogyre du spiro [(5-méthoxy chromane)-3 : 2'-pyrrolidine] on obtient l'isomère dextrogyre du spiro [(5-méthoxy chromane)-3 : 2'-{N-[4'-(8'-azaspiro[4',5'] décane-7', 9'-dione-8'-yl) nbut-1'-yl] pyrrolidine}]
Point de fusion de l'oxalate = 72°C
Pouvoir rotatoire $[\alpha]_D^{20}$ = + 15° (19,6 mg dans 3 $cm^3$ de chloroforme)

**EXEMPLE 60 : ISOMERE LEVOGYRE DU SPIRO [(5-METHOXY CHROMANE)-3 : 2'-{N-[4'-(8'-AZASPIRO[4',5'] DECANE-7',9'-DION-8'-YL) nBUT-1'-YL] PYRROLIDINE}]**

En procédant comme pour l'exemple 8 mais à partir de l'isomère dextrogyre du spiro [(5-méthoxy chromane)-3 : 2'- pyrrolidine] on obtient l'isomère lévogyre du spiro [(5-méthoxy chromane)-3 : 2'-{N-[4'-(8'-azaspiro[4',5'] décane-7', 9'-dion -8'-yl) nbut-1-yl] pyrrolidine}]
Point de fusion de l'oxalate = 72°C
Pouvoir rotatoire $[\alpha]_D^{20}$ = - 16° (21,2 mg dans 3 $cm^3$ de chloroforme)

**EXEMPLE 61 : SPIRO [(5-METHOXY CHROMANE)-3 : 2'- PIPERIDINE]**

STADE I : 3-(5-METHOXY 3-NITROCHROMAN-3-YL) BUTYRATE D'ETHYLE

Ajouter 0,02 cm3 de méthylate de benzyltriméthylammonium puis 0,585 g (3 mmoles) de 4-bromobutyrate d'éthyle à une solution de 0,21 g (1 mmole) de 3-nitro 5-méthoxychromane dans 6 cm3 de N,N-diméthyl formamide.

Chauffer 15 heures à 60°C, puis mettre à sec sous pression réduite et purifier le produit brut obtenu par chromatographie sur colonne de silice (éluant : chlorure de méthylène).

On obtient ainsi 0,145 g (45 %) de 3-(5-méthoxy 3-nitrochroman-3-yl) butyrate d'éthyle sous forme d'huile incolore.
Infra Rouge (film) : 1725 $cm^{-1}$ $\nu$ C = 0
RMN $^1H$ 300 MHz ($CDCl_3$) $\delta$ : ppm

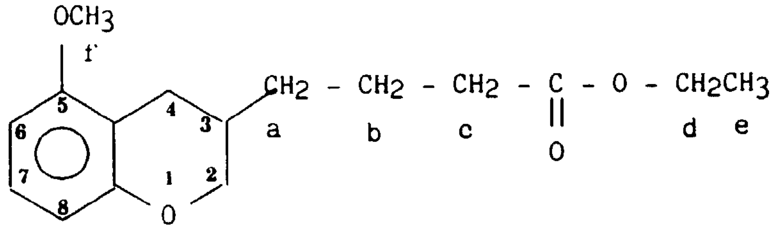

0,94; triplet J = 7,2 Hz; 3H; $H_e$
1,30 à 1,39; multiplet; 2H; $H_b$
2,10 à 2,56; multiplet ; 4H; $H_a$ et $H_c$
2,89 et 3,60; 2 doublets J = 17,5 Hz; 2H; $H_4$
3,84; singulet; ; 3H; $H_f$
4,09 et 4,57; 2 doublets; J = 11,6 Hz; 2H ; $H_2$
4,12; quadruplet; J = 7,2 Hz; 2H ; $H_d$
6,47 et 6,50; 2 doublets; J = 8,3 Hz; 2H ; H aromatiques
7,11; triplet; J = 8,3 Hz; 1H ; H aromatique

STADE II : SPIRO [(5-METHOXY CHROMANE)-3 : 2'-(PIPERIDINE-6'-ONE)]

Dissoudre 0,325 g (1 mmole) de 3-(5-méthoxy 3-nitrochroman-3-yl) butyrate d'éthyle dans 20 cm3 d'éthanol.

Ajouter 40 mg de nickel de Raney et porter à reflux sous atmosphère d'hydrogène pendant une nuit.

Après refroidissement, filtrer le milieu réactionnel sur célite, puis après mise à sec du filtrat, purifier le produit brut obtenu par chromatographie sur colonne de silice (éluant : méthanol 5 / chlorure de méthylène 95)

On obtient ainsi 0,235 g (95 % ) de spiro [(5-méthoxy chromane)-3 : 2'-(piperidine-6'-one)] sous forme d'une huile incolore.

Infra Rouge (film) : $3245\ cm^{-1}$ ν NH $1675\ cm^{-1}$ ν C = 0

RMN $^1H$ 300 MHz ($CDCl_3$) δ : ppm

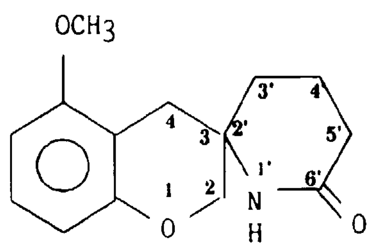

1,48 à 1,63; multiplet ; 2H; $H_{4'}$
1,90 à 2,10; multiplet; 2H; $H_{3'}$
2,39 à 2,51; multiplet ; 2H; $H_{5'}$
2,75 à 2,87; 2 doublets ; 2H; $H_4$
3,83; singulet; 3H; $H_a$
3,88 et 3,93; 2 doublets J = 11,2 Hz; 2H ; $H_2$
5,80; massif; 1H ; NH
6,46 et 6,52; 2 doublets; J = 8,3 Hz; 2H ; H aromatiques
7,10; triplet; J = 8,3 Hz; 1H ; H aromatique

STADE III : SPIRO [(5-METHOXY CHROMANE)-3 : 2'-PIPERIDINE]

Dissoudre 0,25 g (1 mmole) de spiro [(5-méthoxy chromane)-3 : 2'-(pipéridine-6'one)] dans 15 cm3 de THF puis ajouter le complexe diméthylsulfure et porter à reflux pendant 4 heures 30.

Mettre le milieu réactionnel à sec, reprendre le résidu très lentement dans 5 cm3 de méthanol puis ajouter 2,5 cm3 d'acide chlorhydrique 2M et porter 90 minutes à reflux.

Après refroidissement, éliminer le méthanol, neutraliser avec une solution aqueuse de soude 2M, extraire au chlorure de méthylène.

Le produit brut obtenu par mise à sec est purifié par chromatographie sur colonne de silice (éluant : acétate d'éthyle 100 %)

On obtient ainsi 0,160 g (70 %) de spiro [(5-méthoxy chromane)-3 : 2'-pipéridine]

Infra Rouge (film) : 3300 $cm^{-1}$ $\nu$ NH 1580 $cm^{-1}$ $\nu$ C = C aromatique

RMN $^1H$ 300 MHz ($CDCl_3$) $\delta$ : ppm

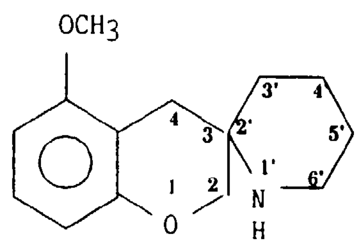

1,31 à 1,40; multiplet ; 2H; $H_{4'}$
1,56 à 1,97; multiplet; 4H; $H_{5'}$ et $H_{3'}$
2,19; singulet; 1H; NH
2,68; singulet ; 2H; $H_4$
2,95 à 3,18; multiplet; 2H; $H_{6'}$
3,81; singulet; 3H ; $H_a$
3,83; singulet; 2H ; H2
6,44 et 6,53; 2 doublets; J = 8,3 Hz; 2H ; H aromatiques
7,07; triplet; J = 8,3 Hz; 1H ; H aromatique

**EXEMPLE 62 à 69**

En procédant comme pour les exemples 5, 8, 13, 17, 19, 20, 22, 24, 27, mais en remplaçant le spiro [(5-méthoxy chromane)-3 : 2'- pyrrolidine] par le spiro [(5-méthoxy chromane)-3 : 2'- pipéridine] on obtient :

- le spiro [(5-méthoxy chromane)-3 : 2'-(N-propyl pipéridine)]

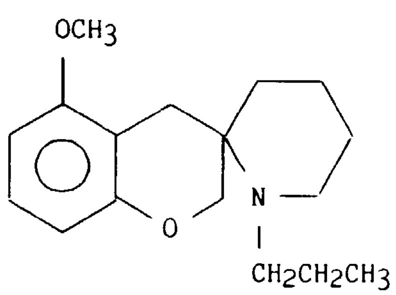

- le spiro [(5-méthoxy chromane)-3 : 2'-{N-[4'-(8'-azaspiro [4',5'] décane-7',9'-dione-8'-yl)nbut-1'-yl]pipéridine}]

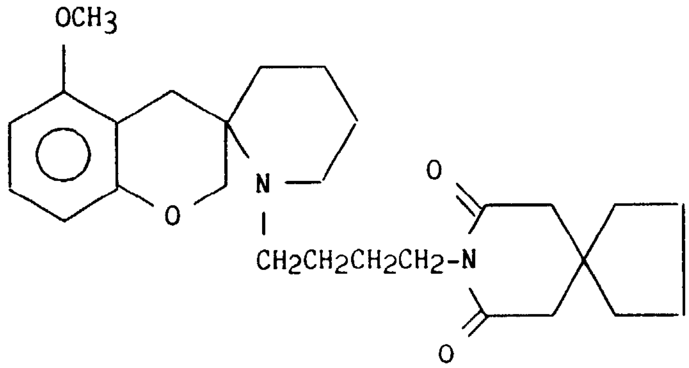

Infra Rouge (film) : 1655 et 1720 $cm^{-1}$ ν C = 0

- le spiro [(5-méthoxy chromane)-3 : 2'-[N-(4'-phtalimido nbut-1'-yl)pipéridine]]

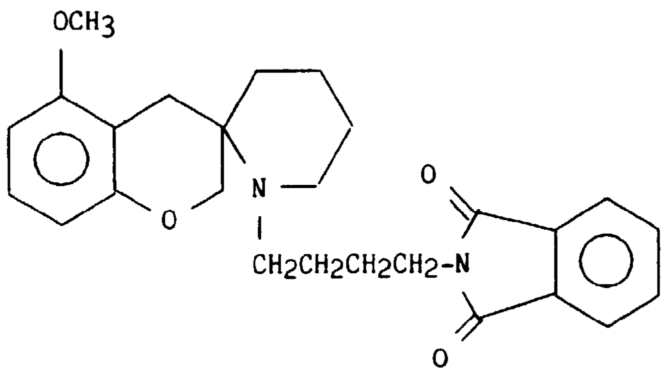

Infra Rouge (film) : 1705 $cm^{-1}$ ν C = 0

- le spiro [(5-méthoxy chromane)-3 : 2'-{N-[2'-(8'-azaspiro [4',5'] décane-7',9'-dion-8'-yl)éthyl] pipéridine}]

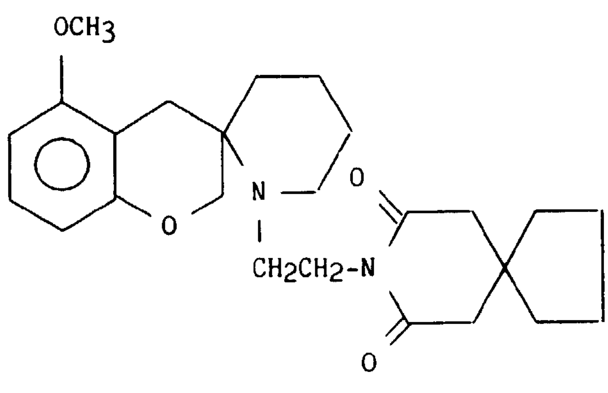

- le spiro [(5-méthoxy chromane)-3 : 2'-{N-[4'-(oxazolo[4',5'-b]pyridine-2'(3H)-one-3'-yl)nbut-1'-yl] pipéridine}]

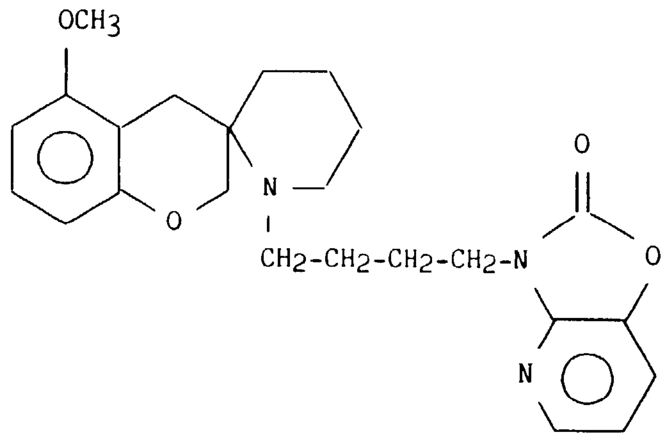

- le spiro [(5-méthoxy chromane)-3 : 2'-{N-[4'-(4',4'-diméthyl pipéridine-2',6'-dione-1'-yl) nbut-1'-yl]pipéridine}]

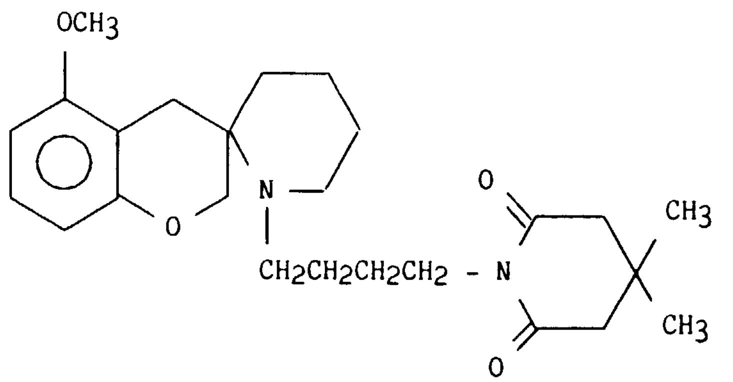

- le spiro [(5-méthoxy chromane)-3 : 2'-{N-[4'-(1',1'-dioxo 1',2'-benzisothiazol-3'(2H)-one-2'-yl) nbut-1'-yl] pipéridine}]

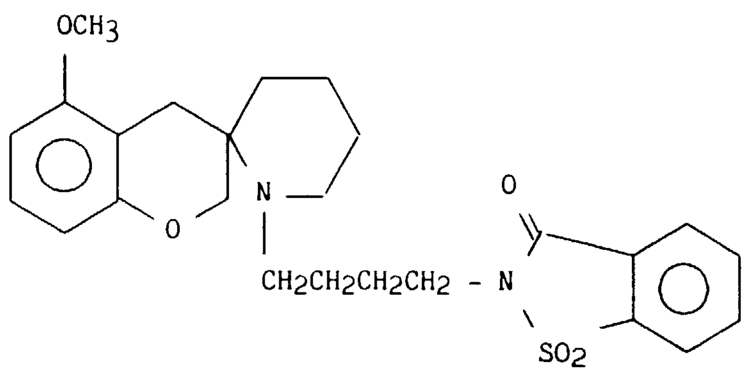

- le spiro [(5-méthoxy chromane)-3 : 2'-(N-benzyl pipéridine)]

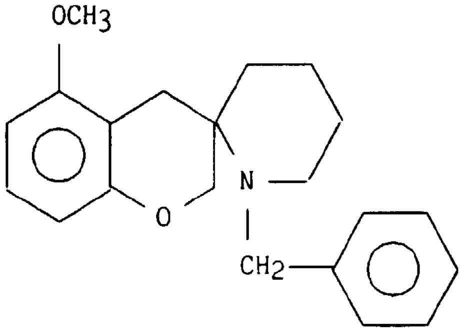

## ETUDE PHARMACOLOGIQUE DES COMPOSES DE L'INVENTION

### EXEMPLE 70 : DETERMINATION IN VITRO DE L'AFFINITE DES COMPOSES DE L'INVENTION POUR LES RECEPTEURS SEROTONINERGIQUES, DOPAMINERGIQUES ET ALPHA ADRENERGIQUES

Les déterminations d'affinité pour les récepteurs sérotoninergiques, dopaminergiques et alpha adrénergiques ont été effectuées selon les techniques classiques par déplacement d'un radio ligand de référence.

| RECEPTEUR | RADIOLIGAND | TISSU UTILISE |
|---|---|---|
| 5 $HT_1A$ | 8-OH DPAT | Hippocampe |
| 5 $HT_1B$ | 5-OH Tryptamine | Cortex+striatum+Globus Pallidus |
| 5 $HT_1C$ | N-Methyl Mesulergine | Cortex, hippocampe |
| 5 $HT_1D$ | 5-OH Tryptamine | Cortex+striatum+Globus Pallidus |
| 5 $HT_2$ | Kétansérine | Cortex |
| 5 $HT_3$ | BRL 43694 | Area postrema |

Les résultats de ces études de binding montrent que les composés de l'invention possèdent une haute affinité associée à une forte sélectivité pour les récepteurs $5HT_1A$ par rapport aux autres récepteurs sérotoninergiques.

Cette sélectivité pour les récepteurs 5 $HT_1A$ est également très importante (au moins un facteur 1000) par rapport aux récepteurs dopaminergiques $D_1$, $D_2$, et adrénergiques $\alpha_1$, $\alpha_2$.

Le spiro [(5-méthoxy chromane)-3 : 2'-{N-[4'-(8'-azaspiro[4',5'] décane-7',9'-dione-8'-yl) nbut-1'-yl] pyrrolidine}] par exemple possède une affinité nanomolaire pour les récepteurs $5HT_1A$ alors que son affinité pour les autres récepteurs sérotoninergiques ($5HT_1B$, $5HT_1C$, $5HT_1D$, $5HT_2$, $5HT_3$) est comprise entre $4x10^{-6}$ M et $2x10^{-5}$ M et que son affinité pour les autres récepteurs $D_1$, $D_2$,$\alpha_1$, $\alpha_2$ est moins bonne que $10^{-6}$ M.

### EXEMPLE 71 : ETUDE DE LA TOXICITE AIGUE

La toxicité aiguë a été effectuée après administration orale à des lots de cinq souris (20 ± 2 grammes) de doses croissantes (0.1 - 0,25 - 0,50 - 0,75 - $1g/Kg^{-1}$) des produits de l'invention.
Les animaux ont été observés à intervalles réguliers au cours de la première journée et quotidiennement pendant les deux semaines suivant le traitement. Il apparait que les composés de l'invention sont totalement atoxiques. Aucun décès n'est observé après administration d'une dose de 1 g. $Kg^{-1}$. On ne constate pas de trouble après administration de cette dose.

### EXEMPLE 72 : ETUDE DE L'ACTIVITE ANTIDEPRESSIVE EFFET SUR LES ECHECS D'ECHAPPEMENT

L'étude des produits est réalisée sur le modèle de "learned helplessness" ou renoncement appris qui consiste à induire chez l'animal, par une série d'évènements aversifs incontrôlables, un déficit lors des tâches d'évitement ultérieures (Martin et al., 1986, Pharmacol. Biochem. Behav., 24, 177-181).

Nous utilisons des rats mâles Wistar A.F. provenant des élevages homogènes CERJ, d'un poids compris entre 180 et 200 grammes. Les animaux sont maintenus à l'animalerie une semaine avant le test, dans des boîtes en plastique, par groupes de 10, à une température ambiante de 21°C ∓ 1°C, avec libre accès à l'eau et à la nourriture.

Les animaux sont isolés dans des boîtes de petites dimensions et sont soumis à 60 chocs électriques inévitables (-0,8 mA toutes les minutes ∓ 15 secondes). Un groupe de rats témoins ne reçoit pas de chocs électriques. La capacité des animaux à réaliser un apprentissage d'évitement (shuttle-box) est appréciée 48 heures après et pendant 3 jours consécutifs. Lors des séances d'apprentissage, les animaux subissent 2 essais par minute pendant 15 minutes. Le nombre d'échecs d'échappement (escape failure) est noté pour chaque rat. Les animaux sont traités (i.p.; 0.5 ml/100 g) 6 heures après les chocs inévitables et 4 jours de suite, le matin 30 minutes avant la séance de shuttle-box et le soir entre 18 et 19h. Les produits étudiés sont mis en solution dans l'eau distillée.
Les produits étudiés sont administrés aux doses 0.25 mg.Kg/jour.

Le test prouve que les produits de l'invention diminuent significativement le nombre d'échecs d'échappement ce qui traduit une activité de type antidépressive.

**EXEMPLE 73 : COMPOSITIONS PHARMACEUTIQUES**

Comprimés dosés à 2,5 mg de spiro [(5-méthoxy chromane)-3 : 2'-{N[4'-(8'-azaspiro[4',5'] décane-7',9'-dione-8-yl) nbut-1'-yl] pyrrolidine}]

Formule pour 1000 comprimés :

| | |
|---|---|
| Spiro [(5-méthoxy chromane)-3 : 2'-{N-[4'-(8'-azaspiro[4',5'] décane-7',9'-dione-8'-yl) nbut-1'-yl] pyrrolidine}] | 2,5 g |
| Amidon de blé | 15 g |
| Amidon de maïs | 15 g |
| Lactose | 65 g |
| Stéarate de magnésium | 2 g |
| Silice | 1 g |
| Hydroxypropyl cellulose | 2 g |

## Revendications

**1.** Composés spirochromaniques de formule générale (I) :

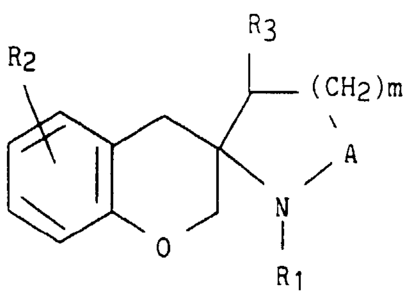

(I)

dans laquelle :

- m, nombre entier peut prendre les valeurs 1 ou 2,
- A représente un méthylène ($CH_2$) ou un carbonyle (CO),
- $R_1$ représente :
  - un hydrogène,
  - un groupement - CO - $R_4$ avec $R_4$ représentant un alkyle linéaire ou ramifié de 1 à 6 atomes de carbone, un phényl éventuellement substitué ou un phénylalkyle éventuellement substitué dont la chaine alkyle comporte de 1 à 3 atomes de carbone,
  - ou un alkyle linéaire ou ramifié de 1 à 6 atomes de carbone éventuellement substitué par :
    - un nitrile,

- un phényl éventuellement substitué,
- un groupement $-NR_5R_6$ avec $R_5$ représentant un hydrogène ou un alkyle linéaire ou ramifié de 1 à 6 atomes de carbone et R6 représentant un hydrogène, un alkyle linéaire ou ramifié de 1 à 6 atomes de carbone éventuellement substitué par un phényl éventuellement substitué, un groupement alkylcarbonyl linéaire ou ramifié comprenant de 2 à 7 atomes de carbone, un groupement benzoyl éventuellement substitué, un groupement phénylalkylcarbonyl éventuellement substitué dont la chaine alkyle comporte de 1 à 3 atomes de carbone, un groupement alkyle sulfonyle linéaire ou ramifié de 1 à 6 atomes de carbone, un groupement phénylsulfonyle éventuellement substitué,
- un quelconque des groupements suivants :

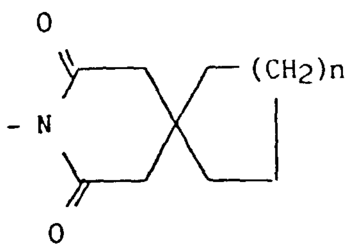
,
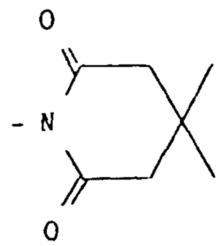
,
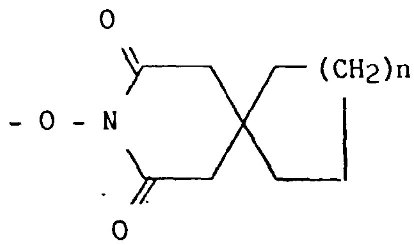

dans lesquels :
- n, nombre entier, peut prendre les valeurs 1 ou 2,

- $R_2$ représente :

    - un hydrogène,
    - un groupement acétyl,
    - un groupement $CF_3SO_2$-O-,
    - ou un groupement - $OR_7$ avec $R_7$ ayant la même définition que $R_1$,

- $R_3$ représente un hydrogène ou un alkyle linéaire ou ramifié de 1 à 4 atomes de carbone avec la réserve que lorsque $R_1 = R_2 = R_3 = H$ alors m ne peut pas être égal à 1,
l'expression "éventuellement substitué" associée aux termes phényl, phénylalkyle, phénylsulfonyl, benzoyl ou phénylalkylcarbonyl signifie que le noyau aromatique peut être substitué par un ou plusieurs alkyles inférieurs de 1 à 4 atomes de carbone ramifié ou non, nitro, alkoxy inférieur de 1 à 4 atomes de carbone, halogène, trifluorométhyle, hydroxy,
- leurs isomères, diastéréoisomères, énantiomères isolés ou sous forme de mélange,
- leurs sels d'addition à un acide minéral ou organique pharmaceutiquement acceptable.

**2.** Composés de formule générale (I), selon la revendication 1, pour lesquels m est égal à 1, ce qui correspond aux spiro [chromane-3 : 2'-pyrrolidine] substitués de formule générale suivante :

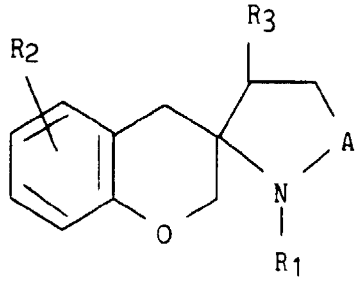

dans laquelle R1, R2, R3 et A ont la même signification que dans la revendication 1,

- leurs isomères, diastéréoisomères, énantiomères isolés ou sous forme de mélange,
- leurs sels d'addition à un acide minéral ou organique pharmaceutiquement acceptable.

3. Composés de formule générale (I) selon la revendication 1, pour lesquels m est égal à 2, ce qui correspond aux spiro [chromane-3 : 2'-pipéridine] substitués de formule générale suivante :

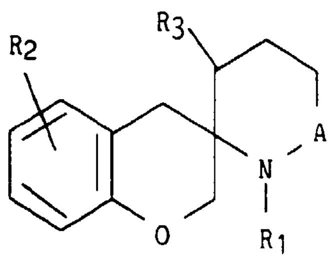

dans laquelle $R_1$, $R_2$, $R_3$ et A ont la même signification que dans la revendication 1,

- leurs isomères, diastéréoisomères, énantiomères isolés ou sous forme de mélange,
- leurs sels d'addition à un acide minéral ou organique pharmaceutiquement acceptable.

4. Composé selon la revendication 1, qui est le spiro [(5-méthoxy chromane)-3 : 2'-{N-[4'-(8'-azaspiro [4',5'] décane-7',9'-dion-8'-yl)nbut-1'-yl] pyrrolidine}] dont la formule est représentée ci dessous, ainsi que ses énantiomères, isolés ou sous forme de mélange, et leurs sels d'addition à un acide minéral ou organique pharmaceutiquement acceptable.

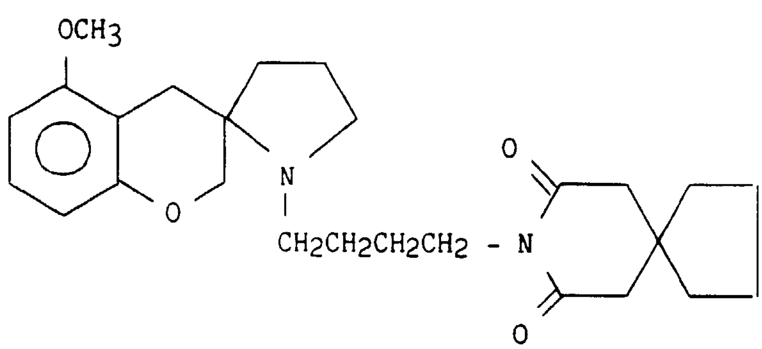

5. Composé selon la revendication 1, qui est l'isomère dextrogyre du spiro [(5-méthoxy chromane)-3 : 2'-{N-[4'-(8'-azaspiro [4',5'] décane-7',9'-dion-8'-yl)nbut-1'-yl] pyrrolidine}] dont la formule est représentée ci dessous, ainsi que ses sels d'addition à un acide minéral ou organique pharmaceutiquement acceptable.

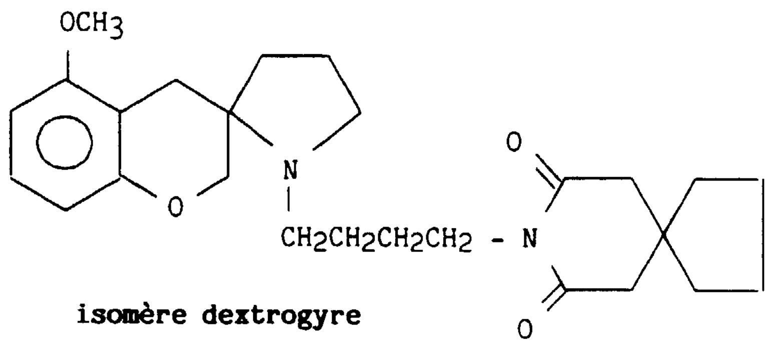

**isomère dextrogyre**

6. Composé selon la revendication 1, qui est l'isomère lévogyre du spiro [(5-méthoxy chromane)-3 : 2'-{N-[4'-(8'-azaspiro [4',5'] décane-7',9'-dion-8'-yl)nbut-1'-yl] pyrrolidine}] dont la formule est représentée ci dessous, ainsi que ses sels d'addition à un acide minéral ou organique pharmaceutiquement acceptable.

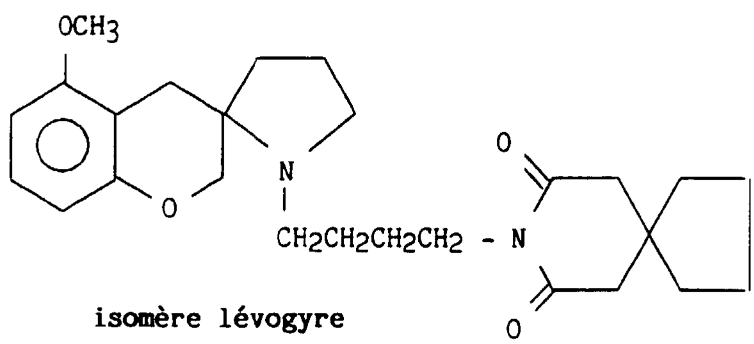

**isomère lévogyre**

7. Composé, selon la revendication 1, qui est le spiro [(5-acétyl chromane)-3 : 2'- (N-propyl pyrrolidine)] dont la formule est représentée ci dessous, ainsi que ses énantiomères, isolés ou sous forme de mélange, et leurs sels d'addition à un acide minéral ou organique pharmaceutiquement acceptable.

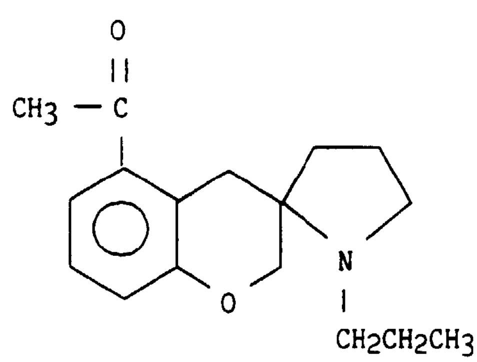

8. Composé, selon la revendication 1, qui est le spiro [(5-méthoxy chromane)-3 : 2'- pyrrolidine] dont la formule est représentée ci dessous, ainsi que ses énantiomères, isolés ou sous forme de mélange, et leurs sels d'addition à un acide minéral ou organique pharmaceutiquement acceptable.

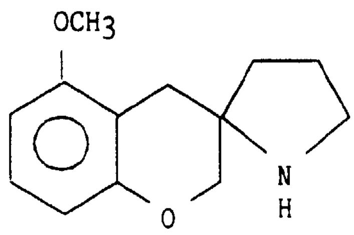

9. Composé, selon la revendication 1, qui est le spiro [(5-méthoxy chromane)-3 : 2'- pipéridine] dont la formule est représentée ci dessous, ainsi que ses énantiomères, isolés ou sous forme de mélange, et leurs sels d'addition à un acide minéral ou organique pharmaceutiquement acceptable.

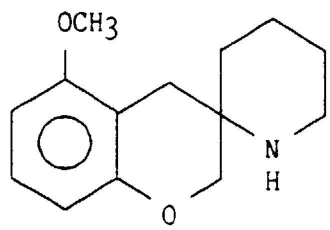

10. Composé selon la revendication 1, qui est le spiro [(5-hydroxy chromane)-3 : 2'-(N-propyl pyrrolidine)] dont la formule est représentée ci dessous, ainsi que ses énantiomères, isolés ou sous forme de mélange, et leurs sels d'addition à un acide minéral ou organique pharmaceutiquement acceptable.

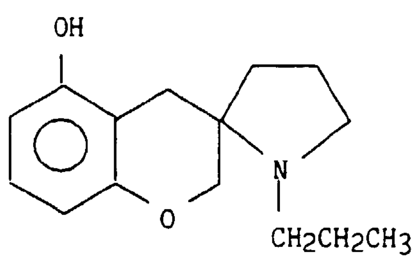

11. Composé selon la revendication 1, qui est le spiro [(5-méthoxy chromane)-3 : 2'-(N-propyl pyrrolidine)] dont la formule est représentée ci dessous, ainsi que ses énantiomères, isolés ou sous forme de mélange, et leurs sels d'addition à un acide minéral ou organique pharmaceutiquement acceptable.

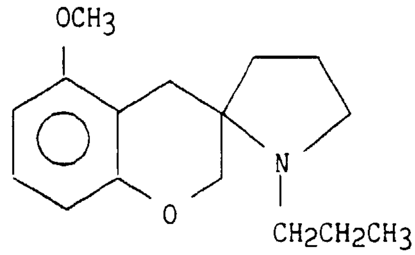

**12.** Composé, selon la revendication 1, qui est le spiro [{5-[4-(8-azaspiro [4,5] décane-7,9-dion-8-yl) nbutyloxy] chromane}-3 : 2'-(N-propyl pyrrolidine)] dont la formule est représentée ci dessous, ainsi que ses énantiomères, isolés ou sous forme de mélange, et leurs sels d'addition à un acide minéral ou organique pharmaceutiquement acceptable.

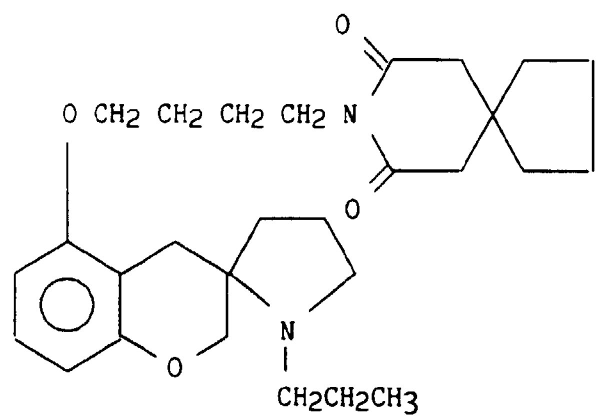

**13.** Procédé d'obtention des composés de formule générale (Ia) :

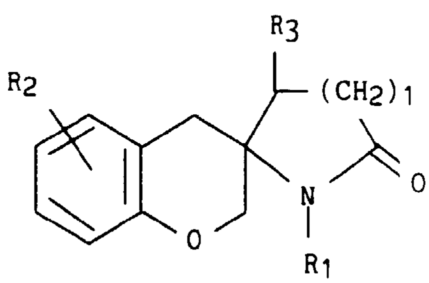

(Ia)

dans laquelle $R_1$, $R_2$ et $R_3$ sont tels que définis dans la revendication 1,
cas particulier des composés de formule (I) pour lesquels m est égal à 1, et A représente un carbonyle (CO), caractérisé en ce que l'on fait réagir un benzaldéhyde substitué de formule générale (II) :

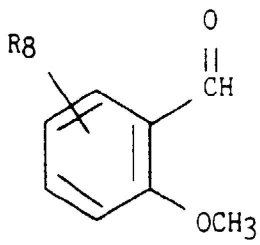

(II)

dans laquelle $R_8$ représente un hydrogène ou un alkoxy linéaire ou ramifié de 1 à 6 atomes de carbone, à une température comprise entre - 5° et 0°C, avec du tribromure de bore de manière à obtenir le composé de formule générale (III) :

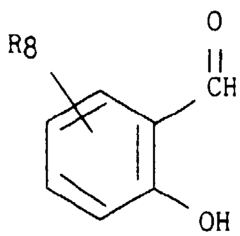

(III)

dans laquelle $R_8$ a la même signification que précédemment, que l'on fait réagir à chaud et en présence d'un sel d'ammonium substitué avec du 2-nitro éthanol, de manière à obtenir le composé de formule générale (IV) :

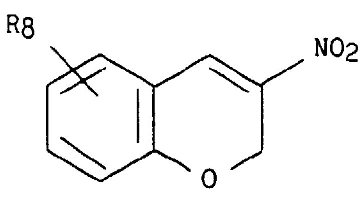

(IV)

dans laquelle $R_8$ a la même signification que précédemment, que l'on réduit de manière à obtenir le composé de formule générale (V) :

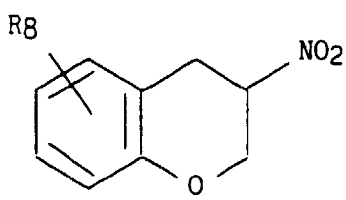

(V)

dans laquelle $R_8$ a la même signification que précédemment, que l'on fait réagir en présence de méthylate de benzyltriméthylammonium, en milieu alcoolique, avec un composé acrylique de formule générale (VI) :

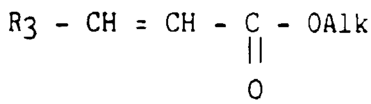

(VI)

dans laquelle $R_3$ a la même signification que précédemment et Alk représente un alkyle de 1 à 4 atomes de carbone, de manière à obtenir les composés de formule générale (VIIa) :

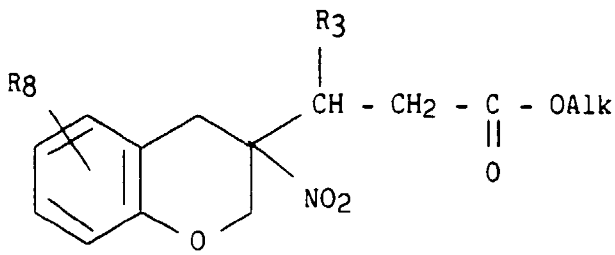

(VIIa)

dans laquelle $R_3$, $R_8$ et Alk ont la même signification que précédemment, que l'on réduit, en présence de Nickel de Raney et sous atmosphère d'hydrogène, de manière à obtenir, après isolement et éventuelle purification, le composé spiranique de formule générale (VIIIa) :

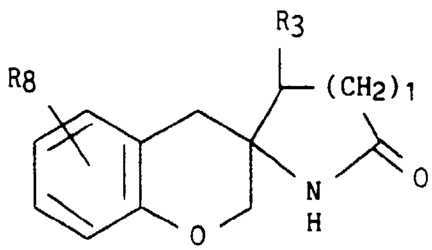

(VIIIa)

dans laquelle $R_3$ et $R_8$ ont la même signification que précédemment,
que l'on peut faire réagir en présence d'une base forte avec un composé de formule générale (IX) :

$$Hal'R'_1 \qquad (IX)$$

dans laquelle $R'_1$ a la même signification que $R_1$ dans la revendication 1 avec la réserve que R'1 ne peut représenter un hydrogène, et Hal' représente un atome d'halogène, de manière à obtenir, après isolement et éventuelle purification, le composé de formule générale (Xa) :

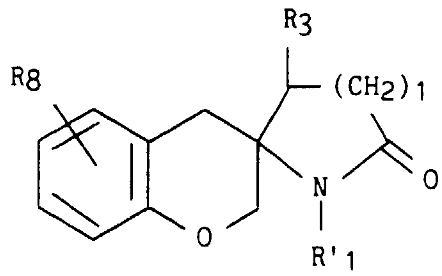

(Xa)

dans laquelle $R'_1$, $R_3$ et $R_8$ ont la même signification que précédemment, composés de formule (VIIIa) et (Xa) que l'on peut traiter, dans le cas où $R_8$ représente un alkoxy, par une solution aqueuse d'acide bromhydrique, pour obtenir, après isolement et éventuelle purification, le composé de formule générale (XIa):

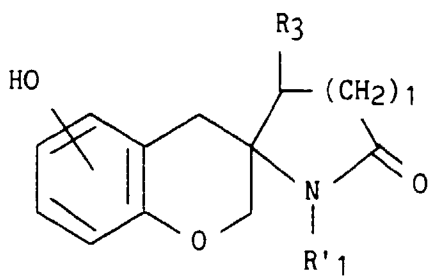

(XIa)

dans laquelle $R'_1$ et $R_3$ ont la même signification que précédemment, que l'on peut faire réagir avec un composé de formule générale (XII) :

$$Hal'' - R'_7 \qquad (XII)$$

dans laquelle Hal" représente un atome d'halogène et $R'_7$ a la même signification que $R_7$ dans la revendication 1, avec la réserve que $R'_7$ ne peut représenter un hydrogène, de manière à obtenir, après isolement et éventuelle purification, le composé de formule générale (XIIIa) :

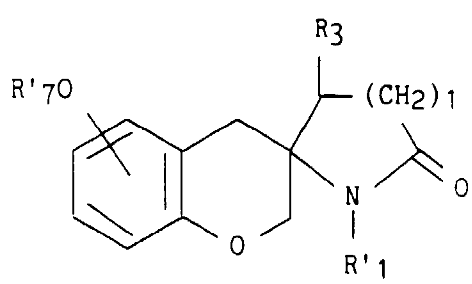

(XIIIa)

dans laquelle $R'_1$, $R_3$ et $R'_7$ ont la même signification que précédemment, étant entendu que les composés de formule générale VIIIa, Xa, XIa, et XIIIa, font partie de l'invention, constituent les composés de formule générale (Ia) tels que définis précedemment, et peuvent, si on le désire, être purifiés, séparés en leurs isomères, ou si cela est possible salifiés avec un acide pharmaceutiquement acceptable.

**14.** Procédé d'obtention des composés de formule générale (Ib) :

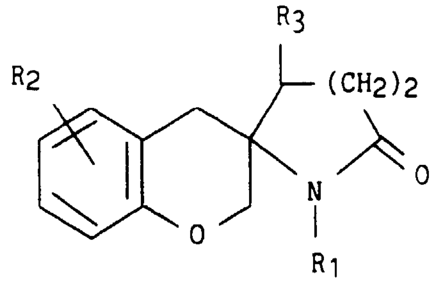

(Ib)

dans laquelle $R_1$, $R_2$ et $R_3$ sont tels que définis dans la revendication 1, cas particulier des composés de formule (I) pour lesquels m est égal à 2 et A représente un carbonyle (CO), caractérisé en ce que l'on fait réagir un composé de formule (V) :

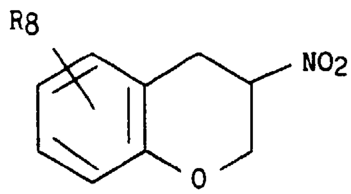

(V)

dans laquelle $R_8$ représente un hydrogène ou un alkoxy linéaire ou ramifié de 1 à 6 atomes de carbone,

avec un halogéno ester de formule générale (X) :

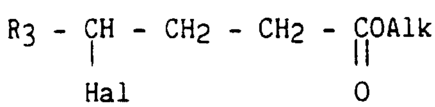

(X)

dans laquelle $R_3$ a la même signification que précedemment, Alk représente un alkyle de 1 à 4 atomes de carbone et Hal représente un atome d'halogène, de manière à obtenir les composés de formule générale (VIIb) :

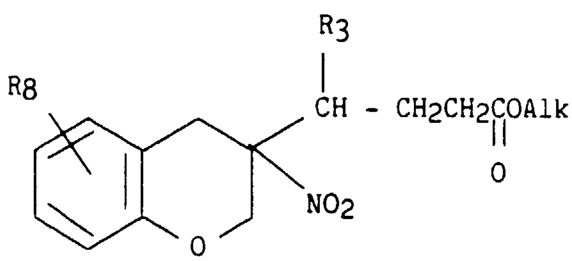

(VIIb)

dans laquelle $R_3$, $R_8$ et Alk ont la même signification que précédemment, que l'on réduit, en présence de Nickel de Raney et sous atmosphère d'hydrogène, de manière à obtenir, après isolement et éventuelle purification, le composé spiranique de formule générale (VIIIb) :

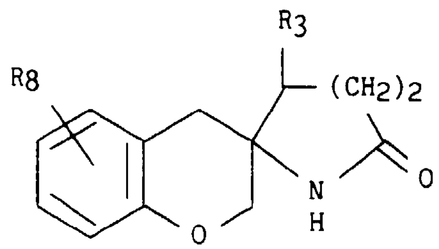

(VIIIb)

dans laquelle $R_3$ et $R_8$ ont la même signification que précédemment que l'on peut faire réagir en présence d'une base forte avec un composé de formule générale (IX) :

$$Hal'R'_1 \qquad (IX)$$

dans laquelle $R'_1$ a la même signification que $R_1$ dans la revendication 1 avec la réserve que $R'_1$ ne peut représenter un hydrogène et Hal' représente un atome d'halogène, de manière à obtenir, après isolement et éventuelle purification, le composé de formule générale (Xb) :

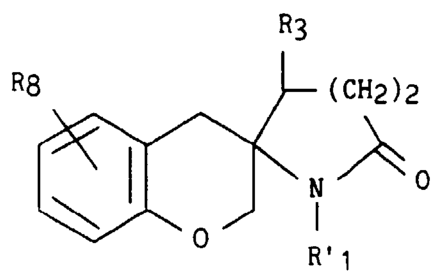

(Xb)

dans laquelle $R_1'$, $R_3$ et $R_8$ ont la même signification que précédemment, composés de formule (VIIIb) et (Xb) que l'on peut traiter, dans le cas où $R_8$ représente un alkoxy, par une solution aqueuse d'acide bromhydrique, pour obtenir, après isolement et éventuelle purification, le composé de formule générale (XIb):

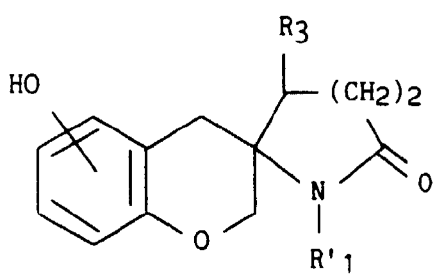

(XIb)

dans laquelle $R'_1$ et $R_3$ ont la même signification que précédemment, que l'on peut faire réagir avec un composé de formule générale (XII) :

$$Hal'' - R'_7 \qquad (XII)$$

dans laquelle Hal" représente un atome d'halogène et $R'_7$ a la même signification que $R_7$ dans la revendication 1, avec la réserve que $R'_7$ ne peut représenter un hydrogène, de manière à obtenir, après isolement et éventuelle purification, le composé de formule générale (XIIIb) :

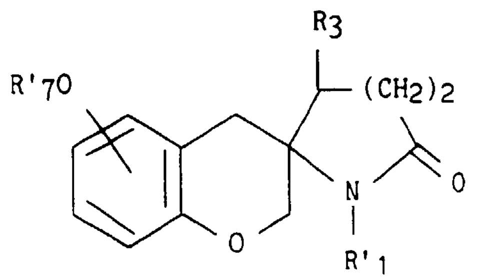

(XIIIb)

dans laquelle $R'_1$, $R_3$ et $R'_7$ ont la même signification que précédemment, étant entendu que les composés de formule générale VIIIb, Xb, XIb, et XIIIb font partie de l'invention, constituent les composés de formule générale (Ib) tels que définis précedemment, et peuvent, si on le désire, être purifiés, séparés en leurs isomères, ou si cela est possible salifiés avec un acide pharmaceutiquement acceptable.

**15.** Procédé d'obtention des composés de formule générale (Ic) :

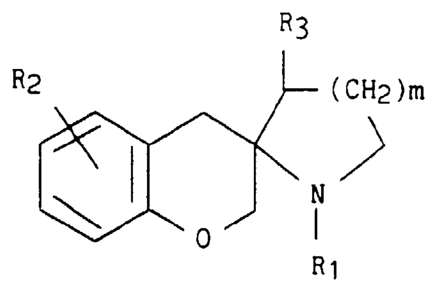

(Ic)

dans laquelle $R_1$, $R_2$, $R_3$ et m sont tels que définis dans la revendication 1,
cas particulier des composés de formule (I) pour lesquels A représente un méthylène ($CH_2$),
caractérisé en ce que l'on réduit en milieu aprotique un composé de formule (VIII) :

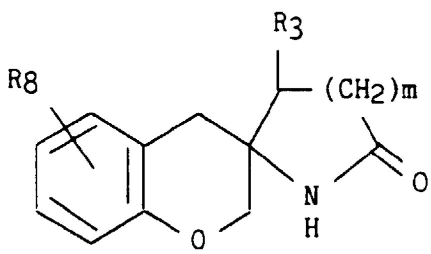

(VIII)

dans laquelle $R_3$ et m sont tels que définis précedemment et $R_8$ représente un hydrogène ou un alkoxy linéaire ou ramifié de 1 à 6 atomes de carbone, par un agent réducteur de manière à obtenir, après isolement et éventuelle purification, le composé de formule générale (XIV) :

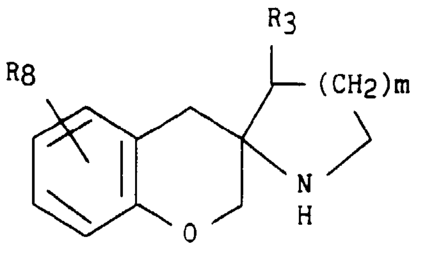

(XIV)

dans laquelle $R_3$, $R_8$ et m ont la même signification que précédemment, que l'on peut faire réagir avec un composé de formule générale (IX) :

$$Hal'R_1' \qquad (IX)$$

dans laquelle $R_1$'a la même signification que $R_1$ dans la revendication 1 avec la réserve que $R'_1$ ne peut représenter un hydrogène et Hal représente un atome d'halogène, de manière à obtenir, après isolement et éventuelle purification, le composé de formule générale (XV):

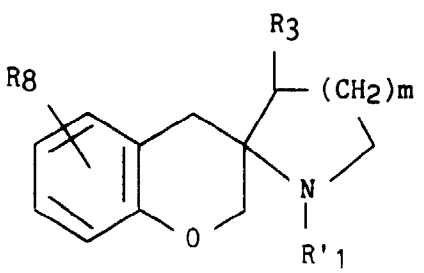

(XV)

dans laquelle $R'_1$, $R_3$, $R_8$ et m ont la même signification que précédemment, composés de formule (XIV) et (XV) que l'on peut traiter, dans le cas où $R_8$ représente un alkoxy, par une solution aqueuse d'acide bromhydrique, pour obtenir, après isolement et éventuelle purification, le composé de formule générale (XVI) :

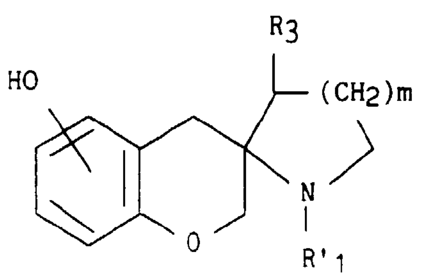

(XVI)

dans laquelle $R'_1$, $R_3$ et m ont la même signification que précédemment, que l'on peut faire réagir avec un composé de formule générale (XII) :

$$Hal'' - R'_7 \qquad (XII),$$

dans laquelle Hal" représente un atome d'halogène et $R'_7$ a la même signification que $R_7$ dans la revendication 1, avec la réserve que $R'_7$ ne peut représenter un hydrogène, de manière à obtenir, après isolement et éventuelle purification, le composé de formule générale (XVII) :

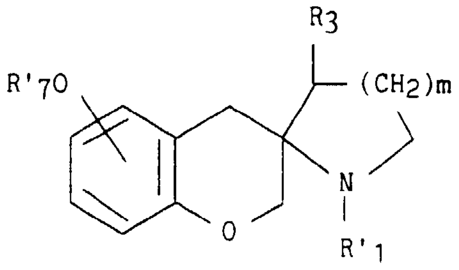

(XVII)

dans laquelle $R'_1$, $R_3$, $R'_7$ et m ont la même signification que précédemment,
étant entendu que les composés de formule générale, XIV, XV, XVI, et XVII font partie de l'invention, constituent les composés de formule générale (Ic), et peuvent, si on le désire, être purifiés, séparés en leurs isomères, si cela est possible salifiés avec un acide pharmaceutiquement acceptable.

**16.** Procédé d'obtention des composés de formule générale (Id) :

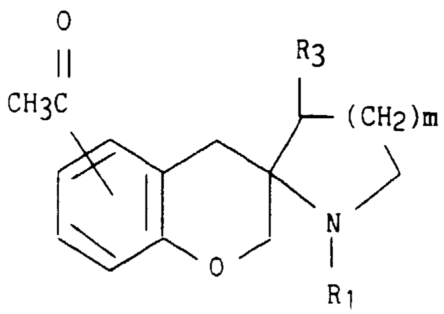

(Id)

dans laquelle $R_1$, $R_3$ et m sont tels que définis dans la revendication 1, cas particulier des composés de formule (I) pour lesquels $R_2$ représente un groupe acétyl, caractérisé en ce que l'on fait réagir avec de l'anhydride trifluorométhanesulfonique un composé de formule générale (XVIII) :

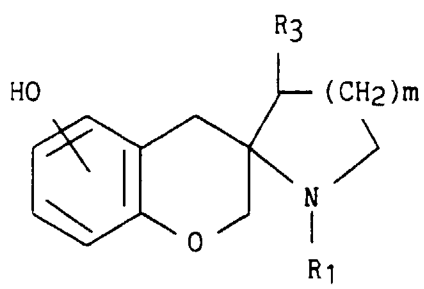

(XVIII)

dans laquelle $R_1$, $R_2$ et m ont la même signification que dans la revendication 1, de manière à obtenir le dérivé trifluorométhylsulfonyloxy de formule générale (XIX) :

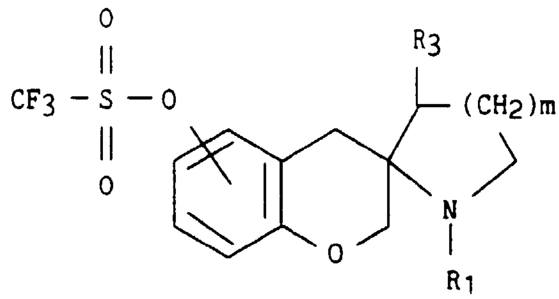

(XIX)

dans laquelle $R_1$, $R_3$ et m ont la même signification que dans la revendication 1, que l'on traite ensuite en milieu aprotique par du butylvinyléther en présence de triéthylamine, de 1,2-bis (diphénylphosphino) éthane et de Pd $(OAc)_2$ de manière à obtenir après isolement et purification le composé acétylé de formule générale (XX) :

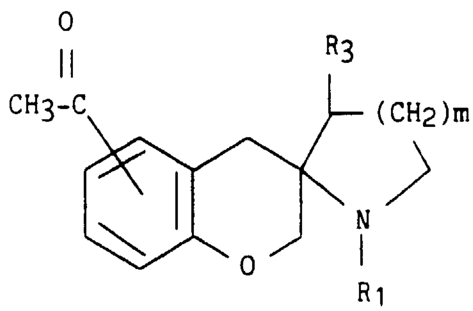

(XX)

dans laquelle $R_1$, $R_3$ et m ont la même signification que dans la revendication 1,
étant entendu que les composés de formule générale XIX et XX font partie de l'invention, et peuvent, si on le désire, être purifiés, séparés en leurs isomères, ou si cela est possible salifiés avec un acide pharmaceutiquement acceptable.

**17.** Compositions pharmaceutiques contenant comme principe actif au moins un composé selon l'une quelconque des revendications 1 à 12, seul ou en combinaison avec un ou plusieurs excipients ou véhicules inertes, non toxiques, pharmaceutiquement acceptables.

**18.** Compositions pharmaceutiques selon la revendication 17, contenant au moins un principe actif selon l'une des revendications 1 à 12, utiles pour le traitement de la dépression, du stress, des psychoses, de l'anxiété, de la schizophrénie, de l'hypertension, de la douleur, des maladies cardiovasculaires, des migraines, de l'ischémie cérébrale et comme modificateurs du comportement alimentaire et sexuel.

**Patentansprüche**

**1.** Spirochromanverbindungen der allgemeinen Formel (I):

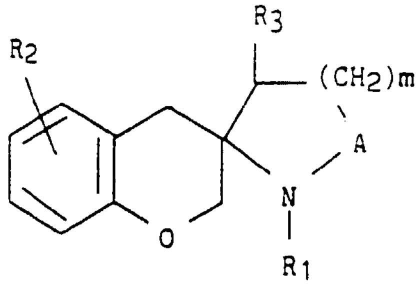

(I)

in der:

- m eine ganze Zahl mit Werten von 1 oder 2,
- A eine Methylengruppe ($CH_2$) oder Carbonylgruppe (CO).
- $R_1$:
  - Wasserstoff,
  - eine Gruppe $-CO-R_4$, in der $R_4$ eine geradkettige oder verzweigte Alkylgruppe mit 1 bis 6 Kohlenstoffatomen, eine gegebenenfalls substituierte Phenylgruppe oder eine gegebenenfalls substituierte Phenylalkylgruppe, deren Alkylkette 1 bis 3 Kohlenstoffatome aufweist,
  - oder eine geradkettige oder verzweigte Alkylgruppe mit 1 bis 6 Kohlenstoffatomen, welche gegebenenfalls substituiert ist durch:
    - eine Nitrilgruppe,
    - eine gegebenenfalls substituierte Phenylgruppe,
    - eine Gruppe $-NR_5R_6$, in der $R_5$ Wasserstoff oder eine geradkettige oder verzweigte Alkylgruppe mit 1 bis 6 Kohlenstoffatomen und $R_6$ Wasserstoff oder eine geradkettige oder verzweigte Alkylgruppe mit 1 bis 6 Kohlenstoffatomen, die gegebenenfalls durch eine gegebenenfalls substituierte Phenylgruppe, eine geradkettige oder verzweigte Alkylcarbonylgruppe mit 2 bis 7 Kohlenstoffatomen, eine gegebenenfalls substituierte Benzoylgruppe, eine gegebenenfalls substituierte Phenylalkylcarbonylgruppe, deren Alkylkette 1 bis 3 Kohlenstoffatome aufweist, eine geradkettige oder verzweigte Alkylsulfonylgruppe mit 1 bis 6 Kohlenstoffatomen, oder eine gegebenenfalls substituierte Phenylsulfonylgruppe substituiert ist, darstellen,
    - irgendeine der folgenden Gruppen:

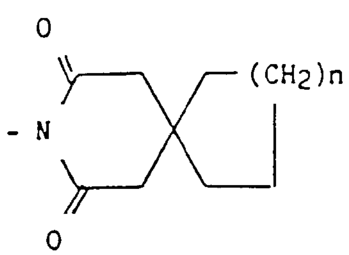

,

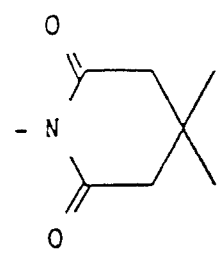

,

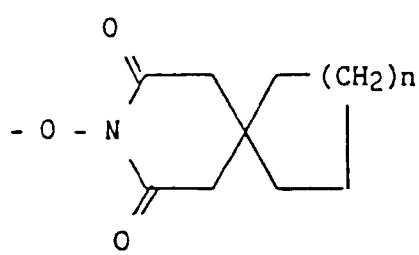

in denen:

- n eine ganze Zahl mit Werten von 1 oder 2 darstellt,

- $R_2$:

  - Wasserstoff,
  - eine Acetylgruppe,
  - eine Gruppe $CF_3SO_2$-O-, oder
  - eine Gruppe $-OR_7$, in der $R_7$ die gleichen Bedeutungen besitzt wie $R_1$, und

- $R_3$ Wasserstoff oder eine geradkettige oder verzweigte Alkylgruppe mit 1 bis 4 Kohlenstoffatomen mit der Maßgabe bedeuten, daß, wenn $R_1 = R_2 = R_3 = H$ ist, dann m nicht 1 sein kann,
  wobei der Begriff "gegebenenfalls substituiert" unter Bezugnahme auf die Begriffe Phenyl, Phenylalkyl, Phenylsulfonyl, Benzoyl oder Phenylalkylcarbonyl bedeutet, daß der aromatische Kern durch eine oder mehrere Niedrigalkylgruppen mit 1 bis 4 Kohlenstoffatomen in gerader oder verzweigter Kette, Nitrogruppen, Niedrigalkoxygruppen mit 1 bis 4 Kohlenstoffatomen, Halogenatomen, Trifluormethylgruppen oder Hydroxygruppen substituiert sein kann,
- deren Isomere, Diastereoisomere und Enantiomere in isolierter Form oder in Form einer Mischung.
- deren Additionssalze mit einer anorganischen oder organischen, pharmazeutisch annehmbaren Säure.

**2.** Verbindungen der allgemeinen Formel (I) nach Anspruch 1, worin m den Wert 1 besitzt, welche den substituierten Spiro[3-chroman:2'-pyrrolidin]-Verbindungen der folgenden allgemeinen Formel entsprechen:

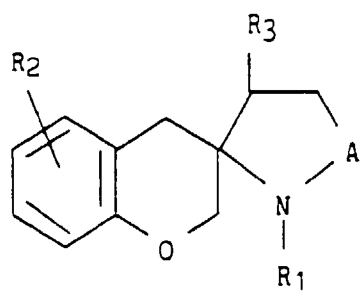

in der $R_1$, $R_2$, $R_3$ und A die in Anspruch 1 angegebenen Bedeutungen besitzen,

- deren Isomere, Diastereoisomere und Enantiomere in isolierter Form oder in Form einer Mischung,
- deren Additionssalze mit einer anorganischen oder organischen, pharmazeutisch annehmbaren Säure.

**3.** Verbindungen der allgemeinen Formel (I) nach Anspruch 1, worin m den Wert 2 besitzt, welche den substituierten Spiro[3-chroman:2'-piperidin]-Derivaten der folgenden allgemeinen Formel entsprechen:

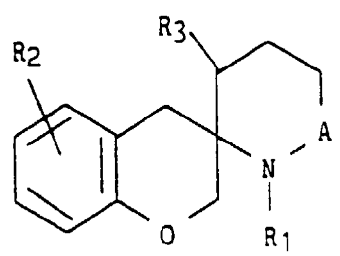

in der $R_1$, $R_2$, $R_3$ und A die in Anspruch 1 angegebenen Bedeutungen besitzen,

- deren Isomere, Diastereoisomere und Enantiomere in isolierter Form oder in Form einer Mischung,
- deren Additionssalze mit einer anorganischen oder organischen, pharmazeutisch annehmbaren Säure.

**4.** Verbindung nach Anspruch 1, nämlich Spiro[3,(5-methoxychroman):2'-{N-[4'-(8'-azaspiro[4',5']decan-7',9'-dion-8'-yl)-n-but-1'-yl]-pyrrolidin}], der nachstehenden Formel:

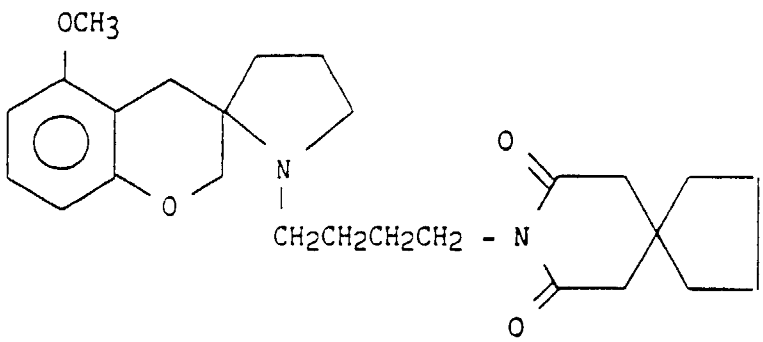

sowie dessen Enantiomere in isolierter Form oder in Form einer Mischung und dessen Additionssalze mit einer anorganischen oder organischen, pharmazeutisch annehmbaren Säure.

5. Verbindung nach Anspruch 1, nämlich das rechtsdrehende Isomere von Spiro[3-(5-methoxychroman):2'-{N,[4'-(8'-azaspiro[4',5']decan,7',9',dion-8'-yl)-n-but-1'-yl]-pyrrolidin}], der folgenden Formel:

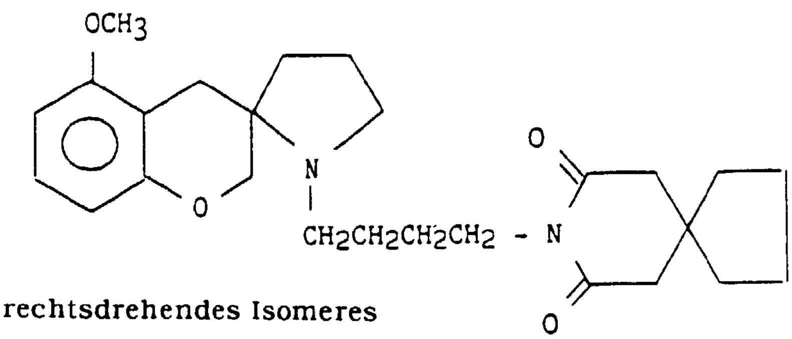

rechtsdrehendes Isomeres

sowie dessen Additionssalze mit einer anorganischen oder organischen, pharmazeutisch annehmbaren Säure.

6. Verbindung nach Anspruch 1, nämlich das linksdrehende Isomere von Spiro[3-(5-methoxychroman):2'-{N-[4',(8'-azaspiro[4',5']decan,7',9'-dion,8',yl)-n-but-1'-yl]-pyrrolidin}], der folgenden Formel:

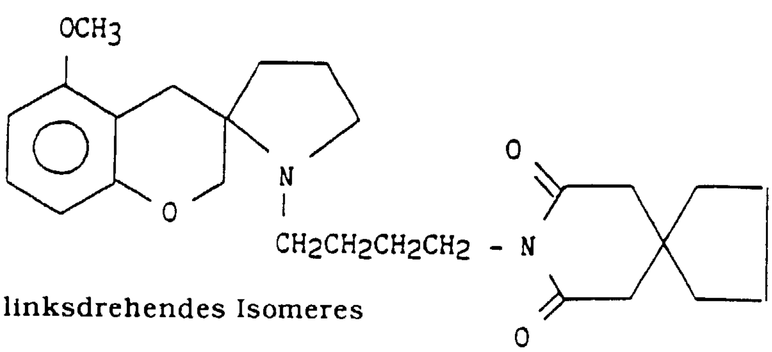

linksdrehendes Isomeres

sowie dessen Additionssalze mit einer anorganischen oder organischen, pharmazeutisch annehmbaren Säure.

7. Verbindung nach Anspruch 1, nämlich Spiro[3-(5-acetylchroman):2'-(N-propylpyrrolidin)] der nachfolgenden Formel:

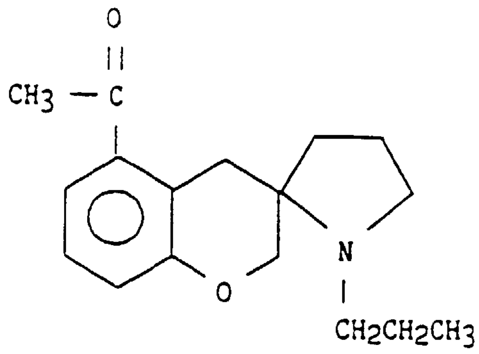

sowie dessen Enantiomere in isolierter Form oder in Form einer Mischung und dessen Additionssalze mit einer anorganischen oder organischen, pharmazeutisch annehmbaren Säure.

**8.** Verbindung nach Anspruch 1, nämlich Spiro[3-(5-methoxychroman):2'-pyrrolidin], der Formel:

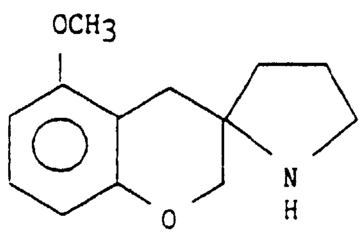

sowie dessen Enantiomere in isolierter Form oder in Form einer Mischung und dessen Additionssalze mit einer anorganischen oder organischen, pharmazeutisch annehmbaren Säure.

**9.** Verbindung nach Anspruch 1, nämlich Spiro[3-(5-methoxychroman):2'-piperidin], der Formel:

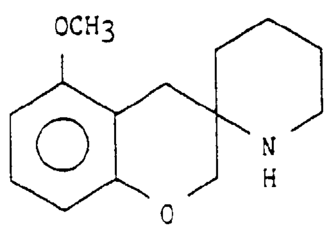

sowie dessen Enantiomere in isolierter Form oder in Form einer Mischung und dessen Additionssalze mit einer anorganischen oder organischen, pharmazeutisch annehmbaren Säure.

**10.** Verbindung nach Anspruch 1, nämlich Spiro[3-(5-hydroxychroman):2'-(N-propyl-pyrrolidin)], der Formel:

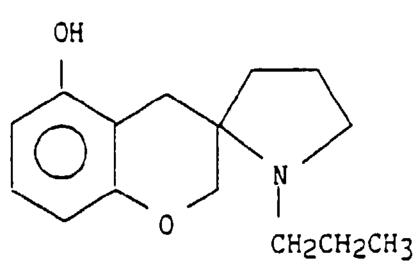

sowie dessen Enantiomere in isolierter Form oder in Form einer Mischung und dessen Additionssalze mit einer anorganischen oder organischen, pharmazeutisch annehmbaren Säure.

**11.** Verbindung nach Anspruch 1, nämlich Spiro[3-(5-methoxychroman):2'-(N-propyl-pyrrolidin], der Formel:

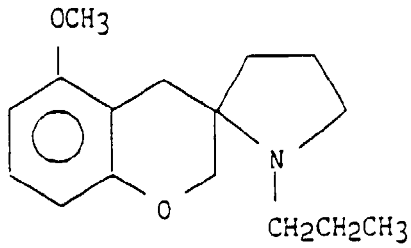

sowie dessen Enantiomere in isolierter Form oder in Form einer Mischung und dessen Additionssalze mit einer anorganischen oder organischen, pharmazeutisch annehmbaren Säure.

**12.** Verbindung nach Anspruch 1, nämlich Spiro[3-{5-[4-(8-azaspiro[4,5]decan-7,9-dion-8-yl)-n-butyloxy]-chroman}:2'-(N-propyl-pyrrolidin)], der Formel:

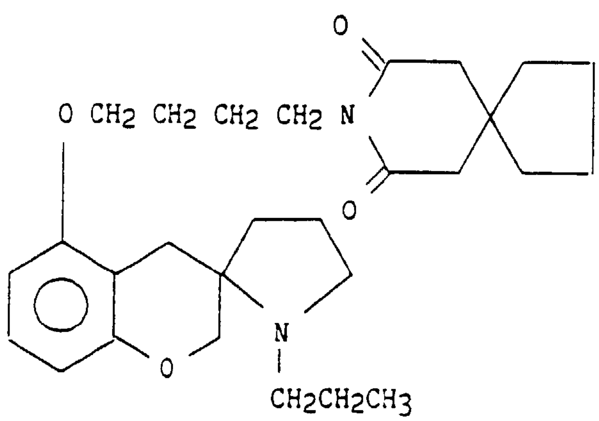

sowie dessen Enantiomere in isolierter Form oder in Form einer Mischung und dessen Additionssalze mit einer anorganischen oder organischen, pharmazeutisch annehmbaren Säure.

**13.** Verfahren zur Herstellung der Verbindungen der allgemeinen Formel (Ia):

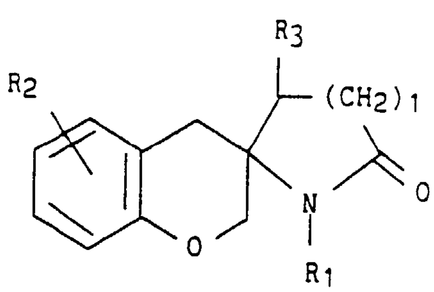

(Ia)

in der $R_1$, $R_2$ und $R_3$ die in Anspruch 1 angegebenen Bedeutungen besitzen, einem Sonderfall der Verbindungen der Formel (I), in der m den Wert 1 besitzt und A eine Carbonylgruppe darstellt (CO),
dadurch gekennzeichnet, daß man einen substituierten Benzaldehyd der allgemeinen Formel (II):

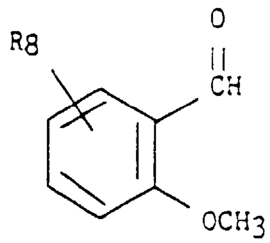

(II)

in der $R_8$ Wasserstoff oder eine geradkettige oder verzweigte Alkoxygruppe mit 1 bis 6 Kohlenstoffatomen darstellt, bei einer Temperatur zwischen -5° und 0°C mit Bortribromid in der Weise umsetzt, daß man die Verbindung der allgemeinen Formel (III) erhält:

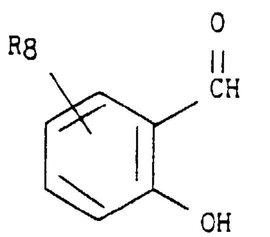

(III)

in der $R_8$ die oben angegebenen Bedeutungen besitzt, welche man in der Wärme und in Gegenwart eines substituierten Ammoniumsalzes mit 2-Nitroethanol in der Weise umsetzt, daß man die Verbindung der allgemeinen Formel (IV) erhält:

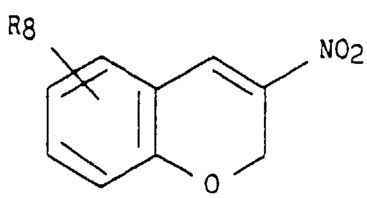

(IV)

in der $R_8$ die oben angegebenen Bedeutungen besitzt, welche man in der Weise reduziert, daß man die Verbindung der allgemeinen Formel (V) erhält:

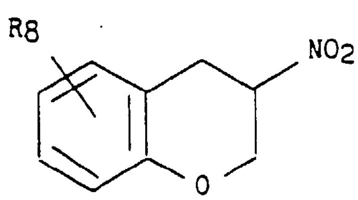

(V)

in der $R_8$ die oben angegebenen Bedeutungen besitzt, welche man in Gegenwart von Benzyltrimethylammoniummethylat in alkoholischem Medium mit einer Acrylverbindung der allgemeinen Formel (VI) umsetzt:

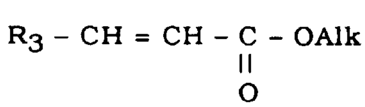

(VI)

in der $R_3$ die oben angegebenen Bedeutungen besitzt und Alk eine Alkylgruppe mit 1 bis 4 Kohlenstoffatomen darstellt, so daß man die Verbindung der allgemeinen Formel (VIIa) erhält:

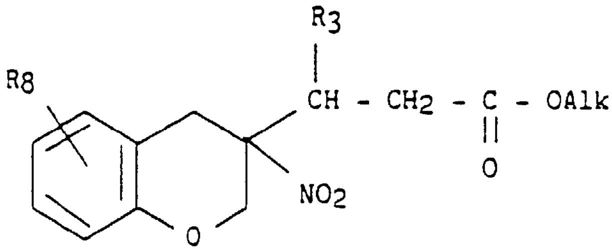

(VIIa)

in der $R_3$, $R_8$ und Alk die oben angegebenen Bedeutungen besitzen, welche man in Gegenwart von Raney-Nickel unter einer Wasserstoffatmosphäre in der Weise reduziert, daß man nach der Isolierung und der eventuellen Reinigung die Spiranverbindung der allgemeinen Formel (VIIIa) erhält:

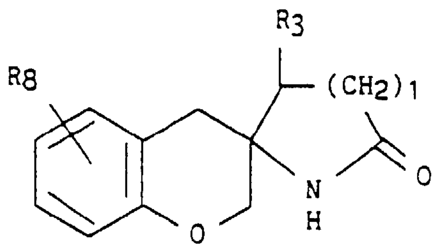

(VIIIa)

in der $R_3$ und $R_8$ die oben angegebenen Bedeutungen besitzen,
welche man in Gegenwart einer starken Base mit einer Verbindung der allgemeinen Formel (IX):

$$Hal'R'_1 \qquad (IX)$$

in der $R'_1$ die für $R_1$ in Anspruch 1 angegebenen Bedeutungen mit der Maßgabe besitzt, daß $R'_1$ nicht Wasserstoff bedeutet, und Hal' ein Halogenatom darstellt, in der Weise umsetzt, daß man nach der Isolierung und der eventuellen Reinigung die Verbindung der allgemeinen Formel (Xa) erhält:

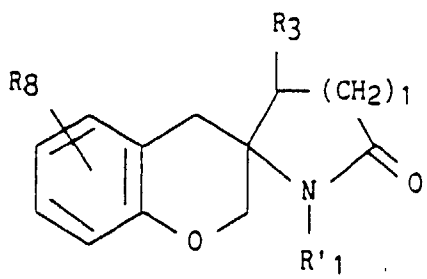

(Xa)

in der $R'_1$, $R_3$ und $R_8$ die oben angegebenen Bedeutungen besitzen, welche Verbindungen der Formeln (VIIIa) und (Xa) man in dem Fall, da $R_8$ eine Alkoxygruppe darstellt, mit einer wäßrigen Bromwasserstoffsäurelösung behandeln kann, so daß man nach der Isolierung und der eventuellen Reinigung die Verbindung der allgemeinen Formel (XIa) erhält:

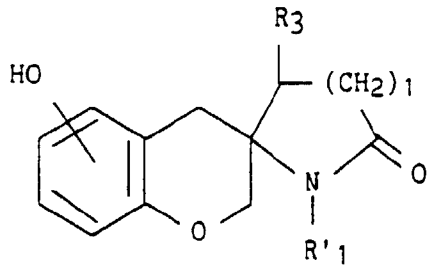

(XIa)

in der $R'_1$ und $R_3$ die oben angegebenen Bedeutungen besitzen, welche man mit einer Verbindung der allgemeinen Formel (XII):

$$Hal'' - R'_7 \qquad (XII)$$

in der Hal" ein Halogenatom darstellt und $R'_7$ die für $R_7$ in Anspruch 1 angegebenen Bedeutungen mit der Maßgabe besitzt, daß $R'_7$ nicht Wasserstoff darstellt, in der Weise umsetzt, daß man nach der Isolierung und der eventuellen Reinigung die Verbindung der allgemeinen Formel (XIIIa) erhält:

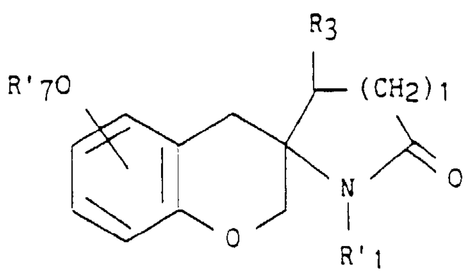

(XIIIa)

in der $R'_1$, $R_3$ und $R'_7$ die oben angegebenen Bedeutungen besitzen, wobei es sich versteht, daß die Verbindungen der allgemeinen Formeln VIIIa, Xa, XIa und XIIIa, die ebenfalls Gegenstand der Erfindung sind, die Verbindungen der allgemeinen Formel (Ia) darstellen, wie sie oben definiert worden ist, und gewünschtenfalls gereinigt, in ihre Isomeren aufgetrennt oder, wenn dies möglich ist, mit einer pharmazeutisch annehmbaren Säure in ihre Salze überführt werden können.

**14.** Verfahren zur Herstellung der Verbindungen der allgemeinen Formel (Ib):

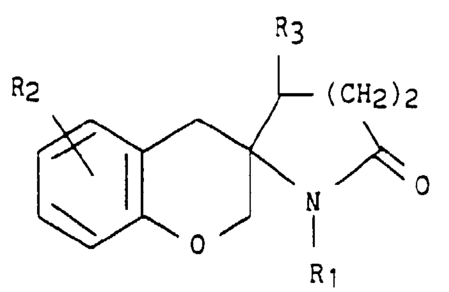

(Ib)

in der $R_1$, $R_2$ und $R_3$ die in Anspruch 1 angegebenen Bedeutungen besitzen, einem Sonderfall der Verbindungen der Formel (I), in der m den Wert 2 besitzt und A eine Carbonylgruppe (CO) darstellt,
dadurch gekennzeichnet, daß man eine Verbindung der Formel (V):

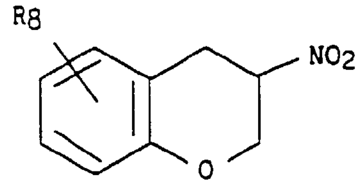

(V)

in der $R_8$ Wasserstoff oder eine geradkettige oder verzweigte Alkoxygruppe mit 1 bis 6 Kohlenstoffatomen darstellt, mit einem Halogenester der allgemeinen Formel (X):

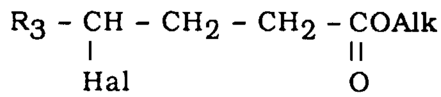

(X)

in der $R_3$ die oben angegebenen Bedeutungen besitzt, Alk eine Alkylgruppe mit 1 bis 4 Kohlenstoffatomen und Hal ein Halogenatom bedeuten, in der Weise umsetzt, daß man die Verbindung der allgemeinen Formel (VIIb) erhält:

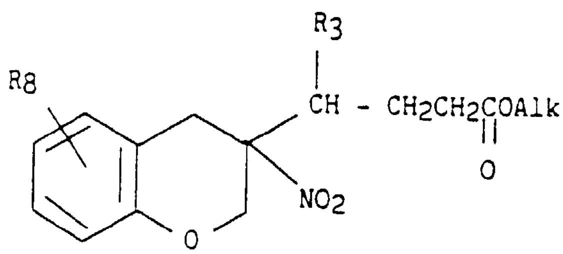

(VIIb)

in der $R_3$, $R_8$ und Alk die oben angegebenen Bedeutungen besitzen, welche man in Gegenwart von Raney-Nickel unter einer Wasserstoffatmosphäre in der Weise reduziert, daß man nach der Isolierung und der eventuellen Reinigung die Spiranverbindung der allgemeinen Formel (VIIIb) erhält:

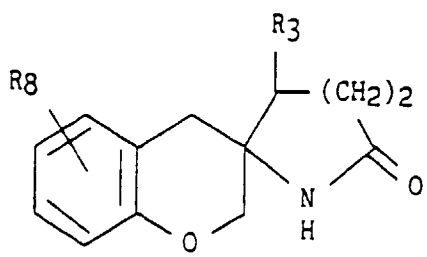

(VIIIb)

in der $R_3$ und $R_8$ die oben angegebenen Bedeutungen besitzen, welche man in Gegenwart einer starken Base mit einer Verbindung der allgemeinen Formel (IX):

$$Hal'R'_1 \qquad (IX)$$

in der $R'_1$ die in Anspruch 1 angegebenen Bedeutungen von $R_1$ mit der Maßgabe besitzt, daß $R'_1$ nicht Wasserstoff bedeutet und Hal' ein Halogenatom darstellt, in der Weise umsetzt, daß man nach der Isolierung und der eventuellen Reinigung die Verbindung der allgemeinen Formel (Xb) erhält:

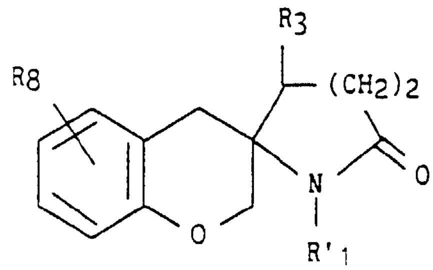

(Xb)

in der $R'_1$, $R_3$ und $R_8$ die oben angegebenen Bedeutungen besitzen, welche Verbindungen der Formeln (VIIIb) und (Xb) man dann, wenn $R_8$ eine Alkoxygruppe darstellt, mit einer wäßrigen Bromwasserstoffsäurelösung behandeln kann, so daß man nach der Isolierung und der eventuellen Reinigung die Verbindung der allgemeinen Formel (XIb) erhält:

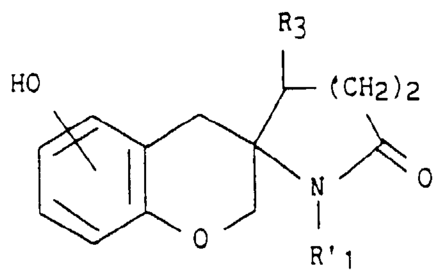

(XIb)

in der $R'_1$ und $R_3$ die oben angegebenen Bedeutungen besitzen, welche man mit einer Verbindung der allgemeinen Formel (XII):

$$Hal''\text{-}R'_7 \qquad (XII)$$

in der Hal" ein Halogenatom darstellt und $R'_7$ die in Anspruch 1 angegebenen Bedeutungen von $R_7$ mit der Maßgabe besitzt, daß $R'_7$ nicht Wasserstoff bedeutet, in der Weise umsetzt, daß man nach der Isolierung und der eventuellen Reinigung die Verbindung der allgemeinen Formel (XIIIb) erhält:

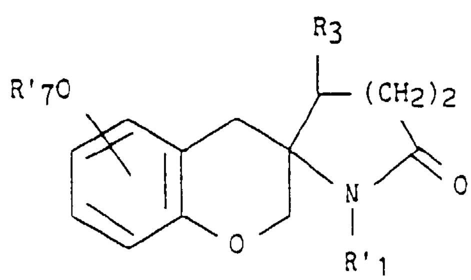

(XIIIb)

in der $R'_1$, $R_3$ und $R'_7$ die oben angegebenen Bedeutungen besitzen,
wobei es sich versteht, daß die Verbindungen der allgemeinen Formeln VIIIb, Xb, XIb und XIIIb, die ebenfalls Gegenstand der Erfindung sind, die Verbindungen der allgemeinen Formel (Ib), wie sie oben definiert worden ist, darstellen, und gewünschtenfalls gereinigt, in ihre Isomeren aufgetrennt oder, wenn dies möglich ist, mit einer pharmazeutisch annehmbaren Säure in ihre Salze überführt werden können.

**15.** Verfahren zur Herstellung der Verbindungen der allgemeinen Formel (Ic):

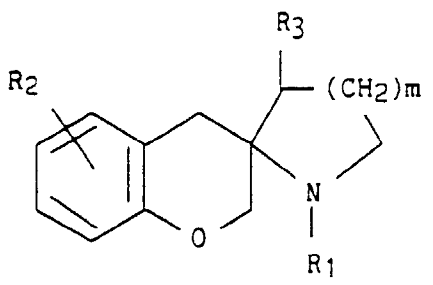

(Ic)

in der $R_1$, $R_2$, $R_3$ und m die in Anspruch 1 angegebenen Bedeutungen besitzen, einem Sonderfall der Verbindungen der Formel (I), in der A eine Methylengruppe ($CH_2$) darstellt,
dadurch gekennzeichnet, daß man eine Verbindung der Formel (VIII):

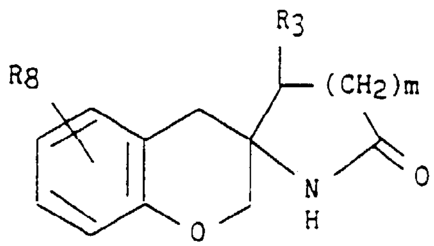

(VIII)

in der $R_3$ und m die oben angegebenen Bedeutungen besitzen und $R_8$ Wasserstoff oder eine geradkettige oder verzweigte Alkoxygruppe mit 1 bis 6 Kohlenstoffatomen darstellt, in einem aprotischen Medium mit einem Reduktionsmittel in der Weise reduziert, daß man nach der Isolierung und der eventuellen Reinigung die Verbindung der allgemeinen Formel (XIV) erhält:

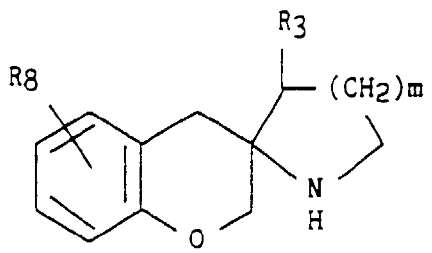

(XIV)

in der $R_3$, $R_8$ und m die oben angegebenen Bedeutungen besitzen, welche man mit einer Verbindung der allgemeinen Formel (IX):

$$Hal'R'_1 \qquad (IX)$$

in der $R'_1$ die in Anspruch 1 angegebenen Bedeutungen von $R_1$ mit der Maßgabe besitzt, daß $R'_1$ nicht Wasserstoff bedeutet und Hal' ein Halogenatom darstellt, in der Weise umsetzt, daß man nach der Isolierung und der eventuellen Reinigung die Verbindung der allgemeinen Formel (XV) erhält:

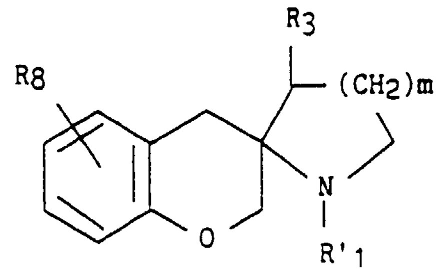

(XV)

in der $R'_1$, $R_3$, $R_8$ und m die oben angegebenen Bedeutungen besitzen,
welche Verbindungen der Formeln (XIV) und (XV) man dann, wenn $R_8$ eine Alkoxygruppe darstellt, mit einer wäßrigen Bromwasserstoffsäurelösung behandeln kann, so daß man nach der Isolierung und der eventuellen Reinigung die Verbindung der allgemeinen Formel (XVI) erhält:

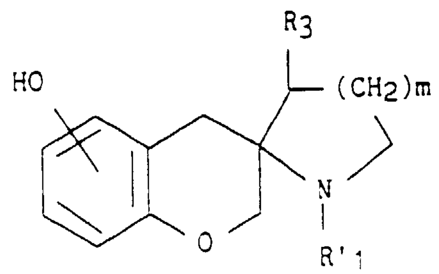

(XVI)

in der $R'_1$, $R_3$ und m die oben angegebenen Bedeutungen besitzen, welche man mit einer Verbindung der allgemeinen Formel (XII):

$$Hal''\text{-}R'_7 \qquad (XII)$$

in der Hal" ein Halogenatom darstellt und $R'_7$ die in Anspruch 1 angegebenen Bedeutungen von $R_7$ besitzt, mit der Maßgabe, daß $R'_7$ nicht Wasserstoff bedeutet, in der Weise umsetzt, daß man nach der Isolierung und der eventuellen Reinigung die Verbindung der allgemeinen Formel (XVII) erhält:

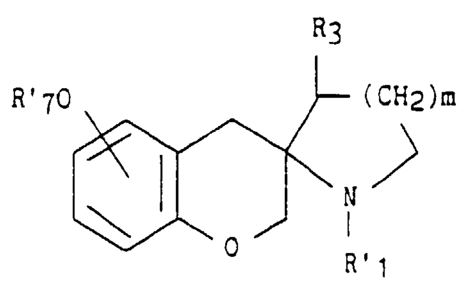

(XVII)

in der $R'_1$, $R_3$, $R'_7$ und m die oben angegebenen Bedeutungen besitzen,
wobei es sich versteht, daß die Verbindungen der allgemeinen Formeln XIV, XV, XVI und XVII, die Gegenstand der Erfindung sind, die Verbindungen der allgemeinen Formel (Ic) darstellen und gewünschtenfalls gereinigt, in ihre Isomeren aufgetrennt und, wenn dies möglich ist, mit einer pharmazeutisch annehmbaren Säure in ihre Salze überführt werden können.

**16.** Verfahren zur Herstellung der Verbindungen der allgemeinen Formel (Id):

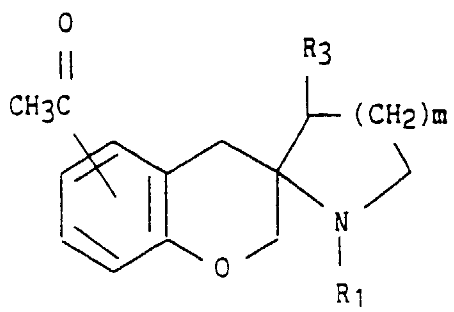

(Id)

in der $R_1$, $R_3$ und m die in Anspruch 1 angegebenen Bedeutungen besitzen,
einem Sonderfall der Verbindungen der Formel (I), in der $R_2$ eine Acetylgruppe darstellt, dadurch gekennzeichnet, daß man eine Verbindung der allgemeinen Formel (XVIII):

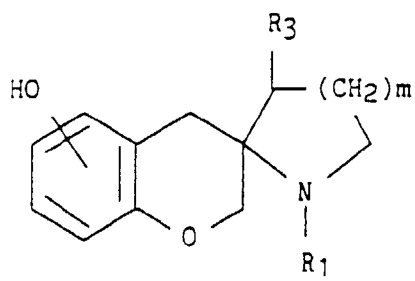

(XVIII)

in der $R_1$, $R_2$ und m die in Anspruch 1 angegebenen Bedeutungen besitzen, in der Weise mit Trifluormethansulfonsäureanhydrid umsetzt, daß man das Trifluormethylsulfonyloxy-Derivat der allgemeinen Formel (XIX) erhält:

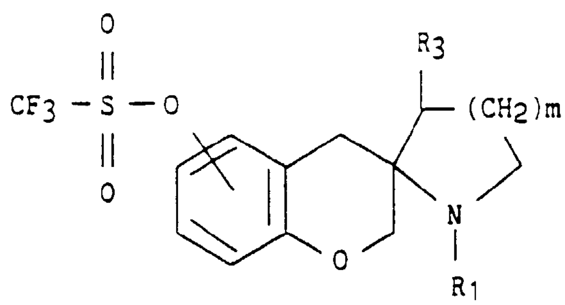

(XIX)

in der $R_1$, $R_3$ und m die in Anspruch 1 angegebenen Bedeutungen besitzen, welche man anschließend in einem aprotischen Medium und in Gegenwart von Triethylamin, 1,2-Bis(diphenylphosphino)-ethan und Pd $(OAc)_2$ in der Weise mit Butylvinylether umsetzt, daß man nach der Isolierung und der Reinigung die Acetylverbindung der allgemeinen Formel (XX) erhält:

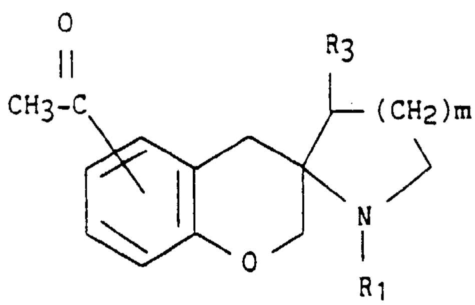

(XX)

in der $R_1$, $R_3$ und m die in Anspruch 1 angegebenen Bedeutungen besitzen,
wobei es sich versteht, daß die Verbindungen der allgemeinen Formel (XIX) und (XX) Gegenstand der Erfindung sind und gewünschtenfalls gereinigt, in ihre Isomeren aufgetrennt und, wenn dies möglich ist, mit einer pharmazeutisch annehmbaren Säure in ihre Salze überführt werden können.

**17.** Pharmazeutische Zubereitungen enthaltend als Wirkstoff mindestens eine Verbindung nach irgendeinem der Ansprüche 1 bis 12 allein oder in Kombination mit einem oder mehreren inerten, nichttoxischen, pharmazeutisch annehmbaren Trägermaterialien oder Bindemitteln.

**18.** Pharmazeutische Zubereitungen nach Anspruch 17, enthaltend mindestens einen Wirkstoff nach einem der Ansprüche 1 bis 12 zur Behandlung von Depressionen, Streß, Psychosen, der Angst, der Schizophrenie, der Hypertension, von Schmerzen, kardiovaskulären Erkrankungen, Migräne, Zerebralischämie und als Mittel zur Modifizierung des Ernährungs- und Sexualverhaltens.

**Claims**

**1.** Spirochroman compounds of the general formula (I):

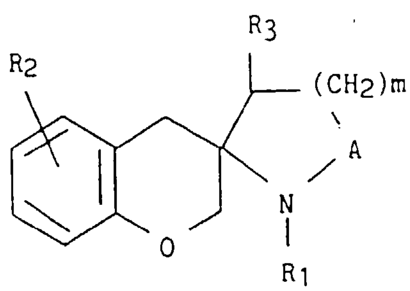

(I)

wherein:

- m, which is an integer, may have the values 1 or 2,
- A represents a methylene group ($CH_2$) or a carbonyl group (CO),
- $R_1$ represents:
    - a hydrogen atom,
    - a -CO-$R_4$ group wherein $R_4$ represents a linear or branched alkyl group having from 1 to 6 carbon atoms, an optionally substituted phenyl group or an optionally substituted phenylalkyl group, the alkyl chain of which has from 1 to 3 carbon atoms, or
    - a linear or branched alkyl group having from 1 to 6 carbon atoms optionally substituted by:
        - a nitrile group,
        - an optionally substituted phenyl group,
        - a -$NR_5R_6$ group wherein $R_5$ represents a hydrogen atom or a linear or branched alkyl group having

from 1 to 6 carbon atoms and $R_6$ represents a hydrogen atom, a linear or branched alkyl group having from 1 to 6 carbon atoms optionally substituted by an optionally substituted phenyl group, a linear or branched alkylcarbonyl group having from 2 to 7 carbon atoms, an optionally substituted benzoyl group, an optionally substituted phenylalkylcarbonyl group, the alkyl chain of which has from 1 to 3 carbon atoms, a linear or branched alkylsulphonyl group having from 1 to 6 carbon atoms or an optionally substituted phenylsulphonyl group,

- any one of the following groups:

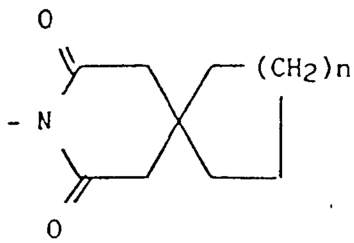
,
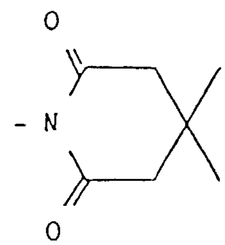
,
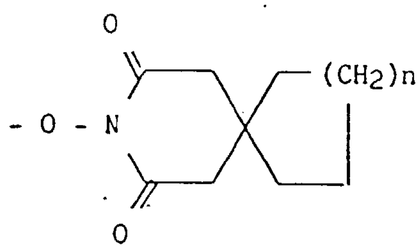

wherein:

- n, which is an integer, may have the values 1 or 2,

- $R_2$ represents:
  - a hydrogen atom,
  - an acetyl group,
  - a $CF_3SO_2$-O- group, or
  - an $-OR_7$ group wherein $R_7$ has the same definition as $R_1$,

- R3 represents a hydrogen atom or a linear or branched alkyl group having from 1 to 4 carbon atoms, with the proviso that when $R_1=R_2=R_3=H$, then m cannot be 1,

the term "optionally substituted" associated with the terms "phenyl", "phenylalkyl", "phenylsulphonyl", "benzoyl" and "phenylalkylcarbonyl" means that the aromatic nucleus may be substituted by one or more branched or unbranched lower alkyls having from 1 to 4 carbon atoms, nitro, lower alkoxy having from 1 to 4 carbon atoms, halogen, trifluoromethyl, hydroxy,

- their isomers, diastereoisomers and enantiomers, isolated or in the form of a mixture,

- addition salts thereof with a pharmaceutically acceptable mineral acid or organic acid.

**2.** Compounds of general formula (I), according to claim 1, wherein m is 1, which corresponds to the substituted spiro [chroman-3:2'-pyrrolidines] of the following general formula:

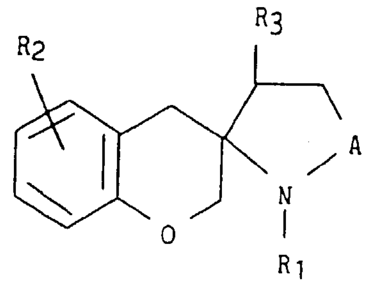

wherein $R_1$, $R_2$, $R_3$ and A have the same meanings as in claim 1,

- their isomers, diastereoisomers and enantiomers, isolated or in the form of a mixture,
- addition salts thereof with a pharmaceutically acceptable mineral acid or organic acid.

**3.** Compounds of general formula (I) according to claim 1 wherein m is 2, which corresponds to the substituted spiro [chroman-3:2'-piperidines] of the following general formula:

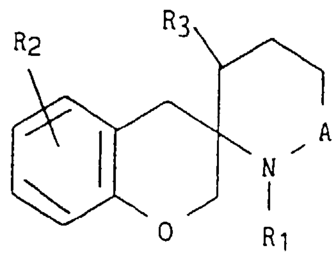

wherein $R_1$, $R_2$, $R_3$ and A have the same meanings as in claim 1,

- their isomers, diastereoisomers and enantiomers, isolated or in the form of a mixture,
- addition salts thereof with a pharmaceutically acceptable mineral acid or organic acid.

**4.** Compound according to claim 1 which is spiro[(5-methoxychroman)-3:2'-{N-[4'-(8'-azaspiro[4',5']decane-7',9'-dion-8'-yl)-n-but-1'-yl]pyrrolidine}], the formula of which is given below, and its enantiomers, isolated or in the form of a mixture, and addition salts thereof with a pharmaceutically acceptable mineral acid or organic acid.

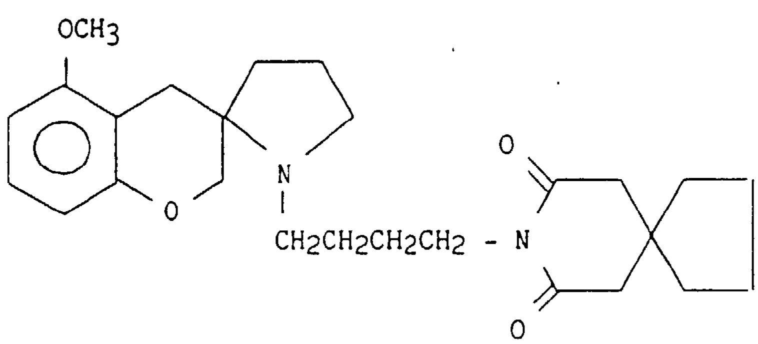

**5.** Compound according to claim 1 which is the dextrorotatory isomer of spiro[(5-methoxychroman)-3:2'-{N-[4'-(8'-azaspiro[4',5']decane-7',9'-dion-8'-yl)-n-but-1'-yl]pyrrolidine}], the formula of which is given below, and its addition

salts with a pharmaceutically acceptable mineral acid or organic acid.

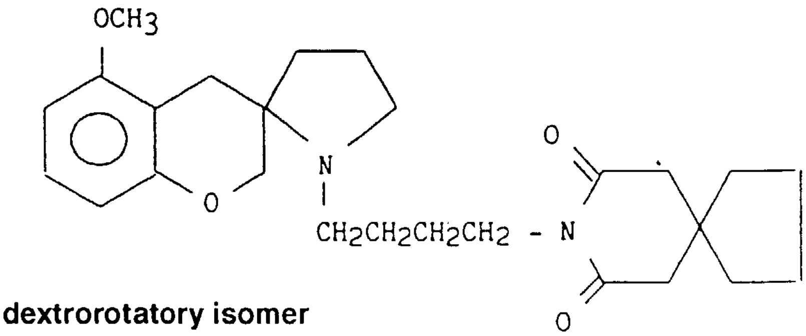

dextrorotatory isomer

6. Compound according to claim 1 which is the laevorotatory isomer of spiro[(5-methoxychroman)-3:2'-{N-[4'-(8'-azaspiro[4',5']decane,7',9'-dion-8'-yl)-n-but-1'-yl]pyrrolidine}], the formula of which is given below, and its addition salts with a pharmaceutically acceptable mineral acid or organic acid.

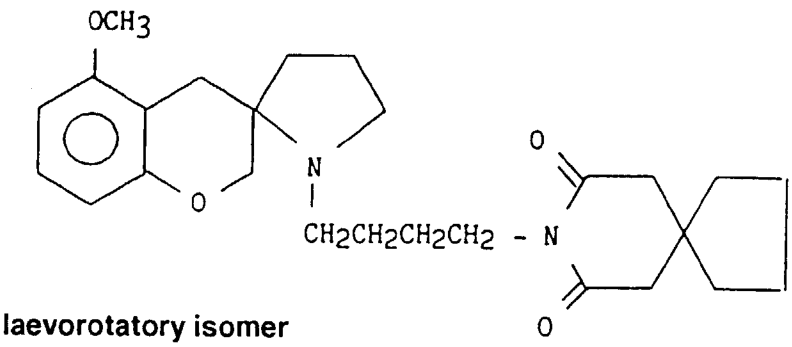

laevorotatory isomer

7. Compound according to claim 1 which is spiro[(5-acetylchroman)-3:2'-(N-propylpyrrolidine)], the formula of which is given below, and its enantiomers, isolated or in the form of a mixture, and addition salts thereof with a pharmaceutically acceptable mineral acid or organic acid.

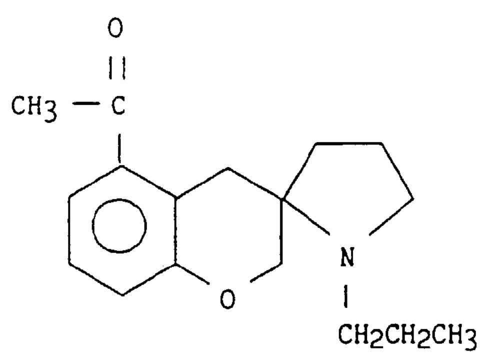

8. Compound according to claim 1 which is spiro[(5-methoxychroman)-3:2'-pyrrolidine], the formula of which is given below, and its enantiomers, isolated or in the form of a mixture, and addition salts thereof with a pharmaceutically acceptable mineral acid or organic acid.

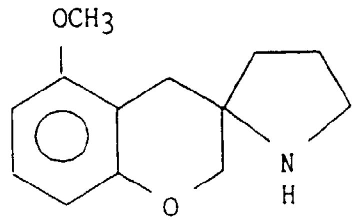

9. Compound according to claim 1 which is spiro[(5-methoxychroman)-3:2'-piperidine], the formula of which is given below, and its enantiomers, isolated or in the form of a mixture, and addition salts thereof with a pharmaceutically acceptable mineral acid or organic acid.

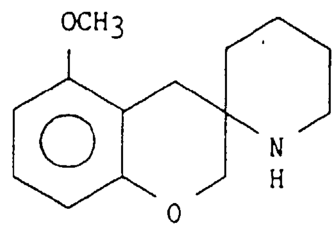

10. Compound according to claim 1 which is spiro[(5-hydroxychroman)-3:2'-(N-propylpyrrolidine)], the formula of which is given below, and its enaniomers, isolated or in the form of a mixture, and addition salts thereof with a pharmaceutically acceptable mineral acid or organic acid.

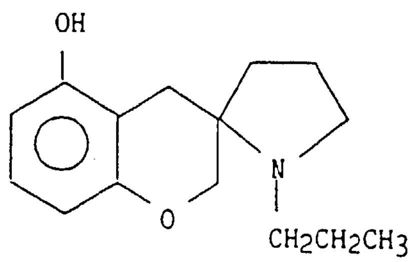

11. Compound according to claim 1 which is spiro[(5-methoxychroman)-3:2'-(N-propylpyrrolidine)], the formula of which is given below, and its enantiomers, isolated or in the form of a mixture, and addition salts thereof with a pharmaceutically acceptable mineral acid or organic acid.

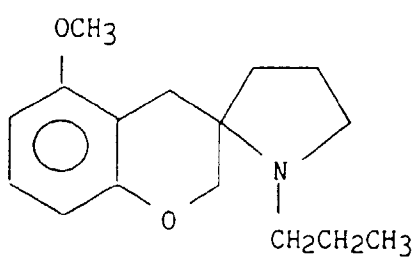

12. Compound according to claim 1 which is spiro[{5-[4-(8-azaspiro[4,5]decane-7,9-dion-8-yl)-n-butyloxy]chroman}-3:2'-(N-propylpyrrolidine)], the formula of which is given below, and its enantiomers, isolated or in the form of a mixture, and addition salts thereof with a pharmaceutically acceptable mineral acid or organic acid.

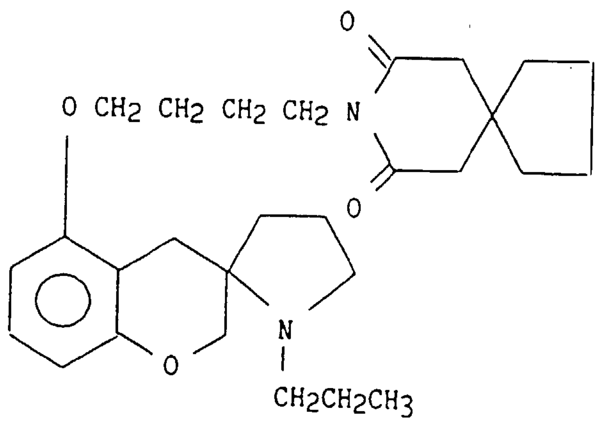

**13.** Process for obtaining compounds of the general formula (Ia):

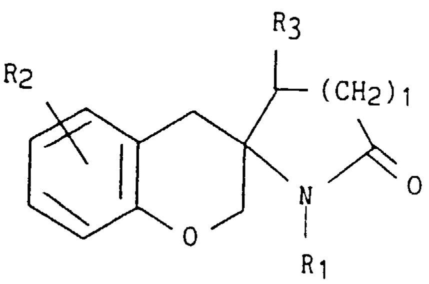

(Ia)

wherein $R_1$, $R_2$ and $R_3$ are as defined in claim 1, which is a particular case of the compounds of formula (I) wherein m is 1 and A represents a carbonyl group (CO), characterised in that a substituted benzaldehyde of the general formula (II):

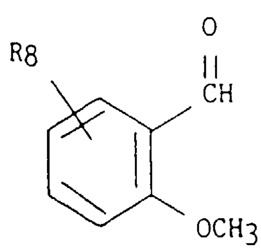

(II)

wherein $R_8$ represents a hydrogen atom or a linear or branched alkoxy group having from 1 to 6 carbon atoms, is reacted, at a temperature of from -5° to 0°C, with boron tribromide so as to obtain a compound of the general formula (III):

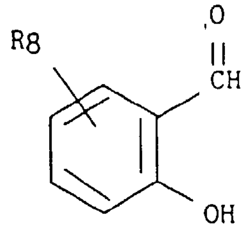

(III)

wherein $R_8$ has the same meaning as above, which is reacted, with heating and in the presence of a substituted ammonium salt, with 2-nitroethanol, so as to obtain a compound of the general formula (IV):

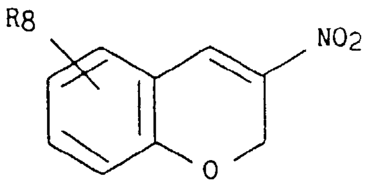

(IV)

wherein $R_8$ has the same meaning as above, which is reduced so as to obtain a compound of the general formula (V):

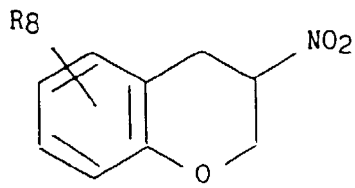

(V)

wherein $R_8$ has the same meaning as above, which is reacted in the presence of benzyltrimethylammonium methylate, in an alcoholic medium, with an acrylic compound of the general formula (VI):

$$R_3 - CH = CH - \underset{\underset{O}{\|}}{C} - OAlk \qquad (VI)$$

wherein $R_3$ has the same meaning as above and Alk represents an alkyl group having from 1 to 4 carbon atoms, so as to obtain compounds of the general formula (VIIa):

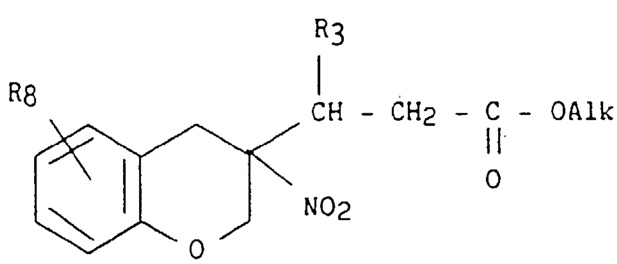

(VIIa)

wherein $R_3$, $R_8$ and Alk have the same meanings as above, which are reduced, in the presence of Raney nickel and under a hydrogen atmosphere, so as to obtain, after isolation and optional purification, a spiran compound of the general formula (VIIIa):

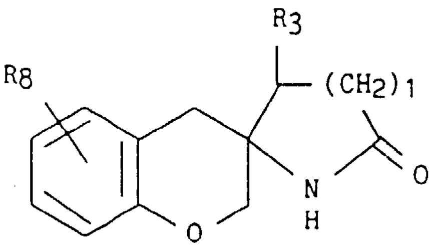

(VIIIa)

wherein $R_3$ and $R_8$ have the same meanings as above,
which may be reacted, in the presence of a strong base, with a compound of the general formula (IX):

$$Hal'R'_1 \qquad (IX)$$

wherein $R'_1$ has the same meaning as $R_1$ in claim 1, with the proviso that $R'_1$ cannot represent a hydrogen atom, and Hal' represents a halogen atom, so as to obtain, after isolation and optional purification, a compound of the general formula (Xa):

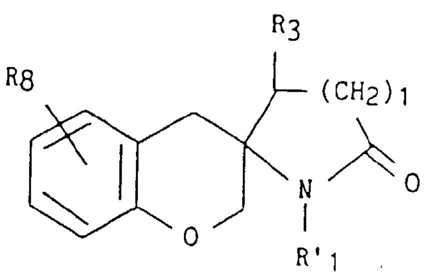

(Xa)

wherein $R'_1$, $R_3$ and $R_8$ have the same meanings as above, which compounds of formulae (VIIIa) and (Xa) may, when $R_8$ represents an alkoxy group, be treated with an aqueous hydrobromic acid solution, to obtain, after isolation and optional purification, a compound of the general formula (XIa):

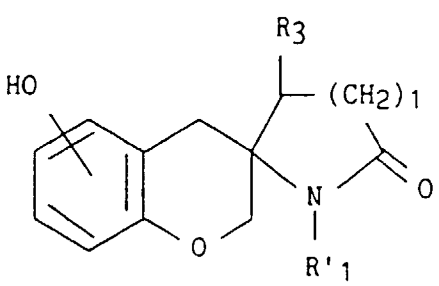

(XIa)

wherein $R'_1$ and $R_3$ have the same meanings as above, which may be reacted with a compound of the general formula (XII):

$$Hal''-R'_7 \qquad (XII)$$

wherein Hal" represents a halogen atom and $R'_7$ has the same meaning as $R_7$ in claim 1, with the proviso that $R'_7$ cannot represent a hydrogen atom, so as to obtain, after isolation and optional purification, a compound of the general formula (XIIIa):

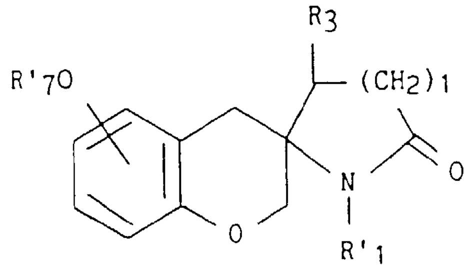

(XIIIa)

wherein $R'_1$, $R_3$ and $R'_7$ have the same meanings as above,
it being understood that the compounds of general formulae VIIIa, Xa, XIa and XIIIa form part of the invention, constitute the compounds of the general formula (Ia) as defined above and may, if desired, be purified, separated into their isomers or, if possible, converted into salts with a pharmaceutically acceptable acid.

**14.** Process for obtaining compounds of the general formula (Ib):

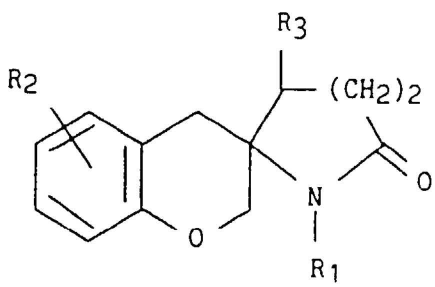

(Ib)

wherein $R_1$, $R_2$ and $R_3$ are as defined in claim 1,
which is a particular case of the compounds of formula (I) wherein m is 2 and A represents a carbonyl group (CO), characterised in that a compound of formula (V):

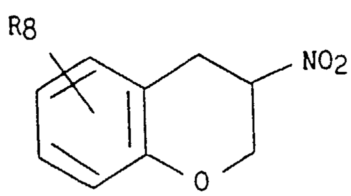

(V)

wherein $R_8$ represents a hydrogen atom or a linear or branched alkoxy group having from 1 to 6 carbon atoms, is reacted with a halo ester of the general formula (X):

$$R_3 - \underset{\substack{|\\ Hal}}{CH} - CH_2 - CH_2 - \underset{\substack{\|\\ O}}{C}OAlk \qquad (X)$$

wherein $R_3$ has the same meaning as above, Alk represents an alkyl group having from 1 to 4 carbon atoms and Hal represents a halogen atom, so as to obtain the compounds of the general formula (VIIb):

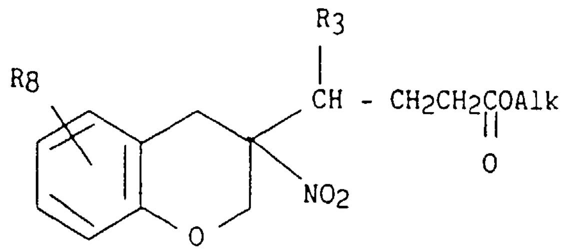

(VIIb)

wherein $R_3$, $R_8$ and Alk have the same meanings as above,
which are reduced, in the presence of Raney nickel and under a hydrogen atmosphere, so as to obtain, after isolation and optional purification, a spiran compound of the general formula (VIIIb):

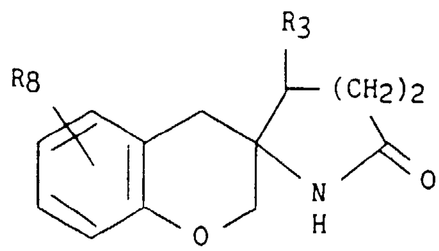

(VIIIb)

wherein $R_3$ and $R_8$ have the same meanings as above, which may be reacted, in the presence of a strong base, with a compound of the general formula (IX):

$$Hal'R'_1 \quad (IX)$$

wherein $R'_1$ has the same meaning as $R_1$ in claim 1, with the proviso that $R'_1$ cannot represent a hydrogen atom, and Hal' represents a halogen atom, so as to obtain, after isolation and optional purification, a compound of the general formula (Xb):

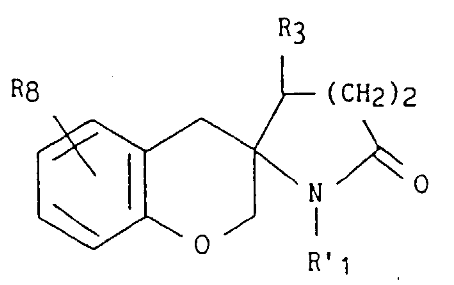

(Xb)

wherein $R_1'$, $R_3$ and $R_8$ have the same meanings as above,
which compounds of formulae (VIIIb) and (Xb) may, when $R_8$ represents an alkoxy group, be treated with an aqueous hydrobromic acid solution, to obtain, after isolation and optional purification, a compound of the general formula (XIb):

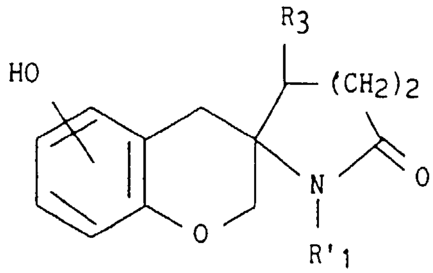

(XIb)

wherein $R'_1$ and $R_3$ have the same meanings as above, which may be reacted with a compound of the general formula (XII):

$$Hal'' - R'_7 \qquad (XII)$$

wherein Hal" represents a halogen atom and $R'_7$ has the same meaning as $R_7$ in claim 1, with the proviso that $R'_7$ cannot represent a hydrogen atom, so as to obtain, after isolation and optional purification, a compound of the general formula (XIIIb):

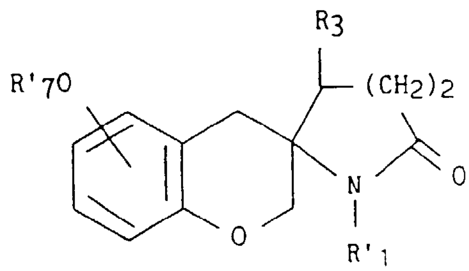

(XIIIb)

wherein $R'_1$, $R_3$ and $R'_7$ have the same meanings as above,
it being understood that the compounds of general formulae VIIIb, Xb, XIb and XIIIb form part of the invention, constitute the compounds of the general formula (Ib) as defined above and may, if desired, be purified, separated into their isomers or, if possible, converted into salts with a pharmaceutically acceptable acid.

**15.** Process for obtaining compounds of the general formula (Ic):

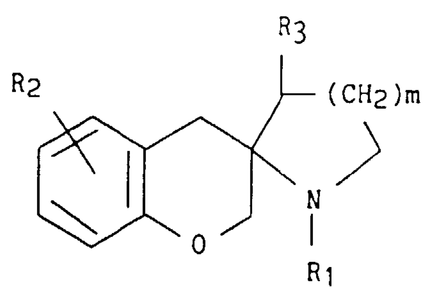

(Ic)

wherein $R_1$, $R_2$, $R_3$ and m are as defined in claim 1,
which is a particular case of the compounds of formula (I) wherein A represents a methylene group ($CH_2$), characterised in that a compound of formula (VIII):

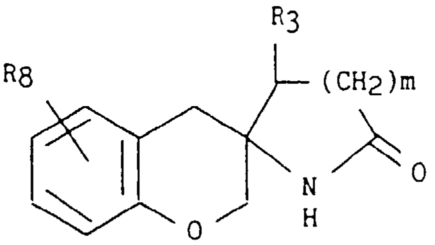

(VIII)

wherein $R_3$ and m are as defined above and $R_8$ represents a hydrogen atom or a linear or branched alkoxy group having from 1 to 6 carbon atoms, is reduced, in an aprotic medium, with a reducing agent so as to obtain, after isolation and optional purification, a compound of the general formula (XIV):

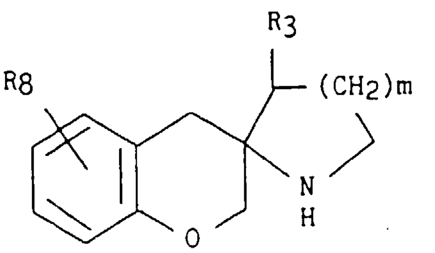

(XIV)

wherein $R_3$, $R_8$ and m have the same meanings as above, which may be reacted with a compound of the general formula (IX):

Hal'R'$_1$ (IX)

wherein R'$_1$ has the same meaning as $R_1$ in claim 1, with the proviso that R'$_1$ cannot represent a hydrogen atom, and Hal represents a halogen atom, so as to obtain, after isolation and optional purification, a compound of the general formula (XV):

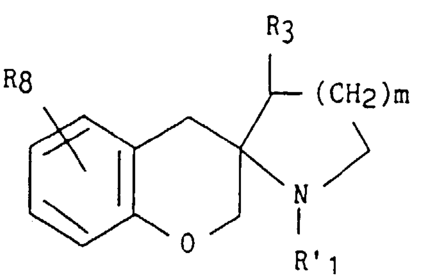

(XV)

wherein R'$_1$, $R_3$, $R_8$ and m have the same meanings as above,
which compounds of formulae (XIV) and (XV) may, when $R_8$ represents an alkoxy group, be treated with an aqueous hydrobromic acid solution, to obtain, after isolation and optional purification, a compound of the general formula (XVI):

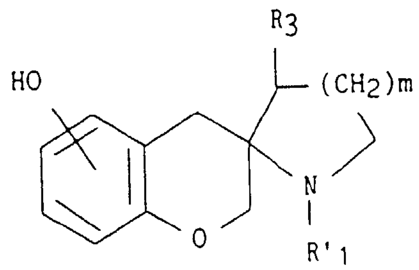

(XVI)

wherein $R'_1$, $R_3$ and m have the same meanings as above, which may be reacted with a compound of the general formula (XII):

$$Hal'' - R'_7 \qquad (XII)$$

wherein Hal" represents a halogen atom and $R'_7$ has the same meaning as $R_7$ in claim 1, with the proviso that $R'_7$ cannot represent a hydrogen atom, so as to obtain, after isolation and optional purification, a compound of the general formula (XVII):

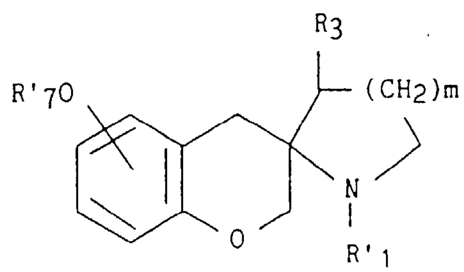

(XVII)

wherein $R'_1$, $R_3$, $R'_7$ and m have the same meanings as above,
it being understood that the compounds of general formulae XIV, XV, XVI and XVII form part of the invention, constitute the compounds of the general formula (Ic) and may, if desired, be purified, separated into their isomers, if possible converted into salts with a pharmaceutically acceptable acid.

**16.** Process for obtaining compounds of the general formula (Id):

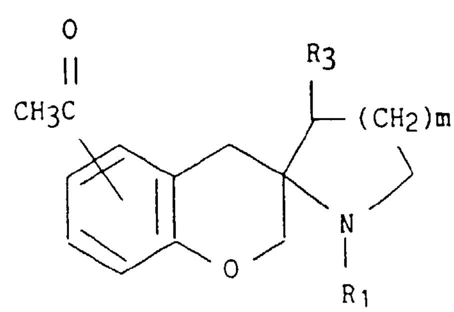

(Id)

wherein $R_1$, $R_3$ and m are as defined in claim 1,
which is a particular case of the compounds of formula (I) wherein $R_2$ represents an acetyl group, characterised in that a compound of the general formula (XVIII):

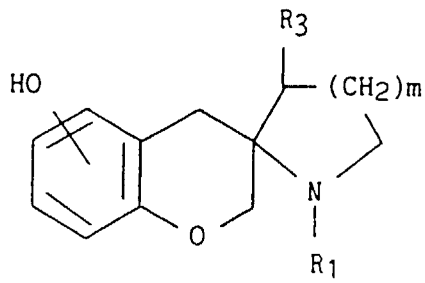

(XVIII)

wherein $R_1$, $R_2$ and m have the same meanings as in claim 1 is reacted with trifluoromethanesulphonic anhydride so as to obtain a trifluoromethylsulphonyloxy compound of the general formula (XIX):

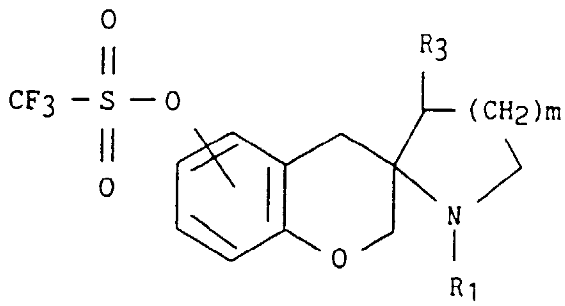

(XIX)

wherein $R_1$, $R_3$ and m have the same meanings as in claim 1, which is then treated, in an aprotic medium, with butyl vinyl ether in the presence of triethylamine, 1,2-bis-(diphenylphosphino)ethane and $Pd(OAc)_2$ so as to obtain, after isolation and purification, an acetylated compound of the general formula (XX):

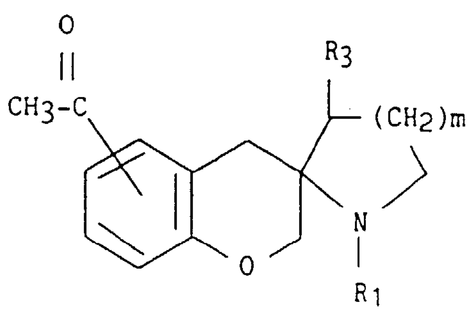

(XX)

wherein $R_1$, $R_3$ and m have the same meanings as in claim 1,
it being understood that the compounds of general formulae XIX and XX form part of the invention and may, if desired, be purified, separated into their isomers or, if possible, converted into salts with a pharmaceutically acceptable acid.

**17.** Pharmaceutical compositions comprising as active ingredient at least one compound according to any one of claims 1 to 12, on its own or in combination with one or more inert non-toxic pharmaceutically acceptable excipients or carriers.

**18.** Pharmaceutical compositions according to claim 17, comprising at least one active ingredient according to any one of claims 1 to 12, for use in the treatment of depression, stress, psychoses, anxiety, schizophrenia, hypertension, pain, cardiovascular diseases, migraines and cerebral ischaemia, and as modifiers of eating behaviour and sexual behaviour.